# EUROPEAN PATENT SPECIFICATION

(11) **EP 2 723 355 B1**
(45) Date of publication and mention of the grant of the patent: **25.10.2017**
(21) Application number: 12727384.5
(22) Date of filing: 14.06.2012
(51) Int. Cl.: C07K 14/705, A61K 39/002, C07K 14/44, C12N 15/79, A61K 35/68, A61K 39/00

(54) **CONDITIONAL KNOCKOUT MUTANTS OF SORTILIN-LIKE RECEPTOR IN APICOMPLEXAN PARASITES AND USES THEREOF**
BEDINGTE KNOCKOUT-MUTANTEN VON SORTILIN-LIKE-REZEPTOREN BEI APICOMPLEXA-PARASITEN UND VERWENDUNGEN DAVON
MUTANTS À INACTIVATION CONDITIONNELLE D'UN RÉCEPTEUR DE TYPE SORTILINE DANS DES PARASITES APICOMPLEXA ET LEURS UTILISATIONS

(30) Priority: 24.06.2011 US 201161500790 P
(43) Date of publication of application: 30.04.2014
(73) Proprietor: Centre National de la Recherche Scientifique, 75794 Paris Cedex 16 (FR); Universite De Lille 2, 59800 Lille (FR); I.N.S.E.R.M. Institut National de la Santé et de la Recherche Médicale, 75654 Paris Cedex 13 (FR)
(72) Inventor: TOMAVO, Stanislas, F-59520 Lille (FR); SLOVES, Pierre-Julien, F-59600 Maubeuge (FR); MOUVEAUX, Thomas, F-59870 Bouvignies (FR)
(74) Representative: Grosset-Fournier, Chantal Catherine
(86) International application number: PCT/EP2012/061385
(87) International publication number: WO 2012/175410

(56) References cited:
- EP-A2- 0 100 710
- WO-A1-2009/058712
- WO-A2-2008/076262
- GB-A- 2 388 112
- US-A- 5 874 526
- PIERRE-JULIEN SLOVES ET AL: "Toxoplasma Sortilin-like Receptor Regulates Protein Transport and Is Essential for Apical Secretory Organelle Biogenesis and Host Infection", CELL HOST & MICROBE, vol. 11, no. 5, 1 May 2012 (2012-05-01), pages 515-527, XP55037040, ISSN: 1931-3128, DOI: 10.1016/j.chom.2012.03.006
- S. FAUQUENOY ET AL: "Proteomics and Glycomics Analyses of N-Glycosylated Structures Involved in Toxoplasma gondii-Host Cell Interactions", MOLECULAR & CELLULAR PROTEOMICS, vol. 7, no. 5, 1 May 2008 (2008-05-01), pages 891-910, XP55037002, ISSN: 1535-9476, DOI: 10.1074/mcp.M700391-MCP200 cited in the application
- SABINA I. BELLI ET AL: "Global protein expression analysis in apicomplexan parasites: Current status", PROTEOMICS, vol. 5, no. 4, 1 March 2005 (2005-03-01), pages 918-924, XP55037123, ISSN: 1615-9853, DOI: 10.1002/pmic.200401161
- DATABASE UniProt [Online] 16 December 2008 (2008-12-16), "SubName: Full=Sortilin, putative;", XP002682790, retrieved from EBI accession no. UNIPROT:B6KKQ8 Database accession no. B6KKQ8
- DATABASE UniProt [Online] 2 September 2008 (2008-09-02), "SubName: Full=Sortilin, putative;", XP002682791, retrieved from EBI accession no. UNIPROT:B3L9P9 Database accession no. B3L9P9
- DATABASE EMBL [Online] 23 March 2009 (2009-03-23), "Homo sapiens sortilin 1 (SORT1) gene, complete cds.", XP002693152, retrieved from EBI accession no. EM_HUM:FJ525881 Database accession no. FJ525881
- ZENG J ET AL: "The inactivation of the sortilin gene leads to a partial disruption of prosaposin trafficking to the lysosomes", EXPERIMENTAL CELL RESEARCH, ACADEMIC PRESS, US, vol. 315, no. 18, 1 November 2009 (2009-11-01), pages 3112-3124, XP026688629, ISSN: 0014-4827, DOI: 10.1016/J.YEXCR.2009.08.016 [retrieved on 2009-09-02]
- JANSEN PERNILLE ET AL: "Roles for the pro-neurotrophin receptor sortilin in neuronal development, aging and brain injury", NATURE NEUROSCIENCE, NATURE AMERICA, INC, US, vol. 10, no. 11, 1 November 2007 (2007-11-01), pages 1449-1457, XP002478373, ISSN: 1097-6256, DOI: 10.1038/NN2000
- M. MEISSNER: "Tetracycline analogue-regulated transgene expression in Plasmodium falciparum blood stages using Toxoplasma gondii transactivators", PROCEEDINGS OF THE NATIONAL ACADEMY OF SCIENCES, vol. 102, no. 8, 22 February 2005 (2005-02-22), pages 2980-2985, XP055055115, ISSN: 0027-8424, DOI: 10.1073/pnas.0500112102
- MAZELLA ET AL: "Sortilin/neurotensin receptor-3: a new tool to investigate neurotensin signaling and cellular trafficking?", CELLULAR SIGNALLING, ELSEVIER SCIENCE LTD, GB, vol. 13, no. 1, 1 January 2001 (2001-01-01), pages 1-6, XP027373262, ISSN: 0898-6568 [retrieved on 2001-01-01]
- CHRISTIAN B VAEGTER ET AL: "Sortilin associates with Trk receptors to enhance anterograde transport and neurotrophin signaling", NATURE NEUROSCIENCE, vol. 14, no. 1, 1 January 2011 (2011-01-01), pages 54-61, XP055055099, ISSN: 1097-6256, DOI: 10.1038/nn.2689

## Description

The application deals with conditional knockout mutants of Apicomplexan parasites and uses thereof.

Apicomplexans are protozoan parasites defined by the presence of a complex of specialized organelles at their apical end. The phylum Apicomplexa contains many important pathogens of humans and animals, including the causative agents of malaria (Plasmodium spp.) and chicken coccidiosis (Eimeria spp.) as well as some of the opportunistic infections associated with AIDS patients such as Cryptosporidium parvum and Toxoplasma gondii.

T. gondii is uniquely adapted to infect a wide range of hosts, including virtually all warm blooded animals and up to 50% of the world's human population. The primary transmission route in humans is via ingestion of undercooked contaminated meat, particularly lamb, and contact with feces from infected domestic cats. The parasite causes a serious opportunistic disease (toxoplasmosis) in congenitally infected infants that can lead to severe syndromes including congenital malformations such as blindness, mental retardation, and hydrocephaly in children exposed in utero. More attention has been paid to T. gondii because toxoplasmosis is a common opportunistic infection in immunocompromised individuals with AIDS and in transplant patients. Although the parasite has a sexual cycle occurring in cats, in mammalian non-feline hosts, T. gondii is found in two haploid asexual forms, the rapidly replicating tachyzoites and the slowly dividing, quiescent encysted bradyzoites. Infection during the acute phase is rapidly established in the host by the fast replicating tachyzoites, which can invade a broad range of cell types. Unlike other protozoan pathogens that hijack the pre-existing host cell uptake machinery, T. gondii actively gains entry into host cells. Penetration is of paramount importance for survival and relevant to its pathogenesis during acute infection. In response to immune defense, tachyzoites differentiate into encysted bradyzoites that remain in the brain and other organs throughout the life of chronically infected hosts. The reactivation of encysted bradyzoites into actively replicating and cytolytic tachyzoites is the cause of fatal toxoplasmic encephalitis in AIDS patients. Like all apicomplexan parasites, T. gondii lacks cilia or flagella throughout most of its life cycles and enters into host cells using an unusual form of gliding motility. The process of host cell invasion also requires contact with the host cell plasma membrane followed by reorientation and generation of a motive force ensured by the actomyosin motor, which drives gliding and penetration into a novel, parasite-induced structure called the parasitophorous vacuole. Successful invasion of host cells requires a highly regulated release of proteins from several parasite organelles, namely micronemes, rhoptries, and dense granules, microneme discharge occuring first. Their contents are involved in the attachment to the host cell membrane and the formation of a connection with the parasite's actomyosin system, thereby providing the motive force that drives motility and invasion.

Recently the inventors have provided new evidence that T. gondii contains numerous N-glycosylated proteins that unexpectedly are components of the glideosome, moving junction, and other glycoproteins involved in host cell-parasite interactions and thus contribute to parasites survival and pathogenesis during mammalian host cell infection. One of said proteins is a putative sortiline (Fauquenoy S, Morelle W, Hovasse A, Bednarczyk A, Slomianny C, Schaeffer C, Van Dorsselaer A, Tomavo S. Proteomics and glycomics analyses of N-glycosylated structures involved in Toxoplasma gondii--host cell interactions. Mol. Cell Proteomics. 2008 May;7(5):891-910). Said sortilin-like receptor which is mainly localized in the Golgi compartment of the parasite, belongs to a growing family of multi-ligand type-1 membrane receptors that are involved in protein sorting and trafficking. The receiver appointed TgSORTLR ("Toxoplasma gondii Sortilin Receptor-Like") involved in the sorting of proteins to apical organelles (rhoptries and micronemes) that are exclusively found in apicomplexan parasites, is also present in all apicomplexan whose genomes have been sequenced.

Sortilin sometimes also referred to as Neurotensin receptor 3 (NTR3), Glycoprotein 95 (Gp95) or 100 kDa NT receptor of Swiss Prot ID No. Q99523 is the archetypical member of a mammalian family of neuronal receptors defined by the unique Vps1 Op-domain (Vps1 Op-D) that among other ligands binds neurotrophic factors and neuropeptides. This domain constitutes the entire luminal part of Sortilin (sSortilin) and is activated for ligand binding by enzymatic propeptide cleavage. Sortilin is a multifunctional type-1 receptor capable of endocytosis as well as intracellular sorting, and as shown recently, it also engages in signaling by triggering proneurotrophin-induction of p75^{NTR}-mediated neuronal apoptosis. Sortilin is synthesized as a proprotein, which is converted to mature Sortilin by enzymatic cleavage and removal of a short N-terminal propeptide. Only the mature receptor binds ligands and interestingly, all its known ligands, e.g. Neurotensin (NT), lipoprotein lipase, the proforms of nerve growth factor-∮ (proNGF) and brain derived neurotrophic factor (proBDNF), receptor associated protein (RAP), and its own propeptide, compete for binding, indicating that the diverse ligands target a shared or partially shared bi ndi ng site.

Parasitic diseases continue to bring misery to much of the world's population. Humans with Cryptosporidiosis and Toxoplasmosis suffer greatly and many people die from the diseases, or lose their ability to be productive members of their communities. Similarly, these and other parasites annually kill large numbers of the vertebrates (cows, sheep, goats, sheep, pigs, and chickens) that are human primary food sources worldwide.

Several methods are being used to roll back these parasitic diseases, including reduction of insect vectors, drugs, and vaccines. None of these are completely effective, though. One problem with existing vaccine is that they target surface antigens of poorly understood or unknown function. In addition, the targets mutate and render the organism resistant to the vaccine. Therefore, new discoveries and new approaches are essential to combat parasitic protozoan diseases.

The inventors have now found that an acute virulence-attenuated and non-infectious strain, which is a conditional knock-out mutant of Toxoplasma gondii, has the ability to protect animals against Toxoplasma infection.

The application describes vaccines comprising said conditional knock-out mutant of Toxoplasma gondii, which are capable of providing protection against infection with Toxoplasma gondii.

The application describes a method of immunizing healthy animals against Toxoplasma gondii infection comprising administering to a healthy animal an effective amount of a vaccine comprising said conditional knock-out mutant of Toxoplasma gondii.

The application describes a nucleic acid construct which comprises:
(i) a sequence comprising 100 to 10000 bp, advantageously 150 to 4000 bp, more advantageously 1000 to 2000 bp contiguous nucleotides of a 5' non-coding and promoter regions of Sortilin of an eukaryote organism,
(ii) a selection gene cassette, said gene cassette having its own 5' and 3' UTR flanking sequence on both side of its coding sequence,
(iii) a sequence comprising 100 to 10000 bp, advantageously 150 to 4000 bp, more advantageously 1000 to 2000 bp contiguous nucleotides of a 3' coding region of Sortilin of said eukaryote organism, said sequence being driven by an anhydrotetracycline(ATc)-sensitive transactivator.

The invention relates to a nucleic acid construct which comprises:
(i) a sequence comprising 100 to 10000 bp, advantageously 150 to 4000 bp, more advantageously 1000 to 2000 bp contiguous nucleotides of a 5" non-coding and promoter regions of Sortilin of an Apicomplexan parasite, notably chosen among Toxoplasma gondii, Plasmodium falciparum, Babesia bovis, Cryptosporidium hominis or Neospora caninum,
(ii) a selection gene cassette, said gene cassette having its own 5' and 3'UTR flanking sequence on both side of its coding sequence,
(iii) a sequence comprising 100 to 10000 bp, advantageously 150 to 4000 bp, more advantageously 1000 to 2000 bp contiguous nucleotides of a 3' coding region of Sortilin of said Apicomplexan parasite, said sequence being driven by an anhydrotetracycline(ATc)-sensitive transactivator.

According to the application, Sortilin of an eukaryote organism includes sequences SEQ ID no 5, SEQ ID:NO 8, SEQ ID:NO 11, SEQ ID:NO 14, SEQ ID:NO 17, SEQ ID:NO 20 and SEQ ID:NO 29 and homologues, orthologues, and paralogues thereof. As used herein the term 'orthologues_are separate occurrences of the same gene in multiple species. The separate occurrences have similar, albeit non-identical, ami no acid sequences, the degree of sequence similarity depending, in part, upon the evolutionary distance of the species from a common ancestor having the same gene. As used herein, the term 'paralogues_ indicates separate occurrences of a gene in one species. The separate occurrences have similar, albeit non-identical, amino acid sequences, the degree of sequence similarity depending, in part, upon the evolutionary distance from the gene duplication event giving rise to the separate occurrences. As used herein, the term 'homologues_ is generic to 'orthologues_ and 'paralogues_.

The sequence of the 5' non-coding and promoter regions of Sortilin of an eukaryote organism is located upstream to the ATG codon and sufficiently near of it in order to permit the entrance of said sequence i n an homologue manner i n the genome.

The sequence of 3' coding regions of Sortilin of said eukaryote organism is located downstream of the ATG codon and sufficiently near of it in order to permit the entrance of said sequence i n a homologue manner i n the genome.

In an advantageous embodiment of the application, the 5' non-coding and promoter region of Sortilin is selected from a group consisting of SEQ ID:NO 1, SEQ ID:NO 6, SEQ ID:NO 9, SEQ ID:NO 12, SEQ ID:NO 15, SEQ ID:NO 18 and SEQ ID:NO 27 and the 3' coding region of Sortilin is selected from a group consisting of SEQ ID:NO 3, SEQ ID:NO 7, SEQ ID:NO 10, SEQ ID:NO 13, SEQ ID:NO 16, SEQ ID:NO 19, and SEQ ID:NO 28.

In an advantageous embodiment of the invention, the 5' non-coding and promoter region of Sortilin is selected from a group consisting of SEQ ID:NO 1, SEQ ID:NO 6, SEQ ID:NO 9, SEQ ID:NO 12, SEQ ID:NO 15 and SEQ ID:NO 18, and wherein the 3' coding region of Sortilin is selected from a group consisting of SEQ ID:NO 3, SEQ ID:NO 7, SEQ ID:NO 10, SEQ ID:NO 13, SEQ ID:NO 16 and SEQ ID:NO 19

In another advantageous embodiment of the application or invention, the selection gene cassette is a nucleic acid sequence of dihydrofolate reductase EC 1.5.1.3.

According to the application, the eukaryote organism is chosen among Apicomplexan parasites and Homo sapiens.The apicomplexa are an extremely large and diverse group of protozoa, characterized by the presence of a unique organelle called an apical complex. They are single-celled, spore-forming, and exclusively parasites of animals. Seven species infect humans (Plasmodium, Babesia, Cryptosporidium, Neospora, Isospora, Cyclospora, Sarcocystis, and Toxoplasma) whereas several apicomplexan parasites are important in terms of veterinary medicine and agriculture. Most notable are Babesia and Theileria in cattle and Eimeria in poultry. Apicomplexan protozoans include, for example, Babesia microti, Babesia bovis and Babesi divergens (causing babesiosis), Plasmodium (causing malaria), Cryptosporidium parvum and Cryptosporidium hominis (causing cryptosporidiosis), Cyclospora cayetanensis (causing cyclosporiasis), and Toxoplasma gondii (causing toxoplasmosis) and Neospora caninum (causing neosporosis).

According to the application or invention, A pi compl exan parasites include but are not limited to Toxoplasma gondii, Plasmodium falciparum, Babesia bovis, Cryptosporidium hominis or Neospora caninum

In an advantageous embodiment of the application or invention, the anhydrotetracycline(ATc)-sensitive transactivator is chosen among TetO7-Sag1 or TetO7-Sag4.

In another advantageous embodiment of the application or invention, the selection gene cassette is a nucleic acid sequence of dihydrofolate reductase EC 1.5.1.3, and/or the anhydrotetracycline(ATc)-sensitive transactivator is chosen among TetO7-Sag1 or TetO7-Sag4.

The application describes and the invention is also directed to a nucleic acid construct as defined above comprising:
(i) a sequence of 2000 contiguous nucleotides of the 5' non-coding and promoter regions of Sortilin-like receptor of Toxoplasma gondii said sequence being SEQ ID:NO 2,
(ii) a dihydrofolate reductase gene cassette, said gene cassette having its own 5' and 3'UTR flanking sequence on both side of its coding sequence,
(iii) a sequence of 2000 contiguous nucleotides of the 3' coding regions of Sortilin-like receptor of Toxoplasma gondii, said sequence being SEQ ID:NO 4 and said sequence bei ng driven by an inducible TetO7-Sag 4 promoter.

Another object of the application or invention is a vector comprising a nucleic acid construct as defined above.

According to the application or invention all vectors known from the man skilled in the art may be used, for example a plasmid, a cosmid, a phagemid, a bacterial artificial chromosome or a yeast artificial chromosome.

The transgenic non-pathogenic Apicomplexan parasite may be prepared by any method known from the man skilled i n the art.

Thus the application describes and the invention is also directed to a method for producing transgenic-non pathogenic Apicomplexan parasite, wherein the method comprises:
(i) providing a vector as defined above,
(ii) providing a drug inducible and high homologous integration of transfected DNA Apicomplexan parasite,
(iii) transfecting said Apicomplexan parasite by said vector,
(iv) selecting the transformed A picomplexan parasite by using a second drug,
(v) cloning the transformed Apicomplexan parasite.

The application describes and the invention is also directed to a method for producing transgenic-non pathogenic Toxoplasma gondii strain, wherein the method comprises:
(i) providi ng a vector as defi ned above,
(ii) providing a drug inducible and high homologous integration of transfected DNA Toxoplasma gondii strain,
(iii) transfecting said Toxoplasma gondii strain by said vector,
(iv) sel ecti ng the transformed Toxoplasma gondii strain by usi ng a second drug,
(v) cloning the transformed Toxoplasma gondii strain.

In an advantageous embodiment, the application describes and the invention is also directed to a method for producing transgenic-non pathogenic Apicomplexan parasite, notably Toxoplasma gondii strain, wherein the method comprises:
(i) providi ng a vector as defi ned above,
(ii) providing a drug inducible and high homologous integration of transfected DNA Apicomplexan parasite, notably Toxoplasma gondii strain,
(iii) transfecting said Apicomplexan parasite, notably Toxoplasma gondii strain, by said vector,
(iv) selecting the transformed Apicomplexan parasite, notably Toxoplasma gondii strain, by using a second drug,
(v) cloning the transformed Apicomplexan parasite, notably Toxoplasma gondii strain.

As drug inducible and high homologous integration of transfected DNA Apicomplexan parasite one can cite the transgenic lines RH ∀ku80TaTi (ATc inducible and higher homologous recombination of plasmid) prepared from RH TaTi (Transactivator trap identified inducible with anydrotetracycline or ATc) disclosed by Meissner, M., Schlaeter, D., Soldati, D. Role of Toxoplasma gondii myosin A in powering parasite gliding and host cell invasion. Science 298, 837-40 (2002) and RH ∀ku80 (high homologous integration of transfected DNA) Huynh, M.H., Carruthers, V.B. Tagging of endogenous genes in a Toxoplasma gondii strain lacking Ku80. Eukaryot Cell. 8, 530-539 (2009).

Another object of the invention is a transgenic-non pathogenic strain of Apicomplexan parasite, wherein the wild type locus of Sortilin is substituted by an artificial nucleotide sequence comprising:
i. 100 to 10000 bp, advantageously 150 to 4000 bp, more advantageously 1000 to 2000 bp contiguous nucleotide of a 5' non-coding and promoter regions of Sortilin of said Apicomplexan parasite,
ii. a selection gene cassette, and
iii. a sequence comprising 100 to 10000 bp, advantageously 150 to 4000 bp, more advantageously 1000 to 2000 bp of a 3' coding regions of Sortilin of said Apicomplexan parasite, said coding region being driven by a tetracycline-controlled transactivator.

Another object of the invention is a transgenic-non pathogenic strain of Toxoplasma gondii, wherein the wild type locus of Toxoplasma gondii Sortilin-like receptor (TgSORTLR) is substituted by an artificial nucleotide sequence comprising:
i. a fragment of 2 kb of TgSORTLR 5' non-coding and promoter regions, said sequence being SEQ ID:NO 2,
ii. a di hydrofol ate reductase gene cassette, and
iii. a fragment of 2 kb of DNA coding region of the TgSORTLR, said sequence being SEQ ID:NO 4, said coding region being driven by an inducible TetO7-Sag 4 promoter.

The transgenic-non pathogenic strains of Apicomplexan parasite, in particular the transgenic-non pathogenic strains of Toxoplasma gondii, according to the invention are avirulent strains in a parasitic form totally attenuated which may be used as a live vaccine.

Thus another object of the invention is a composition comprising an effective amount of the transgenic-non pathogenic strain of an Apicomplexan parasite as defined above, in particular the transgenic-non pathogenic strain of Toxoplasma gondii as defined above, and a nontoxic and sterile pharmaceutically acceptable carrier.

Suitable carriers are well known to those of skill in the art. However, exemplary carriers include sterile saline, lactose, sucrose, calcium phosphate, gelatin, dextrin, agar, pectin, peanut oil, olive oil, sesame oil and water. Additionally, the carrier or diluent may include a time delay material, such as glyceryl monostearate or glyceryl distearate alone or with a wax.

The application describes a vaccine comprising the composition as defined above. Said vaccine may comprise a conventional, adjuvant. The adjuvant is used as a non-specific irritant to attract or enhance an immune response. Such adjuvants include, among others, amphigen, aluminum hydroxide, muramyl dipeptide and saponins such as Quil A.

This vaccine may be used to protect the animal against pathologies due to apicomplexan parasites.

Consequently the application describes a method for immunizing mammals against pathologies caused by Apicomplexan parasites comprising the step of administering thereto an amount of transgenic-non pathogenic strain of Apicomplexan parasite as defined above for developing immunity to Apicomplexan parasites.

The application describes a method for immunizing mammals against toxoplasmosis comprising the step of administering thereto an amount of transgenic-non pathogenic strain of Toxoplasma gondii as defined above for developing immunity to Toxoplasma gondii.

The application describes a method for stimulating T cell response against cells infected by Toxoplasma gondii in a patient persistently infected with said parasite, wherein said method comprises administering to said patient a composition as defined above.

The compositions of the application or invention are advantageously made up in a dosage unit form adapted for the desired mode of administration. The quantity of transgenic-non pathogenic strain of Apicomplexan parasite forming the active ingredients in each dosage unit will be adapted for the case and the composition may be buffered at physiological pH, in a form ready for injection.

Dosage and administration protocol can be optimized in accordance with standard vaccination practices. Typically, the vaccine will be administered subcutaneously, although other routes of administration may be used, such as intramuscular, oral, intraocular, intradermal and intranasal administration. It will be understood that the specific dose level for any particular animal will depend upon a variety of factors including the age, general health, sex, and diet of the animal or the patient; the species of the animal; the time of administration; the route of administration; interactions with any other drugs being administered; and the degree of protection being sought. The vaccine can be administered only once or if desirable, periodically. Of course, as stated above, the administration can be repeated at suitable intervals if necessary or desirable.

The transgenic-non pathogenic strain of Apicomplexan parasite can be used in screening assays to identify new drugs active in the treatment of pathologies due to Apicomplexan parasites.

Thus the appl i cati on descri bes a method for the screeni ng for new drugs in the treatment of toxoplasmosis, babesiosis, malaria, cryptosporidiosis, neosporosis, diabete or Alzheimer disease comprisi ng the fol lowi ng steps:
i. contacting a compound known to, or suspected of being able to be active with a sortilin of an intracellular parasite, and
ii. determi ni ng the effect of the compound on sai d sortilin wherein an effect on sortilin indicates the potential of the compound as a drug.

The application describes a method of screening for compounds that are active against toxoplasmosis, said method comprising the following steps:
i. contacting a compound known to, or suspected of being able to be active with Toxoplasma gondii Sortilin-like receptor, and
ii. determining the effect of the compound on said Toxoplasma gondii Sortilin-like receptor wherein an effect on sortilin indicates the potential of the compound as a drug.

The application describes a method for the identification of new proteins involved in host cell-Apicomplexan parasite comprising the step of interacting sortilin with protei ns of host cell.

The application describes a method for performing the in vitro diagnosis of a pathology caused by Toxoplasma gondii, notably of toxoplasmosis in a mammal comprising the step of determining the presence or amount of Toxoplasma gondii Sortilin-like receptor or of fragments thereof, in a biological sample of said mammal.

The application describes a method for performing the in vitro diagnosis of a pathology caused by Toxoplasma gondii, notably of toxoplasmosis in a mammal comprising the step of determining the presence or amount of an antibody raised against Toxoplasma gondii Sortilin-like receptor in a biological sample of said mammal.

In another embodiment, the application describes a method for performing the in vitro diagnosis of a pathology caused by Toxoplasma gondii, notably of toxoplasmosis in a mammal comprisi ng the step of:
- determining the presence or amount of Toxoplasma gondii Sortilin-like receptor or of fragments thereof, and/or
- determining the presence or amount of an antibody raised against Toxoplasma gondii Sortilin-like receptor,
in a biological sample of said mammal.

As used herein, the term 'antibody_ refers to a polypeptide, at least a portion of which is encoded by at least one immunoglobulin gene, or fragment thereof and that can bind specifically to a desired target molecule. The term includes naturally-occurring forms, as well as fragments and derivatives. Fragments within the scope of the term 'antibody_ include those produced by digestion with various proteases, those produced by chemical cleavage and/or chemical dissociation, and those produced as recombinant components, so long as the fragment remains capable of specific binding to a target molecule. Among such fragments are Fab, Fab→Fv, F(ab)→2, and single chain Fv (scFv) fragments. Derivatives within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, e.g., Marasco (ed.), Intracellular Antibodies: Research and Disease Applications , Springer-Verlag New York, Inc. (1998) (ISBN: 3540641513). As used herein, antibodies can be produced by any known technique, including harvest from cell culture of native B lymphocytes, harvest from culture of hybridomas, recombinant expression systems, and phage display.

The application describes a kit for in vitro diagnosis of a pathology caused by Toxoplasma gondii, notably of toxoplasmosis comprising:
(i) at least one antibody suitable for binding an epitope comprising or consisting of Toxoplasma gondii Sortilin-like receptor, or of fragments of said pepti de,
(ii) at least one antibody coupled to a detection system suitable for binding the antibody-antigen complex,
and optionally at least one Toxoplasma gondii Sortilin-like receptor or fragments thereof.

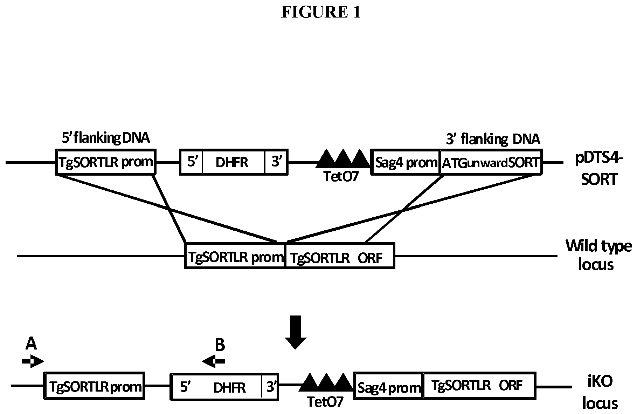

Thefollowing examples 1 to 4 and figures 1 to 4 illustrate the invention.
Figure 1 shows the scheme of the conditional knockout strategy used to conditionally disrupt Sortilin expression in Toxoplasma gondii. A linear DNA fragment containing 2 kb of TgSORTLR 5' non-coding and promoter regions, a sequence encoding dihydrofolate reductase, tetracycline-controlled transactivator TetO7-Sag4 promoter and 2kb of DNA region downstream the TgSORTLR ATG codon was sub-cloned in pDTS4plasmid. Transfection of pDTS4 containing the DNA construct as previously discribed (namely iKO plasmid on figure 1) was performed into the parental RH∀Ku80TaTi Toxoplasma gondii strain (provided by Dr Striepen, University of Georgia, USA). Integration occurs in the wild type locus of T. gondii by homologous recombination, creating a conditional knockout for Sortilin.

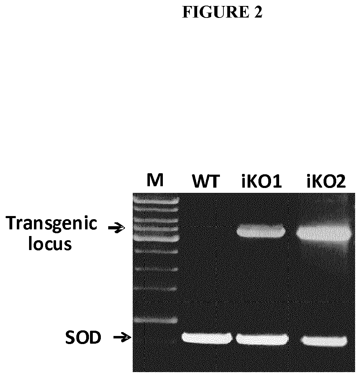

Figure 2 shows detection of transgene integration by analytical PCR ongenomic DNA of wild type parental RH∀Ku80TaTi Toxoplasma gondii strain (second lane) and conditional knockout clones for Sortilin (third and forth lanes), called iKO1 and iKO2. Molecular marker(kb) is in the first lane. The upper band corresponding approximately to 4000 bp is the transgenic locus (TgSORTLR). The lower band of less than 500 bp is the sequence of superoxide dismutase (SOD) and is used as a control for PCR assay.

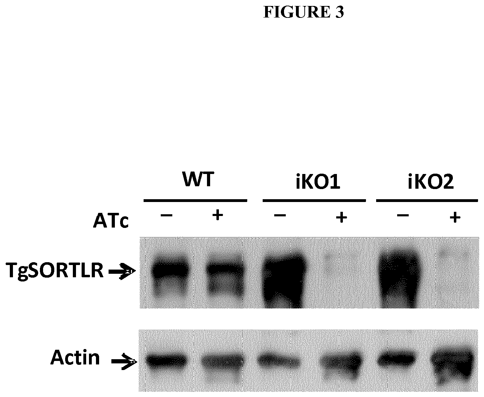

**Figure 3** shows inducibility of Sortilin expression in clones iKO1 (third and forth lanes) and iKO2 (fifth and sixth lanes) which have regulatable Sortilin. Sortilin expression was also analysed in wild type parental RHΔKu80TaTi *Toxoplasma gondii* strain (first and second lanes). Intracellular parasites were grown for 48 hours in the presence or absence of anhydrotetracycline (ATc) before preparation of whole cell lysate. Western blot analysis was performed on whole cell lysate with anti-sortilin antibodies which reveals that these clones lacked Sortilin when treated by ATc. The level of detection of Actin was used as control for equal loading.

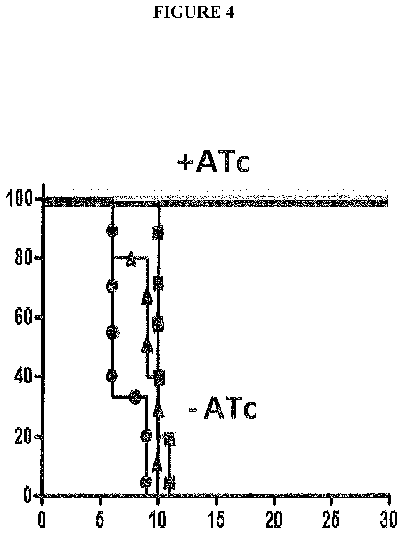

**Figure 4** shows the iKO tachyzoite parasites are avirulent in mice. BALB/c mice were injected intraperitoneally with doses ranging from 5x10³, 10⁴, 2.5x10⁴, 10⁵, 10⁶, 2.5x10⁶ (middle grey lines) of conditional TgSORTLR iKO knockout parasites and received ATc in the drinking water. A second group of mice was injected intraperitoneally with doses ranging from 5x10³ (black squares), 10⁴ (black triangles), 2.5x10⁴ (black points) of conditional TgSORTLR iKO knockout parasites and was not treated with ATc. Results are expressed as the percentage of survival (vertical axis) during the course of infection (horizontal axis).

### Example 1: Preparation of conditional knockout mutant of Sortilin-like receptor in Toxoplasma gondii

### 1.1 - Protocols

### 1.1.1 - pDTS4-SORT construct

DNA fragments of Sortilin-like receptor were amplified with the oligonucleotides 5'TgSORT_F and 5' TgSORT_R for the 5' flanking DNA fragment, and with 3'KISORT_F and 3'KISORT_R for the 3' flanking DNA fragment, which are:

| | |
|---|---|
| 5'TgSORT_F | |
| ccggCATATGATGTCATCAGGAGCCCCTCTGCC | SEQ ID:NO 21 |
| 5'TgSORT_R | |
| ccggCATATGGGTGACTGTCTGTACACCGGAGG | SEQ ID:NO 22 |
| 3'KISORT_F | |
| ccggAGATCTATGGCGCACAGCTTCGCACGAG | SEQ ID:NO 23 |
| 3'KISORT_R | |
| ccggCCTAGGCCGCGGTAGACCAGACGCGTG | SEQ ID:NO 24 |

PCR were performed using 50 ng of genomic DNA from RHΔKu80 *Toxoplasma gondii* strain (Huynh et al. 2009, Eukaryot Cell, 8, 530-539) and High Fidelity PCR Enzyme Mix (Fermentas, K0191) under the following conditions: 3 minutes incubation at 94°C, followed by 35 cycles of 94°C for 30 seconds/ 60°C for 30 seconds/ 72°C for 135 seconds followed by one cycle of 72°C for 10 minutes. The resulting amplified fragments were recovered and cloned in the NdeI/NdeI sites for 5' TgSORT fragment and in the AvrII/BglIII sites 3' TgSORT fragment of pDTS4. This new construct, called pDTS4-SORT is illustrated in figure 1.

### 1.1.2 - Toxoplasma gondii knockout mutant

RHΔKu80TaTi recipient strain of *Toxoplasma gondii* which is anhydrotetracycline (ATc) inducible and higher homologous recombination of plasmid was generously provided by Dr Striepen (University of Georgia, USA). Recipient strain for (ATc)-sensitive transactivator was established by transformation of RHΔKu80TaTi by pDTS4-SORT. Briefly, tachyzoite parasites were purified, filtered and washed twice with cytomix (120 mM KCl, 0.15 mM CaC12, 10 mM K2HPO4/KH2PO4 pH 7.6, 25 mM Hepes pH 7.6, 2 mM EGTA pH 7.6, 5 mM MgC12). Parasites were then concentrated in cytomix supplemented with 2 mM ATP and 5 mM Glutathion (GSH). 10 millions of these parasites were transformed with 50 µg of ApaI linearised pDTS4-SORT by electroporation (BTX electroporation generator Harvard Apparatus, 1.5 kEV, 24Ω for 0.25 msec). The vector was linearised to increase the frequency of homologous recombination. Ten minutes later, transformed parasites were inoculated to confluent Human Foreskin Fibroblast (HFF) grown in DMEM supplemented with 10% fetal calf serum, 2 mM glutamine and 25 µg/ml gentamicin under 5% CO₂, at 37°C.

Four hours later infection, 2 µM pyrimethamine was added to the culture medium. Three days later infection, parasites were added to new culture of HFF and pyrimethamine was added once again four hours post infection. Cloning of pDTS4-SORT transformed RHΔKu80TaTi was done by limiting dilution.

### Example 2: Detection of transgenic locus of Sortilin (TgSORTLR)

### 2.1 - Protocol

Detection of transgenic locus of Sortilin was performed by analytical PCR on genomic DNA of pDTS4-SORT transformed RHΔKu80TaTi. Transgenic locus was amplified with the oligonucleotides iKO-100pb F (A) and DHFR_intR (B), which are:

| | |
|---|---|
| iKO-100pb F (A) | |
| CCGGCGTAGACGTGCTAAGTCTGTGCCA | SEQ ID: NO 25 |
| DHFR_intR (B) | |
| GGCGTTGAATCTCTTGCCGACTGATGGAGAGGGAAGTCC | SEQ ID: NO 26 |

PCR were performed on 50 ng of genomic DNA from pDTS4-SORT transformed RHΔKu80TaTi *Toxoplasma gondii* strain by using Dream Taq Polymerase (Fermentas, EP0701) under the following conditions: 3 minutes incubation at 95°C, followed by 35 cycles of 95°C for 30 seconds/ 58°C for 30 seconds/ 72°C for 4 minutes followed by one cycle of 72°C for 10 minutes. The resulting amplified fragments were migrated of agarose gel with ethidium bromide.

### 2.2 - Results

They are illustrated in figure 2. The transgenic locus (upper band) is present only in iKO1 and iKO2 clones of pDTS4-SORT transformed RHΔKu80TaTi. In the wild type strain corresponding to RHΔKu80TaTi, no amplification fragment corresponding to the transgenic locus can be seen.

### Example 3: Inducibility of transgenic locus of Sortilin (TgSORTLR) expression

### 3.1 - Protocol

pDTS4-SORT transformed RHΔKu80TaTi and wild type RHΔKu80TaTi tachyzoite parasites were incubated in the presence or absence of anhydrotetracycline (ATc) for 48-72 hours before preparation of whole cell lysate.

Parasites were harvested, lysed with Laemmli sample buffer (60 mM Tris-Cl pH 6.8, 2% SDS, 10% glycerol, 5% β-mercaptoethanol, 0.01% bromophenol blue) by boiling 3 minutes. Supernatant was separated of the cellular residues by centrifugation. Said supernatant is the whole protein extract.

Western blots were performed according to the well know techniques in the art. Briefly, whole protein extract was run on SDS-PAGE electrophoresis. Once migration has occurred, the proteins are then transferred to a nitrocellulose or PVDF where they are probed using antibodies specific to the target protein actin and sortilin. Anti-actin antibody and anti-sortilin were produced by the inventors according to known techniques and used at a dilution of 1:1000 in PBS containing 0.5% Tween-20. Specific hybridization is revelead by appropriate use of secondary antibody and detection means.

### 3.2 - Results

They are illustrated in figure 3. In the wild type strain of RHΔKu80TaTi (WT), Sortilin protein is present both in the ATc treated parasites and in non treated parasites. iKO1 and iKO2, the two clones derived from pDTS4-SORT transformed RHΔKu80TaTi, both express Sortilin when unstimulated by ATc. When these clones are treated by ATc, Sortilin expression is completely switch off. Anti-sortilin antobodies reveals that iKO1 and iKO2 clones lacked endogenous Sortilin when cultured with ATc.

### Example 4: Avirulence of conditional knockout of Sortilin (TgSORTLR)

### 4.1 - Protocol

Assays were performed on female Balb/C mice, divided by groups of 6 mice. To determine the virulence of TgSORTLR iKO clones *in vivo,* a group of female Balb/C mice of 6-8 week old were injected intraperitoneally (i.p.) with respectively 5x10³, 10⁴, 2.5x10⁴, 10⁵, 10⁶, 2.5x10⁶ of TgSORTLR iKO tachyzoites. The drinking water was supplemented with 0.2 mg/ml of ATc and 5% of sucrose. A second group of mice were also infected i.p. with 5x10³, 10⁴, and 2.5x10⁴ of TgSORTLR iKO tachyzoites without ATc in the drinking water. As control, 5x10³, 10⁴, 2.5x10⁴ tachyzoites of the parental line (RHΔku80TaTi WT strain) were used to infect two other groups of mice treated with or without ATc in the drinking water, making a total of 15 groups of 6 mice each. These experiments were repeated twice with even more parasite loads up to 10⁷ TgSORTLR iKO mutants in the second experiments. The survival of mice was monitored over a period of 6 weeks, and RHΔku80TaTi seroconversion of all surviving mice was confirmed by Western blots. All care and handling of animals was in accordance with the Amsterdam Protocol on animal protection and welfare, and Directive 86/609/EEC on the Protection of Animals Used for Experimental and Other Scientific Purposes (http://conventions.coe.int/Treaty/EN/Treaties/PDF/123-Arev.pdf). The Pasteur Institute of Lille and the CNRS Committee on the Ethics of Animal Experiments specifically also approved this study.

### 4.2 - Results

They are illustrated in figure 4. After 10 days, all the mice infected by RHΔku80TaTi WT strain tachyzoites were dead (data not shown).

Moreover, all of the mice of the second group infected with TgSORTLR iKO tachizoites without ATc in the drinking water died between day 9 and day 11 after infections.

In contrast, we observed 100% survival in the group of mice infected with TgSORTLR iKO tachyzoites and supplemented with ATc in the drinking water, even for high doses of parasites. At day 30 post-infection, these mice are still alive even with high load of parasites showing that TgSORTLR iKO parasites have lost their infectious capacity.

## Claims

1. A nucleic acid construct which comprises:
(i) a sequence comprisi ng 100 to 10000 bp, advantageously 150 to 4000 bp, more advantageously 1000 to 2000 bp contiguous nucleotides of a 5' non-coding and promoter regions of Sortilin of an A picomplexan parasite, notably chosen among Toxoplasma gondii, Plasmodium falciparum, Babesia bovis, Cryptosporidium hominis or Neospora caninum,
(ii) a selection gene cassette, said gene cassette having its own 5' and 3' UTR flanking sequence on both side of its coding sequence,
(iii) a sequence comprising 100 to 10000 bp, advantageously 150 to 4000 bp, more advantageously 1000 to 2000 bp contiguous nucleotides of a 3' coding region of Sortilin of said Apicomplexan parasite, said sequence being driven by an anhydrotetracycl i ne(AT c)-sensitive transactivator.

2. The nucleic acid construct as claimed in claim 1, wherein the 5' non-coding and promoter region of Sortilin is selected from a group consisting of SEQ ID:NO 1, SEQ ID:NO 6, SEQ ID:NO 9, SEQ ID:NO 12, SEQ ID:NO 15 and SEQ ID:NO 18, and wherein the 3' coding region of Sortilin is selected from a group consisting of SEQ ID:NO 3, SEQ ID:NO 7, SEQ ID:NO 10, SEQ ID:NO 13, SEQ ID:NO 16 and SEQ ID:NO 19.

3. The nucleic acid construct as claimed i n claim 1, wherein the selection gene cassette is a nucleic acid sequence of dihydrofolate reductase EC 1.5.1.3, and/or wherein the anhydrotetracycline(ATc)-sensitive transactivator is chosen among TetO7-Sag1 or TetO7-Sag4.

4. The nucleic acid construct as claimed in claim 1 which comprises:
(i) a sequence of 2000 contiguous nucleotides of the 5' non-coding and promoter regions of Sortilin of Toxoplasma gondii, said sequence being SEQ ID:NO 2,
(ii) a dihydrofolate reductase gene cassette, said gene cassette having its own 5' and 3' UTR flanking sequence on both side of its coding sequence,
(iii) a sequence of 2000 contiguous nucleotides of the 3' coding regions of Sortilin of Toxoplasma gondii, said sequence being SEQ ID:NO 4, and said sequence being driven by an inducible TetO7-Sag 4 promoter.

5. A vector comprising a nucleic acid construct as defined in any of claim 1 to 4.

6. A method for produci ng transgenic-non pathogenic Apicomplexan parasite, notably Toxoplasma gondii strain, wherein the method comprises:
(i) providing a vector as defined in claim 5,
(ii) providing a drug inducible and high homologous integration of transfected DNA A picomplexan parasite, notably Toxoplasma gondii strain,
(iii) transfecting said Apicomplexan parasite, notably Toxoplasma gondii strain, by said vector,
(iv) selecting the transformed Apicomplexan parasite, notably Toxoplasma gondii strain, by using a second drug,
(v) cloning the transformed Apicomplexan parasite, notably Toxoplasma gondii strain.

7. A transgenic-non pathogenic strain of Apicomplexan parasite, wherein the wild type locus of Sortilin is substituted by an artificial nucleotide sequence comprising:
i. 100 to 10000 bp, advantageously 150 to 4000 bp, more advantageously 1000 to 2000 bp contiguous nucleotide of a 5' non-coding and promoter regions of Sortilin of said Apicomplexan parasite,
ii. a selection gene cassette, and
iii. a sequence comprising 100 to 10000 bp, advantageously 150 to 4000 bp, more advantageously 1000 to 2000 bp of a 3' coding regions of Sortilin of said Apicomplexan parasite, said coding region being driven by a tetracycline-controlled transactivator.

8. A transgenic-non pathogenic strain of Toxoplasma gondii, wherein the wild type locus of Toxoplasma gondii Sortilin (TgSORTLR) is substituted by an artificial nucleotide sequence comprising:
i. a fragment of 2kb of TgSORTLR 5' non-coding and promoter regions, said sequence being SEQ ID: NO 2,
ii. a di hydrofol ate reductase gene cassette, and
iii. a fragment of 2kb of DNA coding region of the TgSORTLR, said sequence being SEQ ID:NO 4, said coding region being driven by an inducible TetO7-Sag 4 promoter.

9. A composition comprising the transgenic-non pathogenic strain of a Apicomplexan parasite as claimed in claim 7, or the transgenic-non pathogenic strain of Toxoplasma gondii as claimed in claim 8, and a non toxic and sterile pharmaceutically acceptable carrier.

## Patentansprüche

1. Nukleinsäurekonstrukt, das umfasst:
(i) eine Sequenz, die 100 bis 10000 bp, vorzugsweise 150 bis 4000 bp, bevorzugter 1000 bis 2000 bp zusammenhängender Nukleotide von 5' nicht codierenden und Promotorregionen von Sortilin eines Apicomplexa-Parasiten umfasst, insbesondere ausgewählt aus Toxoplasma gondii, Plasmodium falciparum, Babesia bovis, Cryptosporidium hominis oder Neospora caninum,
(ii) eine Selektionsgenkassette, wobei die Genkassette ihre eigenen 5' und 3' UTR flankierenden Sequenzen an beiden Seiten ihrer codierenden Sequenz hat,
(iii) eine Sequenz, die 100 bis 10000 bp, vorzugsweise 150 bis 4000 bp, bevorzugter 1000 bis 2000 bp zusammenhängender Nukleotide einer 3' codierenden Region von Sortilin eines Apikomplexa-Parasiten umfasst, wobei die Sequenz durch einen Anhydrotetracyclin- (ATc-) empfindlichen Transaktivator angesteuert wird.

2. Nukleinsäurekonstrukt nach Anspruch 1, wobei die 5' nicht codierende und Promotorregion von Sortilin ausgewählt ist aus einer Gruppe bestehend aus SEQ ID:NR 1, SEQ ID:NR 6, SEQ ID:NR 9, SEQ ID:NR 12, SEQ ID:NR 15 und SEQ ID:NR 18 und wobei die 3' codierende Region von Sortilin ausgewählt ist aus einer Gruppe bestehend aus SEQ ID:NR 3, SEQ ID:NR 7, SEQ ID:NR 10, SEQ ID:NR 13, SEQ ID:NR 16 und SEQ ID:NR 19.

3. Nukleinsäurekonstrukt nach Anspruch 1, wobei die Selektionsgenkassette eine Nukleinsäuresequenz von Dihydrofolat-Reductase EC 1.5.1.3 ist und/oder wobei der Anhydrotetracyclin- (ATc-) empfindliche Transaktivator aus TetO7-Sag1 oder TetO7-Sag4 ausgewählt ist.

4. Nukleinsäurekonstrukt, das umfasst:
(i) eine Sequenz aus 2000 bp zusammenhängender Nukleotide der 5' nicht codierenden und Promotorregionen von Sortilin von Toxoplasma gondii, wobei die Sequenz SEQ ID:NR 2 ist,
(ii) eine Dihydrofolat-Reductase-Genkassette, wobei die Genkassette ihre eigenen 5' und 3' UTR flankierenden Sequenzen an beiden Seiten ihrer codierenden Sequenz hat,
(iii) eine Sequenz aus 2000 bp zusammenhängender Nukleotide der 3' codierenden Regionen von Sortilin von Toxoplasma gondii, wobei die Sequenz SEQ ID:NR 4 ist und die Sequenz durch einen induzierbaren TetO7-Sag4-Promotor angesteuert wird.

5. Vektor, umfassend ein Nukleinsäurekonstrukt nach einem von Anspruch 1-4.

6. Verfahren zum Produzieren eines transgenen-nicht pathogenen Apicomplexa-Parasiten, insbesondere vom Toxoplasma gondii-Stamm, wobei das Verfahren umfasst:
(i) Bereitstellen eines Vektors wie in Anspruch 5 definiert;
(ii) Bereitstellen einer Arzneimittel-induzierbaren und hoch homologen Integration eines Apicomplexa-Parasiten transfizierter DNA, insbesondere vom Toxoplasma gondii-Stamm,
(iii) Transfizieren des Apicomplexa-Parasiten, insbesondere vom Toxoplasma gondii-Stamm, durch den Vektor,
(iv) Selektieren des transformierten Apicomplexa-Parasiten, insbesondere vom Toxoplasma gondii-Stamm, unter Verwendung eines zweiten Arzneimittels,
(v) Klonen des transformierten Apicomplexa-Parasiten, insbesondere vom Toxoplasma gondii-Stamm.

7. Transgener-nicht pathogener Stamm eines Apicomplexa-Parasiten, wobei der Wildtyp-Locus von Sortilin durch eine künstliche Nukleotidsequenz substituiert ist, umfassend:
i. 100 bis 10000 bp, vorzugsweise 150 bis 4000 bp, bevorzugter 1000 bis 2000 bp zusammenhängender Nukleotide von 5' nicht codierenden und Promotorregionen von Sortilin des Apicomplexa-Parasiten,
ii. eine Selektionsgenkassette und
iii. eine Sequenz, die 100 bis 10000 bp, vorzugsweise 150 bis 4000 bp, bevorzugter 1000 bis 2000 bp 3' codierender Regionen von Sortilin des Apikomplexa-Parasiten umfasst, wobei die codierende Region durch einen Tetracyclinkontrollierten Transaktivator angesteuert wird.

8. Transgener-nicht pathogener Stamm von Toxoplasma gondii, wobei der Wildtyp-Locus von Toxoplasma gondii Sortilin (TgSORTLR) durch eine künstliche Nukleotidsequenz substituiert ist, umfassend:
i. ein Fragment von 2 kb TgSORTLR 5' nicht codierenden und Promotorregionen, wobei die Sequenz SEQ ID:NR 2 ist,
ii. eine Dihydrofolat-Reductase-Genkassette und
iii. ein Fragment von 2 kb einer DNA codierenden Region des TgSORTLR, wobei die Sequenz SEQ ID:NR 4 ist, wobei die codierende Region durch einen induzierbaren TetO7-Sag4-Promotor angesteuert wird.

9. Zusammensetzung, umfassend den transgenen-nicht pathogenen Stamm eines Apicomplexa-Parasiten nach Anspruch 7 oder den transgenen-nicht pathogenen Stamm von Toxoplasma gondii nach Anspruch 8 und einen nicht toxischen und sterilen, pharmazeutisch annehmbaren Träger.

## Revendications

1. Construction d'acide nucléique qui comprend:
(i) une séquence comprenant de 100 à 10000 pb, avantageusement de 150 à 4 000 pb, plus avantageusement de 1000 à 2000 pb de nucléotides contigus d'une région 5' non codante et promotrice de Sortiline d'un parasite Apicomplexan, notamment choisis parmi Toxoplasma gondii, Plasmodium falciparum, Babesia bovis, Cryptosporidium hominis ou Neospora caninum,
(ii) une cassette de gène de sélection, ladite cassette de gène ayant ses propres séquences flanquantes 5' et 3'UTR des deux côtés de sa séquence codante,
(iii) une séquence comprenant de 100 à 10000 pb, avantageusement de 150 à 4000 pb, plus avantageusement de 1000 à 2000 pb de nucléotides contigus d'une région codante 3 'de Sortiline dudit parasite Apicomplexan, ladite séquence étant induite par un transactivateur sensible à l'anhydrotetracycline (ATc).

2. Construction d'acide nucléique selon la revendication 1, dans laquelle la région 5 'non codante et promotrice de Sortiline est choisie parmi un groupe constitué de SEQ ID: NO 1, SEQ ID: NO 6, SEQ ID: NO 9, SEQ ID: NO 12, SEQ ID: NO 15, et SEQ ID: NO 18, et dans laquelle la région 3' codante de Sortiline est choisie dans un groupe constitué de SEQ ID: NO 3, SEQ ID: NO 7, SEQ ID: NO 10, SEQ ID: NO 13, SEQ ID: NO 16, et SEQ ID: NO 19.

3. Construction d'acide nucléique selon la revendication 1, dans laquelle la cassette de gène de sélection est une séquence d'acide nucléique de la dihydrofolate réductase EC 1.5.1.3 et/ou dans laquelle le transactivateur sensible à l'anhydrotetracycline (ATc) est choisi parmi Tet07-Sagl ou Tet07-Sag4.

4. Construction d'acide nucléique selon la revendication 1, qui comprend:
(i) une séquence de 2000 nucléotides contigus des régions 5' non codante et promotrice de Sortiline de Toxoplasma gondii, ladite séquence étant SEQ ID: NO 2,
(ii) une cassette de gène de dihydrofolate réductase, ladite cassette de gène ayant sa propre séquence flanquante 5'et 3' UTR des deux côtés de sa séquence codante,
(iii) une séquence de 2000 nucléotides contigus de la région codante 3 'de Sortiline de Toxoplasma gondii, ladite séquence étant SEQ ID: NO 4, et ladite séquence étant induite par un promoteur inductible Tet07-Sag 4.

5. Un vecteur comprenant une construction d'acide nucléique selon l'une quelconque des revendications 1 à 4.

6. Procédé pour la production d'un parasite Apicomplexan transgénique non pathogène, notamment de la souche Toxoplasma gondii, dans lequel le procédé comprend:
(i) la fourniture d'un vecteur tel que défini dans la revendication 5,
(ii) la fourniture d'une intégration inductible par un médicament et une intégration hautement homologue de l'ADN transfecté d'un parasite Apicomplexan, notamment de la souche Toxoplasma gondii,
(iii) la transfection dudit parasite Apicomplexan, notamment de la souche Toxoplasma gondii, par ledit vecteur,
(iv) la sélection du parasite Apicomplexan transformé, notamment de la souche Toxoplasma gondii, en utilisant un second médicament,
(v) le clonage du parasite Apicomplexan transformé, notamment de la souche Toxoplasma gondii.

7. Une souche transgénique non pathogène du parasite Apicomplexan, dans laquelle le locus sauvage de Sortiline est substitué par une séquence nucléotidique artificielle comprenant:
i. de 100 à 10000 pb, avantageusement 150 à 4000 pb, plus avantageusement de 1000 à 2000 pb de nucléotides contigus d'une région 5' non codante et promotrice de Sortiline dudit parasite Apicomplexan,
ii. une cassette de gène de sélection, et
iii. une séquence comprenant de 100 à 10000 pb, avantageusement 150 à 4000 pb, plus avantageusement de 1000 à 2000 pb d'une région codante 3' de Sortiline dudit parasite Apicomplexan, ladite région codante étant induite par un transactivateur contrôlé par la tétracycline.

8. Une souche transgénique non pathogène de Toxoplasma gondii, dans laquelle le locus sauvage du récepteur de type Toxoplasma gondii Sortilin (TgSORTLR) est substitué par une séquence nucléotidique artificielle comprenant:
i. un fragment de 2 kb de la région de non-codante 5' et promotrice de TgSORTLR, ladite séquence étant SEQ ID: NO 2,
ii. une cassette du gène de la dihydrofolate réductase, et
iii. un fragment de 2 kb de région de codante d'ADN de TgSORTLR, ladite séquence étant SEQ ID: NO 4, ladite région codante étant induite par un promoteur Tet07-Sag 4 inductible.

9. Composition comprenant la souche transgénique non pathogène d'un parasite Apicomplexan selon la revendication 7, ou la souche transgénique non pathogène de Toxoplasma gondii selon la revendication 8, et un véhicule pharmaceutiquement acceptable non toxique et stérile.


### SEQUENCE LISTING

<110> Centre National de la Recherche Scientifique Université de Lille 2
   Tomavo, Stanislas
   Sloves, Pierre-Julien
   Mouveaux, Thomas
<120> CONDITIONAL KNOCKOUT MUTANTS OF SORTILIN-LIKE RECEPTOR IN APICOMPLEXAN PARASITES AND USES THEREOF
<130> IUS 11 BM CNR SORT
<160> 29
<170> PatentIn version 3.5
<210> 1
   <211> 10001
   <212> DNA
   <213> Toxoplasma gondii ME49
<400> 1
   cccgacggca gacttgactc tccttgactg ctgctgtgag gcaaatgaaa gaggagaaac      60
   gaaaaatcgg aaaccaggaa ggaaggcaga gaggagaaga cgagagagga acggacgaga     120
   ggcgaggtgg ggaagcgagc agaaacgagg agggggacag agagagacga gaaacgagac     180
   agagagaaga aacacgagga cgaaaagaat caaagatgca acggggaaaa cagaagtttt     240
   cactttcttc cgacttgatt tgcgcgtttt tcgtgttttc agtgatcccg aatttttaca     300
   atgaacgcga aagggaagat cttagacgca aacaggctct gcagcaagag cagcagatgg     360
   ccgtcgccag cgcagtgtcc gcgattcaag ctccgccagc gcctgctgct cctcctgctg     420
   ctcctgcctc tgcttcttct cctccttcct ctgctcttcc tgcgccttct tctgcagctc     480
   ttcttgctgt gcgcggccta ccagggcaaa atgggagaga cgaaaagcca aggacttctc     540
   tgcctcagac ccagggggcg tcgccgccga cacagcaggg acaaatgcct gcgcagcagt     600
   cctcgcaggg tgggtcggcg cctctgaggc cttcctggac ttttgctcag aggagtcaga     660
   ccagacaact cgcagacgta agcgagaggg gaggacagag agaagcggag gagaaagaag     720
   acgaagagag aagaagaaga agaagaagga aaggagagga aggagggaag agagaagaag     780
   aggaaggagg acagagagaa gcggaggaga aagaagacga agagagaagg gagagaggag     840
   aagctgaagc gaagacaggg aaggaaagaa ggggaggaac agaaagcagg aaaggcagag     900
   agacaaagag tgtagctcaa aaaaggagga gcagttgaag aagggcgtag gaagtggagg     960
   aaaaagaggt cgagggaacg gagttcgata gtgtgtcaag attctacggc gtgaaggagg    1020
   acgcctctct cttcctgtat gtattctcta cgtcgatgcg ggagcttcgt tctcgggagt    1080
   ctcttctgcg aaggaaacgc tgggagggtg attagtgaaa ctgttttttt tgtgtgtttt    1140
   cagataaaac tcgcttctca cgtctcggtc gttcatgaga ctggcgaaga tttctcctct    1200
   ctcgttcgtc gggtgcgtct ggagtagaat gagaagttca ccttttaaaa aagtttttga 1260
   aagacagtga caacacacct tgctgtgctc taagcagtcc ggggaatgat ggtgtccagc 1320
   agtcctacga cgtcgtctcg tcggttgtct gcgtttcgta acgggagacg tattcagtat 1380
   ccgaggcccc gcactacgcc ggtttcttcg acttgaggga ttcgcaggca aacggtttct 1440
   ccacgattac gcgcagttct ctgaactggc tcgtgaaaga cagttgggtc atgcgcttct 1500
   ctccactttc ttccgctgcc cgactctgaa ttcttcaact cagttgcctg gtatcaaatt 1560
   ccgtatcctg tgccagccta tctgaagttg tgttctcacg tcgcgcaccc ctaggaaagc 1620
   ttggcagttt ccctgtgttg tcttcgtagc tgacggcgcg atagaaggcg cctctcttgt 1680
   ttgcgtaaaa aaactgcaaa tttctgcgaa aggggagata gggggggggg agggggacga 1740
   agtcggcggg tgcttcaagg ttgaggcgaa tctgtgtttc tgtttctgtc acagatttcg 1800
   atcgaccgtc tcgaggaaga ggggaagaaa ataaacaaag aagcttctct gcaggagcct 1860
   cttcctaccg cctcggagtc gaagcgcgac atgtttgcag acagaattct caagcttctt 1920
   ctccgacctc tgatcactca gcaagaagtc tacgccactt tcaacacaga cgctcccttc 1980
   gcgatctcct ggaacgatgt aacatatctg tgcgatgtag taagaccgct gcctttcttc 2040
   atgatttcca tctgtttatc atatatactc catgcgaaga tgtactcgat gtagtaagaa 2100
   tgccacctat gtatatgtat acatacataa atatatatat atatctatat agatatagat 2160
   atatagatat agatatagat atagaagtgc atatatggtt gtttggagat gcatgcaggt 2220
   atgtgtgcac catgtgtctc tgtggccaaa tgaggaggtg aggaagaaag tttcgattcg 2280
   tctgtgtgtt gtctttcagg tgctggacat cgtgaagaac gacgacatgg tcttgcatct 2340
   ccgcgctcca atcaaaggtg agttgctgaa acagcgccga gggactttcc tgcaagactt 2400
   gtggctgcgc ttccaccttg cccgatccgt tacttttgtg gcgggacgca cagcgacggg 2460
   acctggtgta tggacgcccc tcttcggctc tcggtcgctc tctgtcttca ctgttcctcg 2520
   ttgcgtctgt gtctgcgttt cgcagtctac ggggatattc acggccagta ctacgacttg 2580
   atgcgactgt tccacttgta caaaatgcct gtcgaagaag aaagaagcga cgaattcgct 2640
   ggaacctctg gcggacaaca tggcgctgcg acgacgagcg ccgtcggaga catcgacaca 2700
   aacgactacc tgtttcttgg agactacgtc gaccgaggcc tcaacagcct ggaaaccatc 2760
   tgtctcctct tcgccctcaa agtatagagt cgctgcagct ctcctcccgt ctgaagctac 2820
   atggctcgcc gtctgcgctc ttgtcgatgt gttgtacatc tatccagaca tatacttata 2880
   tatatataaa tatatatata tatatatgta tatctttata gtagctatcc gtatgtctat 2940
   gtgtatgcgt ttattgtatg tctccacttg tgtgtatttc tgtgtctgac tctctatata 3000
   agatctacac atatgcacat acacatatat atatatatat gtcttcatcc atatatatat 3060
   atatatgtat atctacgtct ccaccttgcc atctgtgtgt gtatctgcat gcctgggtcg 3120
   acccatggac gtacgtgagc gtgtacgtcg gttcccgttt gcatttgcga cgttcctaca      3180
   gattttgctg atttcttgca ggcatttctc gaactgtcga gagaaaaatg cctccacgta      3240
   tatatacata tatatattta tatatttata tttatatata tacatattta tatatatata      3300
   tatatattta tgtcaatgta gatgcggaag gaaatgtgat ttgtggtttc aaatttaaca      3360
   taacgtggaa atgagcgttc tgtttttcag tcgaagcatc cgtatagtta catgtacatg      3420
   aagttcttgt gttttctagg tcaagagaaa gaacacatgt gcaggtattt tccgtttctt      3480
   ccaggtcaaa catatataca tatacagaca tatatatata tatatgcata tatatggatt      3540
   tatgcatgta gacgttgata tgtttctgtt ttttaggtaa agtatccacg tcagattcac      3600
   ctgctgcgcg ggaatcacga agatccggcg atcaattctt tgtacggatt tcaagacgag      3660
   tgcaaacggc gtttgcgcga agaccctttc gatccgagta gctgctggag gaagttcaac      3720
   ttggttttcg agtacttgcc cgtcgcggcc atcatcgacg attcaattct ctgcattcac      3780
   ggcggaattg gcggaagcat ttcttcggtc gaggagctgg cggcttttca gcggcctctc      3840
   aaagtcgcgc aggtcccgca aaatgtctac gtaaggcaaa acgtattcct tcacctttgg      3900
   agaattcatg caatctcctt cgaacgcaca gattcctgtg ataacgtacg tacctgcata      3960
   tgcatctata tgcgtattta tatatatata tatatataga tatagatata cgcgtgtgca      4020
   tgtccgtgtg ttttgagata tatgtgaatg ttcacgcatg gcgacatgta tgagtgtagt      4080
   tttgtgcgac agtgacgcgt ttgttcgatt ttcaggaaca gagagtcacc gatctgctct      4140
   ggtcagatcc gacagacaac gacagcatgc tgggtgtcgt gcgaaacgac atccgcgacc      4200
   ctgacggttc cgggagaatt tacaaattcg gtcccgaccg agtgcatcga tttctcgcgg      4260
   aaaacgacct tcaactcatc atcaggtcgg cgaatggaga aggcgctttt ctgcgtctgc      4320
   atgcgagcac gagacacatc cgtttgtctc tatgaacgcc agtgcgtgat ctgcgttgtt      4380
   cttcgaaccg cggatgcttg tgtttccaaa acaccctcac atatatatat atatatatat      4440
   atataatagt gaatatatgc cgcacataca cagtatatac acatatatat atatatatat      4500
   aattatcaat agccatgctg atcgccagct gtgaataagc agaacaggtt ggaggcatgc      4560
   atgtgtcgca cttttggtcc acgtgaatgc actacttggt ttttgtgtct ttcggacgtc       4620
   tgtgttgaag ttatttttca aataaattcc tgaatttttt cgtttttttc agagcacatg     4680
   agtgcgtgat ggacggcttt gagcgatttg cgggaggcaa gctgattact cttttctctg      4740
   gtaggtgtcg cgttcaggtt tttccttcgt tctttctttt gtatgtgagg tccctctctc      4800
   tgcttttctc tgtcttcttc tcttcctgcc tcccgcgcgg cttcgatccc tgcgcgaatg      4860
   gacgtggaga acctttctct cgacataaac ttacattcac atacctatat atatatatat      4920
   atatgtacat gtttgtatgt ggatgggtaa aagtgcataa gtgtttgtat atttgtagat      4980
   caaatcatag agtatctttt gaccgacctt tcatgaagcc aaagtgctat gaatgcgtgt 5040
   gtctatttat ctgcgtgcat gtgtattcac ctctgtagat atgtattaaa acctacattg 5100
   catgtatgta cctgttttct ggtgtaccta tgaagtccag acgcatgtgt tcgggaaccg 5160
   gcgtgtgtag gcgcatgcag acacctaagc atttcttttg tcgatttcca cgtttctctt 5220
   tcagcgacga actattgcaa ccaccatcag aacgcggggg cgcttctctt cattcggcga 5280
   gacatgacga tcatccccaa gttgatttat cctgcaaatg cgccttccca gtacaactcc 5340
   tggtgcgttc tctcttgcaa gaaaggcgcc tccacacaca aaaaaaacac acacagctca 5400
   gtcttgcaca gaaataccga gagccctgct tgggcgtata gaaacaaaca gaaatgccat 5460
   atatatatat atatatatat gtatatgtat gtgtatggat ccttactagt atttccgcct 5520
   gcacagacgc ttgtagatct gcacgtagga agagcggtag accactccgg tgtatatgca 5580
   gatggagaca gcgaatagag ggaggtctct agcgagaggt tgagacgcag aaaggtttac 5640
   gcagactagg tagctgtcag tagacaaata gaagagatct atgtggataa gacgagggat 5700
   aggaagctcg agaaagagag acttccgtgg gtggtcaaag aatgaggaag ccttgctgat 5760
   ggctttcgag agcgtctgtt cctttttctc tccagggatt cgcgcatggc ggaacttcgt 5820
   cctccgacgc cgcctcgcgc tgcaccgcgc atgcgagaga cagactttgg tgcggggggg 5880
   tgatttctcc gactctatct cctgcatttt ctctttcttg tttctctttc gcgtccctct 5940
   cggctcgaag gagagaaaag tttgctggaa tgcggaacgc atcttgcgtt cgatcgcaat 6000
   cttccttctt cacggttgtc tctttttcca ctgaacccgc atccgcgcat ttactatacg 6060
   tgtctatatt tctgtatatg ttccgcatat gtctgtctgt cactctatat acaagatata 6120
   gatgcatata gatatatctg gatacatttg gatatatatg ggcatatata tatatataca 6180
   tatatgtata tgtacatctt tctgtgtggg atctgttagc gcatgcgtga ggggaggcct 6240
   cgatgaaagt tggctgcttt cgcagaaact gctctttgtg aatttcgttg aattagggac 6300
   atcgaagagc ggttcgtctc tcgcatgtgt gcgccactct gcgttgagaa aacgcaggcg 6360
   ccggcttctt ccaaagcgaa gacagaggca gtgcgttgca tgcgtttttc gggtgcatcg 6420
   atgagccttt ctctgccgcg cgcgctctga actaggaaaa aagcgtctcc aatgcgggaa 6480
   ctgtgcaatt gcctcttgga gaaaacatga aaagccaagg cgcgagacag aggctcattt 6540
   gagacagagg ctgcgtctcg agttcactat ttcacacgac actctaaagg ccttccacgt 6600
   ttctcctgag cgtaaaattg gaacattgtc gtttcactcg cagttcacaa tgtatttttg 6660
   caagaaacac cacatttaca caactcacaa aggtgtgtgt atgtatcgac acatgtattt 6720
   atacacataa agatcaatct acatagatat ctacatgtat atatatatat atatataatg 6780
   tacgtacctg cattcgtgtg tgcatttgtc tctcgacgaa acaacgagtg agatctgcct 6840
   ctgtaaatgt gtacagacga cagacgtatg cagctgatcc ttaagtcacg gatatacata 6900
   tacatccata tatatatata tacatatata tatgttaaat ttaaaaaaat gagggctatt 6960
   taggacgaga tatcgaactc tgagtttttt ttctgtagac tgcgtgcgtt tccctgtgcc 7020
   tgacgagacc gattcgaagg aatcgttgtg cacttctccc gcgcttgcac atctcgaaag 7080
   cattgctttg tgtgaatcga gcagtgcttg tcgccttcct ctctccccac gcgagctctg 7140
   ttttcaagag ggtgcgagag tgaagtgcta agaggctttt gacgccacaa gagtcttgtc 7200
   gcctctttcg taaaattccc cggaaggtga catcgtcttt gtgcttcata tttccgcgtt 7260
   ttctttgcga ttcgggattc gcggttcttt cttgcttcgt ctgctcagag aggtccctcc 7320
   cccacttcgc ttcttcgttc ctaagcaaag caccgaacgg cagacagtag acttaaacat 7380
   tgaggtgaaa aacctgtctt tacttgaaaa gaatcgcgag gatatctacg cgtcgttcag 7440
   aaaaagagag atccccgtag acgtgctaag tctgtgccat ctgatccaaa gtggaaatgc 7500
   gtggcactca tgcggagaca caaagaacag aaacgagtcg ctgcgggttt gaaggtggtg 7560
   aatgaaccag tgctcgcctt tatctcgcaa gcgcttcgac acgcactgtg caagactcgc 7620
   aacgcactgg agaaaacctc gactgttttc tggaaagctc agaggaaaaa gtgcagcaga 7680
   cggtctgtgg atctgcgtct caaagaatac caaaggaaag agggattaaa gaagaggaaa 7740
   aaatcagaaa gggacaaagg aagcgaggga gacagcaaga aaagggggag tgtgcctctg 7800
   cgtcttgtga agggaagaaa aaggggtatg aaaacaacga ggaaacaaag agagagtgtg 7860
   tgtgtatctg tgtctacgaa gaaagcagga aatgtcgtga cctatgccac tgaggtgcac 7920
   agactgaaca agattgacat tctggaaagg caagaagcca cactctctcc cttctcgttt 7980
   ttcctccgag atttgtgcga tatgtcatca ggagcccctc tgccttgaga acgctacaca 8040
   cagagaatag agaagaaaaa tgcgtcgagt ccggcttctc catgcctttc tttggaacac 8100
   ttctcccgaa gaaatctgtt tctcctatcg caagcttctc ccgcggccgc tcacttggtg 8160
   tgatgcattt tgcgcgacac gaggaactct ttgtaaaaga actagaggca cgtccgcatt 8220
   cgtttcactg ccacgagctc tttcagatgg cagagacgag aaagcagaca cttccaccgg 8280
   ggaagaattt gctgtgtgtt catcctgccc aagcctggct ttcctcccct gatcattagc 8340
   ctcttctccc ctccccttgg tttgccgtta aggcgagaaa cgaacgagag attcacagaa 8400
   aggcggcacc cacgtgtttt gtggagtgta catacacccc atgtgtttgt caggcttcgg 8460
   tgagctgtca gttgttgcct ctttcctcgc gatttgctgg aactttctgg ccacacgaga 8520
   cctcggggaa aagcgactcc ggagaaactt cgtcgcctgg cccgatcgcg cctccgctgg 8580
   ctaacaactt ctttcgtttc cgcattctta gtttctttca tggattcgag agctcgaaac 8640
   tggcacctgc caagcacgga gttgcggtgt atatacaccg caaatcatgt gcaagtttgt 8700
   cgcttttggc gcgttgaagg gcgcagctcg cacacctggg gctggacagg ttagaagtct 8760
   tgagagaggc tgcctggaag cctgttttct ggaattttgt tttctcgaaa gtcttctttt      8820
   ctgtcgcgtt cgcgcgtttc tctgttcttg ggctgctagc ggtatttttc agcggctctg      8880
   gggatcgcaa gtcgctcgct gttttctcgg cggcaccttt ctcgcggctc tccccggggg      8940
   gagggggaga cgcgtgtgcg ctgtctttgc tttttttctg cagtttttcc cctttttttt      9000
   gtctgccgca gtcgtcgcga atctttcctt ttccgggact tcgcagccga ggttccactg      9060
   ctttctgccg cattcagacg gtgcgtcaag cacttctttc cctcctcaga gagaagcttg      9120
   tctttctgtg caagcgaggg cacgcgggcg cacggtctcg aagagtcagt tgtcggaaaa      9180
   gcggagactc gctctgtttt cttctcatct gtcgccagcg aagacattcc tttcccttcg      9240
   tcctccacgg cgcctgtctt cgctcttcgc cgcgcctcgc tgtctgcacc gccgggcgca      9300
   gggaggatcg gcgagactcc cgtccttggc tattccctct cttcacacaa aactcctttc      9360
   ggactctcct accttctcgg atcgccgctc tctcgtgcgc gacgccgcgt ctgtctttgc      9420
   cggactctac acacactcgc aacccccaca cgggacgcgt tttcgtaaca caggagactc      9480
   ctaggtcttg catgccagtc tgccttttcc acgccgtaga ccaccggttt ctgcgtcttc      9540
   gtccctccct gagccgtcgc ccgcgtttct cggatttcgg ccgatttcca gacgcacact      9600
   gtctcccttc gccgctctcg ttttctcagc gaagcaaagg actccgttct tgtcgccggt      9660
   ctctcagctg aagcagctgc ctcgagaaga gacgcaccag gagagacagc gaaactgaga      9720
   gaagaaggga caagcttctc gaatctagcg aacgcacagg cttcgcggcg cgcccagtct      9780
   tctggtgtat gtacacctcg cgcacaccgc atggcggcct ccgtcgttgg cctttgtctc      9840
   ggtcgtcagt gagcgccgtc gccagtcgcg tgttgtctcg ttcagttgct tttcaccggc      9900
   gactcgcctc gttcctgctc tcgaggaaga agacacctct gtgtgccctc agctcccgtc      9960
   catccggtct gcaggcctcc tccggtgtac agacagtcac c                         10001
<210> 2
   <211> 2000
   <212> DNA
   <213> Toxoplasma gondii ME49
<400> 2
   atgtcatcag gagcccctct gccttgagaa cgctacacac agagaataga gaagaaaaat 60
   gcgtcgagtc cggcttctcc atgcctttct ttggaacact tctcccgaag aaatctgttt 120
   ctcctatcgc aagcttctcc cgcggccgct cacttggtgt gatgcatttt gcgcgacacg 180
   aggaactctt tgtaaaagaa ctagaggcac gtccgcattc gtttcactgc cacgagctct 240
   ttcagatggc agagacgaga aagcagacac ttccaccggg gaagaatttg ctgtgtgttc 300
   atcctgccca agcctggctt tcctcccctg atcattagcc tcttctcccc tccccttggt 360
   ttgccgttaa ggcgagaaac gaacgagaga ttcacagaaa ggcggcaccc acgtgttttg 420
   tggagtgtac atacacccca tgtgtttgtc aggcttcggt gagctgtcag ttgttgcctc 480
   tttcctcgcg atttgctgga actttctggc cacacgagac ctcggggaaa agcgactccg 540
   gagaaacttc gtcgcctggc ccgatcgcgc ctccgctggc taacaacttc tttcgtttcc 600
   gcattcttag tttctttcat ggattcgaga gctcgaaact ggcacctgcc aagcacggag 660
   ttgcggtgta tatacaccgc aaatcatgtg caagtttgtc gcttttggcg cgttgaaggg 720
   cgcagctcgc acacctgggg ctggacaggt tagaagtctt gagagaggct gcctggaagc 780
   ctgttttctg gaattttgtt ttctcgaaag tcttcttttc tgtcgcgttc gcgcgtttct 840
   ctgttcttgg gctgctagcg gtatttttca gcggctctgg ggatcgcaag tcgctcgctg 900
   ttttctcggc ggcacctttc tcgcggctct ccccgggggg agggggagac gcgtgtgcgc 960
   tgtctttgct ttttttctgc agtttttccc cttttttttg tctgccgcag tcgtcgcgaa 1020
   tctttccttt tccgggactt cgcagccgag gttccactgc tttctgccgc attcagacgg 1080
   tgcgtcaagc acttctttcc ctcctcagag agaagcttgt ctttctgtgc aagcgagggc 1140
   acgcgggcgc acggtctcga agagtcagtt gtcggaaaag cggagactcg ctctgttttc 1200
   ttctcatctg tcgccagcga agacattcct ttcccttcgt cctccacggc gcctgtcttc 1260
   gctcttcgcc gcgcctcgct gtctgcaccg ccgggcgcag ggaggatcgg cgagactccc 1320
   gtccttggct attccctctc ttcacacaaa actcctttcg gactctccta ccttctcgga 1380
   tcgccgctct ctcgtgcgcg acgccgcgtc tgtctttgcc ggactctaca cacactcgca 1440
   acccccacac gggacgcgtt ttcgtaacac aggagactcc taggtcttgc atgccagtct 1500
   gccttttcca cgccgtagac caccggtttc tgcgtcttcg tccctccctg agccgtcgcc 1560
   cgcgtttctc ggatttcggc cgatttccag acgcacactg tctcccttcg ccgctctcgt 1620
   tttctcagcg aagcaaagga ctccgttctt gtcgccggtc tctcagctga agcagctgcc 1680
   tcgagaagag acgcaccagg agagacagcg aaactgagag aagaagggac aagcttctcg 1740
   aatctagcga acgcacaggc ttcgcggcgc gcccagtctt ctggtgtatg tacacctcgc 1800
   gcacaccgca tggcggcctc cgtcgttggc ctttgtctcg gtcgtcagtg agcgccgtcg 1860
   ccagtcgcgt gttgtctcgt tcagttgctt ttcaccggcg actcgcctcg ttcctgctct 1920
   cgaggaagaa gacacctctg tgtgccctca gctcccgtcc atccggtctg caggcctcct 1980
   ccggtgtaca gacagtcacc 2000
<210> 3
   <211> 10001
   <212> DNA
   <213> Toxoplasma gondii ME49
<400> 3
   atggcgcaca gcttcgcacg aggccgccca ccggcggccg gccgaacact cgcggttttc 60
   cttgcttttc tcgcagttct ttcgccttcg cttctgccgg gattcttcag tgaagacgcg      120
   cagaagcgtg aatcctcagt cctcgtcgcc aacgctgcgt cctacgcaaa caaaaagggt      180
   acgtcttcag aggaacacaa gtgcctcttt tctcatgcag gaagtcggag gccgtagaac      240
   acggtttctc gtcttctctc tagttctgtt gcaagaaaga atgcgtgctc gacacacgtg      300
   gagtcgcagc gctttcgaag gacaatagtg tctgcgattt gaggtgtcta tacacttctg      360
   catccgtata gaggggttat cttccgtttg tcagcatgag agacggaatt tgtcgtattc      420
   agaacttcag tggaggtggc ttagagcccc ggaatcgagg cgagttgatg ttttagaaat      480
   ggagaaaggc tttcttttgg gggaagacga atctcaggaa gtagggtatc gcgttgatgc      540
   tgtcagttgc gtttatcctc ttgcgtttct tttttgcgtc gtgcctgaag cttccacttc      600
   ttcgctctct ttcactcttt ttgctctcta cgtatcttct tgtatggtct tctggtctct      660
   gctctttgcc ttccaagttg ccctctttct cgctattttc tctttttttc ttgtcctctg      720
   tttttcctct tgcattctcc gttcctcttg ttttgatctg tccccattcg tctcgtttct      780
   catctcttct ctgtcatggt gctgatgctt cttggtcttc actttttctt tccccctccc      840
   gcgccccacc tcctctcgtt ggccgtttgc gctggctcca cctccgttcg cttctccgtc      900
   tccgcgcgtc cgaacgtcgc ggctgcgcct gtgggtttgt gtttgcgctg cccgtgcagt      960
   ctcggtgtcc gaggtttcgt tcgacagtca catcgaggac attcagtggt gcggcaccga     1020
   ccaccggacg attttgttga aaacgcggcg ggggcggctg taccgttcgc aggacggagg     1080
   caagtcctgg acggaaatca cggatctgct caagtcgtcg gaggcggcca ccggcactgt     1140
   ggcggtcgac tccatcatcg tgtcgccagt ggacaagcgc gtcgtgttga ttgtggggtc     1200
   caagcgaaac cacttcatct ctgaggacag cgccgcgacg ttccgccggc tcaagtacaa     1260
   gaacacgatt cacaacttcc acttccaccc cacgcggccc aagtacgcga ttctgtcgac     1320
   ctggacggac gcatgctact cgggcagcgg caccgcgagc cgcgcgcaga gccagcaaga     1380
   ctgcaaccac cagctcttct acacgcggga cctgggtcgc tccttcaaac tcgtagccga     1440
   ctacgtcgtc cagttctcct ggggagacaa gaagctcgga aacaccgacc atatcttctt     1500
   cacgcagcac cgcggaagat ccggagatca gccccgatac ggcggatggg tgcgaagtcg     1560
   aaaaacagac ggaaaaaaag agaaaaaaag agaaaaaaag agcaaagaag aagggagaag     1620
   aaggaagaga aagagaaaag aagaagaagg aagagaaaga ggaagagaaa gagaagaaac     1680
   gtagggtggg tgaaggaagg agccagatgt ggggggtttc tggggggaga cagaccggtg      1740
   cagagagagg gagagagaga gatacgcaga cacccagaaa ggaagagaaa ccgttggagc     1800
   tagacagagg aacttggtga gatacaaaac agagatagcc agacagagga gatgtccgtt     1860
   tttacacgca ccttggttgt gtgtgttgtg tttttcgtag tctaaaaacg tggatctgat     1920
   gtacactccg gactttggag cgaccatcac gcgtctggtc taccgcggaa acaagttcct     1980
   tttgtcgaat ggatatttct tcgttgcgaa agtgaaagac gccgccaagc aaacggtctc 2040
   tctcctcgtg tccacggatg gaggcaagtc attccaggta ggtctttctt ccgtcctcct 2100
   cgcttctccc gttctctctc tcctcatgcc gcgctctctt ctcgtccggt gggggaggag 2160
   tgtagacgga tcttgacgag cagcgtctgg cgcctttgag acgcagaaat ctaggagagc 2220
   tctgctgcgg tctccagtgt gcagttcctg cttcccacgc agccacacgc ccattcttga 2280
   atttattttt gcagatacct gtcgaattgc atgtccgtgg acatacgccc atcgagaggc 2340
   ccgcggaggt gcatgaccat ttgggatgct tctgaggacc tttcctcgtc tcgacttgtg 2400
   caatgtgctc atatacctat ctctacatgt atatatctct gcacacccac atgcatatac 2460
   atacaacttc ttatatatgt atatgcatat atatttatat atatatatat atttatatat 2520
   atattcgcgt gaatgtgagt gtctgcgtgt ttcctttttc aagatttccg gagggtgaaa 2580
   gtgacgctct tgtcttttcc atttcgtcgg tgtcttgtgc agatggccaa gctgccggtt 2640
   gaaatagagg agaggagtta cacggtcttg gatacgtctg aagacgcgat tatgttgcat 2700
   gtgaatcacg gccacgacaa cgtaagatct ttggaagcaa cttgtcttcc aggcgtcatc 2760
   tacggattcg tgttctcact cgtaataaca acgagagaag acgattgcgc aggggttcat 2820
   ttgaccagga ccaccgtcct aggagtcatc atctctgcaa caggattttt tcatttgtcc 2880
   atcaagtgtt tgcacggatt tttatttttt cttcatgaaa ctcgaatatt cttcctaaag 2940
   atccccgaaa gactgagcca tgcgcaccat ttcgaagaaa ctgacaagta ctcactgtcg 3000
   agctgatggc gagtgtttgt ggttgtctgc ttcgaagtca aatttgaaga gcgtccatgg 3060
   ccacacacac acatacatac aggcaggcat acatacataa catacataca tacatacttg 3120
   catacataac atacatacat acataacata catacataca tacagacgcg tcgtccgttt 3180
   cctgctggac acgacaaagt gctccagaga gcggtgtata tacaccggac attttcctcc 3240
   ttgaaacaca cacatgcgta cctcaaagtc gcgcgaggga gagagcccgt ttattgccgc 3300
   gtcgcgtttg ctttttctcc tggagcgttc gttttctttt ttccagtttt gcaagtcttc 3360
   acgagctcgt tccgcgttct ccggttttcg ttgttcgctt ccctcagaag ggtgacacag 3420
   gcaacgtgta catttcggac gcgaagggtg tccgatactc gctgtcgctg ccgaacaaca 3480
   tccgcaccag caccggagag tgcgagttcg acaaagtgct ttctctcgag ggtgtgtact 3540
   tggcgaactt caaggactca gtcgactcgt ctgcgtccgt ggacggggga cagcaaggcg 3600
   acctggagaa gttggaggag gagatcgaag aggaggccga aggcgtccag gtggacctcg 3660
   agaagaagca caaaagcgtc gctacgcgga gccgtcagga agaggtgatt cgcacagtga 3720
   tctccttcga caaaggcggc gtttggtcgt atctgaaggc tccgaaagtc gacagcaggg 3780
   gacagaagat cgactgtccg cccgatcgct gctggcttca cttgaatgga atcacgcgct 3840
   tcagcgactt cgcacccttc tactcagtcg agaacgccgt cggaatcatc atgggcactg 3900
   gaaacgtcgg cagctatctc aggcctgaaa aagacgaggt gcgtgtttca agtggaaaca 3960
   gcgagaaggg gaggccggag ccagaaacaa gatggcgcgc aaacagaact ggatgcggag 4020
   acaaagaaga gaaacgcaga gggatagaga agaagagaac aaggagaaga gaaaggaaaa 4080
   acggagagaa caagaggagg agaacgagag gcgacgggtg aagggaggag gccaaagggg 4140
   aagaatgaga ggagaagcca agactaggaa agatcgtgta aagaaagaca tctttcccca 4200
   cagatgttct tccgattgca tgctgcttgt cgtctcttcg ttttcctcag gcaaacactt 4260
   acctctctcg agatggagga gtctcgtgga tcgaagctca caaaggagct ttcatctacg 4320
   aaatgggtga ccatggcggc ttgttagtca tggccgacga cacgaagaaa acgaatcagg 4380
   tgcgacgcgt tcgtctcttt gcagttgttc ttcttcccct tcaaagactt caattccgct 4440
   tgtgttctcc gtcttgattt ctttgcaaaa ttgttctttt ctttctgtcg ctctttgctt 4500
   tctctcgagt tcgccgtacc gcttctcttc tgaggtctgt gactctcgaa gcgcgtgtct 4560
   ctttttttcc gcgtatccta tccaccttct ggctttttcc aaaggcggaa gtctgtgcga 4620
   agataccgac tttccacgtt gggacagagg actccatttg tatccgattt ttgactctct 4680
   tccatttctc tttctgatct tgtgcgtttg ctgcttgagg cattcgttgt tttttctcac 4740
   ctgcgttgta gcatctctat cgagtcttga aaaatagcga ctctctgcag ttcctactct 4800
   gctgaaattc actcttgttg tctaaagaaa tggcgcgggt gcctgttctc tcctcgaagt 4860
   ccatctgcat cgtcagaccc gttgttagtt gccgtcgagt ttcccccgtg tagactcgca 4920
   cgagcgagaa aacaaagata gactccagaa cgctgcaagt tgtcagtaga aaactgtgaa 4980
   ttctggattt tctgctgcgc atcgaaaagc agacgtggat ggctgtcgga aggagactcg 5040
   aacgttttaa tttcaggtcg tcttcagctg gaacgaggga cagagctggt acgacttcga 5100
   gctgggtgcg gcgccgcttt tcgtcgacaa catcgtcatc gagccgaatg cgtcaagtgt 5160
   cgagtttctt ctctacggca agcgagagca agactccgca ggtcggaaaa cagcgcctcc 5220
   cgtggcaacg cacgtcgaaa cagacgagaa ggggagacag agagagagga gaagagagag 5280
   cagttcttca aaattcactt ccagagaaac gcgcagctaa aactctcgag tcgggagcag 5340
   agtctccaaa atcttgcaaa tcggaaagga atgcaagaca cacacaaatg tggacaaaca 5400
   gtgcgatata tatatgtata tatatatata tatatatata tgcatatgtc tacagagagg 5460
   cgagggaagc aagggaagga agctctttgg tgttgatttc gatggaaatg cttttttgcg 5520
   gcagcagaat cggaagagaa ggctctgtcg cggcggtttt cttctgcttt acattttttg 5580
   ttttcaggtg ttctatttca cctcgacttc aacgcgctga accagcagca gtgcaaaggc 5640
   atttgggccg cagactccgt cagcagcgac tacgaaacgt ggagtccttc gggtgcgcga 5700
   actggaccgc tcgcagctga agcagcggaa tgaacaaaag atctagtccc gtcgcatctg 5760
   aaaagtatat atatatatat atatatgtat atatatacat gtatatatat atatatatat 5820
   atgtatgcgt gtacaggaag acagggggaa gaattgcgtt cgatatgcgc atgtgcgtcc 5880
   gacaaacgcg tctatgagca tgtatcaata catcattatg catgtgcaca aatgcaggga 5940
   aaaggaggag actgtgaact ttgttttctt tcctcagatg gccgcgccgg tggcgagaga 6000
   tgcattttgg ggaagcatat cacgtacacc cggagaaagc aaacgagcga gtgcttcaac 6060
   ggccgagact ttgaccgccc gaaagtttcc aaggtacctt tttctctcgt caattcatgt 6120
   acagcgaacc gtttccatga ttctccgcat aaaccacgtc acctgttacg ccttctcttg 6180
   ccgcgaattc taccttctga gaggactggc agacgaacgg tcactctcta ctcgttcctc 6240
   catcaacgcc ccccatctcc gtttgtcaaa agtccagatc tcgcgaaaga gcgagtcttt 6300
   ctgtagacac tttgaacaag cgacgggggt ggttctctgt gcttgccagt ctcccagcgt 6360
   agtaatgcct acgcacacat gcacgctgtg tgtatcctag ggtcacaaag agaaattctc 6420
   acctgtttcg atctccctct gtatgtgtag ttatacagat atctatacat ctagatattt 6480
   ttttatatat gcatatacat atatgtatgc gtatatgtaa tatatattta tatacataca 6540
   tatatttata tgcatacata tacatatata tatatatata tatatatata tatatatata 6600
   tatatgctca attatttatc ttgtatctgt ggacatgcct ggatctaggg acacatgggg 6660
   acgtgcgtgt ttttctctgc atgtgaagga gtttgcttga aaacacttta tatatatata 6720
   tatatttata tatatgtata tttatatatt tatatatata tatatattta tttatattta 6780
   tattatagat tgatgcgtct ccgtagaaat gtctatacgc aggaccacct gtttgttttt 6840
   agataaaagt gaaagtgttg aaaataacgt ttttcagtac cgttcgcttg tctctctgag 6900
   atgtttttca tgactttttg tgagttttcc gcgatttttt caggtgtgtc cgtgcactat 6960
   ggaggactat gagtgcgagt ttggcttcac gagagccatc gggtccaccc agtgcgtggc 7020
   gaccgacgca gcagcagccg ccgccgcgac agcgactggc ctcgcgcagt tcgccgacga 7080
   atcagacgcc gcagccgccg ccgcatgcac ctcctcgtct ttcttctaca cctctgccta 7140
   tcgaaaagta agtcgaaaaa cgagaaattg gagaaaaggg agagcagaga gaaggggaga 7200
   aagcgtagtg gagggagaac gaggggggcg gaagagggag ggcaagggag aagagagaca 7260
   gaaaagaaga ggtagggaaa agagtacttg gagatcgctc tcaagaaggg caccagagtt 7320
   tgtggctgca tgcagagaga agaacgcgtg atgagagagt ggagaaaact tcgttggtct 7380
   cctttccttc caggttccgg gagatgtctg cgagggcggc tggatgcccg agaaggtcgc 7440
   tgtgccttgt cccgcccatt cacccgtcag tcgaggagga aagacagtgt tgcttcttct 7500
   tctctttatc gtcgtcgtca tggtcgtcat caactatctg gcgaaaactg gaagactgaa 7560
   gaagttcttc cgtaacgcag gttcgcgaaa cctcttgttg acagttctgt ctcgtgttat 7620
   cgtgtttcca tttcctcctt tctgtccgtc gctcgtccac gcgttgtctc ttccttattt 7680
   cttctggctc cgtctttgcg tctgtgtctt cctgtcgccc tttccgcgtc ttgtctctcg 7740
   agttctgtct ctctctcctt atctttatcc ttctgttcct ctctctggat ctctcgtctg 7800
   cttcctcttc ctctctgttt ttcgttgtcc ttatctcttc cttctgcctt tctccctgtc 7860
   ggtctggagc tccacgcgcg tctgccctgg agtcgccacg tagccatttt gctcttcttt 7920
   tcttgctctt ttctcttcgt ttaggtttcg acagctttgc aaatgtctct tacggccttg 7980
   tcggcgcctc cgcgggaggg cctggcgggt ggctcgacca ggaagctggt gaaagtcgaa 8040
   ggggagaaga acttggagaa agatcaaaat acgaaccgga actgggcttc atcgaagccg 8100
   aacaggtgtg cacctctccc tcgacacggt agcgtttttc tccgtttctc aatgtctgcc 8160
   tgtgtcgata taatcttgtc tttcgatgtc tatccgcatc tgccttacct gtccgtctgt 8220
   ttggatctct gtgtgtatgc ctacgtctat tcatctgtgt gtacctgggc ctctgtgagt 8280
   ctgtgtacat cgacctatta tgtaagcgag ttgtagatgc cgtctacctg aggacctcag 8340
   cttctactct gctgcattca gcaccgttct cggcgatgta tcgatatgtt cttcaatcca 8400
   tttcccaaag gcgttttgga tgctcgaaag cggacatttc tccagttttt aaagaaactt 8460
   ttttctgcaa acttttgtcg ggagggaagg tcgtgctttt ccgcgctgca ctggacctta 8520
   tcctgcagtg gaatttttca aaaaattctg tttttcagga cgagaacgaa gaagacgcgc 8580
   cgacgctgat gaactacggc aacgcagcag gcggtcaaag aacttccggc atgtcttcgc 8640
   ggtcgccgaa accgacagaa gatttcgaac tcgacgattc acggcccctc ttcccctccc 8700
   atgtcagctc tcgcgaaact caaggaagct ccggtcttca ccctcgctcg acttcgacag 8760
   aaaacgaacc tgtatgtgtc ttgtagacgc tcgactccgg gtgtcccgtg cgtcaagcat 8820
   taacggcagc ggattcggct tatattcggc gactccacag cttatttagt acacacccac 8880
   ccacacagtg tacacatacc gcactgaaac tggggtagtg tccgcgtctc tcgttacggt 8940
   gccttcgcgg agtcgcagtt ttcctcttca caggtgcatc cgagggttgc ctctctctgc 9000
   tctctgagca tcgattcttt ttcagttatc gagtggtgct ttggtaggaa agtggagaga 9060
   gatcgacgag atacagaacc ggttatatat atatatatat aatgttgtat aggctacagt 9120
   acatatatga agtatgccgt ctacttcatt atatatatat atatatatat atgttttatt 9180
   tgaaatacag ggtcgtatat agagtacaca acctagttca atcactttta tatttatata 9240
   tttatatata tatatatata tatgcatttg tatgtcgggg cctgcagaga ggagagaggc 9300
   gttttgaggc ggccgggttt gagaagacgg aagcgaagga agagagtgga tgttttcttg 9360
   aattccagaa agaccggatg ttccgttttt agtcgacgtg gaagtccgtt caccctctcc 9420
   ctttttttgc tgagcaggag acgtctcttg tcgaatgaaa cgactcgggt tcacgctgtc 9480
   cggttttctg tgcagattcc gcgtctggcg ccgccgcgat tcgacgagga taacgtcgaa 9540
   cttctttgaa aacgtctttt cttctttctt ctttctccgc cgtcttgcag ctctgtagga      9600
   gcttccgccg aagacgactt tctttcgtgt ctcgcatgtc tttctcgtgt gtcgtctctg      9660
   aaaagaacgt ggtggaccgg ggcggttcga ccggggagag agagtgctct ttcttttgtg      9720
   cctgcaaagg aaatcttcga gcgaaggaag acgcgaagag aaaacggaga agaagttcgc      9780
   gaggatggag aggaagcgcg gtgtctgcga gtgtccagag gtcggaaagc gaacaggttt      9840
   cgcggcagtc gaggatcagt cgaaggaatc gaggaagagc cgagaggaag gaacgcgagg      9900
   aaacgaagaa ctcgactctt tttctcccga cgcggaagca gacgagaacg aatggggaaa      9960
   agaagaagag gcgcctttgg tcggcagacg cgtcttcgca c                         10001
<210> 4
   <211> 2000
   <212> DNA
   <213> Toxoplasma gondii ME49
<400> 4
   atggcgcaca gcttcgcacg aggccgccca ccggcggccg gccgaacact cgcggttttc      60
   cttgcttttc tcgcagttct ttcgccttcg cttctgccgg gattcttcag tgaagacgcg     120
   cagaagcgtg aatcctcagt cctcgtcgcc aacgctgcgt cctacgcaaa caaaaagggt     180
   acgtcttcag aggaacacaa gtgcctcttt tctcatgcag gaagtcggag gccgtagaac     240
   acggtttctc gtcttctctc tagttctgtt gcaagaaaga atgcgtgctc gacacacgtg     300
   gagtcgcagc gctttcgaag gacaatagtg tctgcgattt gaggtgtcta tacacttctg     360
   catccgtata gaggggttat cttccgtttg tcagcatgag agacggaatt tgtcgtattc     420
   agaacttcag tggaggtggc ttagagcccc ggaatcgagg cgagttgatg ttttagaaat     480
   ggagaaaggc tttcttttgg gggaagacga atctcaggaa gtagggtatc gcgttgatgc     540
   tgtcagttgc gtttatcctc ttgcgtttct tttttgcgtc gtgcctgaag cttccacttc     600
   ttcgctctct ttcactcttt ttgctctcta cgtatcttct tgtatggtct tctggtctct     660
   gctctttgcc ttccaagttg ccctctttct cgctattttc tctttttttc ttgtcctctg      720
   tttttcctct tgcattctcc gttcctcttg ttttgatctg tccccattcg tctcgtttct     780
   catctcttct ctgtcatggt gctgatgctt cttggtcttc actttttctt tccccctccc     840
   gcgccccacc tcctctcgtt ggccgtttgc gctggctcca cctccgttcg cttctccgtc     900
   tccgcgcgtc cgaacgtcgc ggctgcgcct gtgggtttgt gtttgcgctg cccgtgcagt     960
   ctcggtgtcc gaggtttcgt tcgacagtca catcgaggac attcagtggt gcggcaccga    1020
   ccaccggacg attttgttga aaacgcggcg ggggcggctg taccgttcgc aggacggagg     1080
   caagtcctgg acggaaatca cggatctgct caagtcgtcg gaggcggcca ccggcactgt    1140
   ggcggtcgac tccatcatcg tgtcgccagt ggacaagcgc gtcgtgttga ttgtggggtc    1200
   caagcgaaac cacttcatct ctgaggacag cgccgcgacg ttccgccggc tcaagtacaa      1260
   gaacacgatt cacaacttcc acttccaccc cacgcggccc aagtacgcga ttctgtcgac      1320
   ctggacggac gcatgctact cgggcagcgg caccgcgagc cgcgcgcaga gccagcaaga      1380
   ctgcaaccac cagctcttct acacgcggga cctgggtcgc tccttcaaac tcgtagccga      1440
   ctacgtcgtc cagttctcct ggggagacaa gaagctcgga aacaccgacc atatcttctt      1500
   cacgcagcac cgcggaagat ccggagatca gccccgatac ggcggatggg tgcgaagtcg      1560
   aaaaacagac ggaaaaaaag agaaaaaaag agaaaaaaag agcaaagaag aagggagaag      1620
   aaggaagaga aagagaaaag aagaagaagg aagagaaaga ggaagagaaa gagaagaaac      1680
   gtagggtggg tgaaggaagg agccagatgt ggggggtttc tggggggaga cagaccggtg      1740
   cagagagagg gagagagaga gatacgcaga cacccagaaa ggaagagaaa ccgttggagc      1800
   tagacagagg aacttggtga gatacaaaac agagatagcc agacagagga gatgtccgtt      1860
   tttacacgca ccttggttgt gtgtgttgtg tttttcgtag tctaaaaacg tggatctgat      1920
   gtacactccg gactttggag cgaccatcac gcgtctggtc taccgcggaa acaagttcct      1980
   tttgtcgaat ggatatttct                                                  2000
<210> 5
   <211> 962
   <212> PRT
   <213> Toxoplasma gondii ME49
<400> 5
   Met Ala His Ser Phe Ala Arg Gly Arg Pro Pro Ala Ala Gly Arg Thr
   1               5                   10                  15
   Leu Ala Val Phe Leu Ala Phe Leu Ala Val Leu Ser Pro Ser Leu Leu
               20                  25                  30
   Pro Gly Phe Phe Ser Glu Asp Ala Gln Lys Arg Glu Ser Ser Val Leu
           35                  40                  45
   Val Ala Asn Ala Ala Ser Tyr Ala Asn Lys Lys Val Ser Val Ser Glu
       50                  55                  60
   Val Ser Phe Asp Ser His Ile Glu Asp Ile Gln Trp Cys Gly Thr Asp
   65                  70                  75                  80
   His Arg Thr Ile Leu Leu Lys Thr Arg Arg Gly Arg Leu Tyr Arg Ser
                   85                  90                  95
   Gln Asp Gly Gly Lys Ser Trp Thr Glu Ile Thr Asp Leu Leu Lys Ser
               100                 105                 110
   Ser Glu Ala Ala Thr Gly Thr Val Ala Val Asp Ser Ile Ile Val Ser
           115                 120                 125
   Pro Val Asp Lys Arg Val Val Leu Ile Val Gly Ser Lys Arg Asn His
       130                 135                 140
   Phe Ile Ser Glu Asp Ser Ala Ala Thr Phe Arg Arg Leu Lys Tyr Lys
   145                 150                 155                 160
   Asn Thr Ile His Asn Phe His Phe His Pro Thr Arg Pro Lys Tyr Ala
                   165                 170                 175
   Ile Leu Ser Thr Trp Thr Asp Ala Cys Tyr Ser Gly Ser Gly Thr Ala
               180                 185                 190
   Ser Arg Ala Gln Ser Gln Gln Asp Cys Asn His Gln Leu Phe Tyr Thr
           195                 200                 205
   Arg Asp Leu Gly Arg Ser Phe Lys Leu Val Ala Asp Tyr Val Val Gln
       210                 215                 220
   Phe Ser Trp Gly Asp Lys Lys Leu Gly Asn Thr Asp His Ile Phe Phe
   225                 230                 235                 240
   Thr Gln His Arg Gly Arg Ser Gly Asp Gln Pro Arg Tyr Gly Gly Trp
                   245                 250                 255
   Ser Lys Asn Val Asp Leu Met Tyr Thr Pro Asp Phe Gly Ala Thr Ile
               260                 265                 270
   Thr Arg Leu Val Tyr Arg Gly Asn Lys Phe Leu Leu Ser Asn Gly Tyr
           275                 280                 285
   Phe Phe Val Ala Lys Val Lys Asp Ala Ala Lys Gln Thr Val Ser Leu
       290                 295                 300
   Leu Val Ser Thr Asp Gly Gly Lys Ser Phe Gln Met Ala Lys Leu Pro
   305                 310                 315                 320
   Val Glu Ile Glu Glu Arg Ser Tyr Thr Val Leu Asp Thr Ser Glu Asp
                   325                 330                 335
   Ala Ile Met Leu His Val Asn His Gly His Asp Asn Lys Gly Asp Thr
               340                 345                 350
   Gly Asn Val Tyr Ile Ser Asp Ala Lys Gly Val Arg Tyr Ser Leu Ser
   355 360 365
   Leu Pro Asn Asn Ile Arg Thr Ser Thr Gly Glu Cys Glu Phe Asp Lys
   370 375 380
   Val Leu Ser Leu Glu Gly Val Tyr Leu Ala Asn Phe Lys Asp Ser Val
   385 390 395 400
   Asp Ser Ser Ala Ser Val Asp Gly Gly Gln Gln Gly Asp Leu Glu Lys
   405 410 415
   Leu Glu Glu Glu Ile Glu Glu Glu Ala Glu Gly Val Gln Val Asp Leu
   420 425 430
   Glu Lys Lys His Lys Ser Val Ala Thr Arg Ser Arg Gln Glu Glu Val
   435 440 445
   Ile Arg Thr Val Ile Ser Phe Asp Lys Gly Gly Val Trp Ser Tyr Leu
   450 455 460
   Lys Ala Pro Lys Val Asp Ser Arg Gly Gln Lys Ile Asp Cys Pro Pro
   465 470 475 480
   Asp Arg Cys Trp Leu His Leu Asn Gly Ile Thr Arg Phe Ser Asp Phe
   485 490 495
   Ala Pro Phe Tyr Ser Val Glu Asn Ala Val Gly Ile Ile Met Gly Thr
   500 505 510
   Gly Asn Val Gly Ser Tyr Leu Arg Pro Glu Lys Asp Glu Ala Asn Thr
   515 520 525
   Tyr Leu Ser Arg Asp Gly Gly Val Ser Trp Ile Glu Ala His Lys Gly
   530 535 540
   Ala Phe Ile Tyr Glu Met Gly Asp His Gly Gly Leu Leu Val Met Ala
   545 550 555 560
   Asp Asp Thr Lys Lys Thr Asn Gln Val Val Phe Ser Trp Asn Glu Gly
   565 570 575
   Gln Ser Trp Tyr Asp Phe Glu Leu Gly Ala Ala Pro Leu Phe Val Asp
   580 585 590
   Asn Ile Val Ile Glu Pro Asn Ala Ser Ser Val Glu Phe Leu Leu Tyr
   595 600 605
   Gly Lys Arg Glu Gln Asp Ser Ala Gly Val Leu Phe His Leu Asp Phe
       610                 615                 620
   Asn Ala Leu Asn Gln Gln Gln Cys Lys Gly Ile Trp Ala Ala Asp Ser
   625                 630                 635                 640
   Val Ser Ser Asp Tyr Glu Thr Trp Ser Pro Ser Asp Gly Arg Ala Gly
                   645                 650                 655
   Gly Glu Arg Cys Ile Leu Gly Lys His Ile Thr Tyr Thr Arg Arg Lys
               660                 665                 670
   Gln Thr Ser Glu Cys Phe Asn Gly Arg Asp Phe Asp Arg Pro Lys Val
           675                 680                 685
   Ser Lys Val Cys Pro Cys Thr Met Glu Asp Tyr Glu Cys Glu Phe Gly
       690                 695                 700
   Phe Thr Arg Ala Ile Gly Ser Thr Gln Cys Val Ala Thr Asp Ala Ala
   705                 710                 715                 720
   Ala Ala Ala Ala Ala Thr Ala Thr Gly Leu Ala Gln Phe Ala Asp Glu
                   725                 730                 735
   Ser Asp Ala Ala Ala Ala Ala Ala Cys Thr Ser Ser Ser Phe Phe Tyr
               740                 745                 750
   Thr Ser Ala Tyr Arg Lys Val Pro Gly Asp Val Cys Glu Gly Gly Trp
           755                 760                 765
   Met Pro Glu Lys Val Ala Val Pro Cys Pro Ala His Ser Pro Val Ser
       770                 775                 780
   Arg Gly Gly Lys Thr Val Leu Leu Leu Leu Leu Phe Ile Val Val Val
   785                 790                 795                 800
   Met Val Val Ile Asn Tyr Leu Ala Lys Thr Gly Arg Leu Lys Lys Phe
                   805                 810                 815
   Phe Arg Asn Ala Gly Phe Asp Ser Phe Ala Asn Val Ser Tyr Gly Leu
               820                 825                 830
   Val Gly Ala Ser Ala Gly Gly Pro Gly Gly Trp Leu Asp Gln Glu Ala
           835                 840                 845
   Gly Glu Ser Arg Arg Gly Glu Glu Leu Gly Glu Arg Ser Lys Tyr Glu
       850                 855                 860
   Pro Glu Leu Gly Phe Ile Glu Ala Glu Gln Asp Glu Asn Glu Glu Asp
   865                 870                 875                 880
   Ala Pro Thr Leu Met Asn Tyr Gly Asn Ala Ala Gly Gly Gln Arg Thr
                   885                 890                 895
   Ser Gly Met Ser Ser Arg Ser Pro Lys Pro Thr Glu Asp Phe Glu Leu
               900                 905                 910
   Asp Asp Ser Arg Pro Leu Phe Pro Ser His Val Ser Ser Arg Glu Thr
           915                 920                 925
   Gln Gly Ser Ser Gly Leu His Pro Arg Ser Thr Ser Thr Glu Asn Glu
       930                 935                 940
   Pro Ile Pro Arg Leu Ala Pro Pro Arg Phe Asp Glu Asp Asn Val Glu
   945                 950                 955                 960
   Leu Leu
<210> 6
   <211> 10001
   <212> DNA
   <213> Toxoplasma gondii GT1
<400> 6
   gaaaaatcgg aaaccaggaa ggaaggcaga gaggagaaga cgagagagga acggacgaga      60
   ggcgaggtgg ggaagcgagc agaaacgagg agggggacag agagagacga gaaacgagac     120
   agagagaaga aacacgagga cgaaaagaat caaaggtgca acgggggaaa cagaagtttt     180
   cactttcttc cgacttgatt tgcgcgtttt tcgtgttttc agtgatcccg aatttttaca     240
   atgaacgcga aagggaagat cttagacgca aacaggctct gcagcaagag cagcagatgg     300
   ccgtcgccag cgcagtgtcc gcgattcaag ctccgccagc gcctgctgct cctcctgctg     360
   ctcctgctgc tcctgcctct gcttcttctc ctccttcctc tgctcttcct gcgccttctt     420
   ctgcagctct tcttgctgtg cgcggcctac cagggcaaaa tgggagagac gaaaagccaa     480
   ggacttctct gcctcagacc cagggggcgt cgccgccgac acagcaggga caaatgcctg     540
   cgcagcagtc ctcgcagggt ggatcggcgc ctctgaggcc ttcctggact tttgctcaga     600
   ggagtcagac cagacaactc gcagacgtaa gcgagagggg aggacagaga gaagcggagg     660
   agaaagaaaa cgaagagaga agaagaagga aaggagagga aggagggaag agagaagaag     720
   aaggaaagga gaggaaggag ggaagagaga agaagaggaa ggaggacaga gagaagcgga     780
   ggagaaagaa gagagaaggg agagaggaga agctgaagcg aagacaggga aggaaagaag     840
   gggaggaaca gaaagcagga aaggcagaga gacaaagagt gtagctcaaa aaaggaggag 900
   cagttgaaga agggcgtagg aagtggagga aaaagaggtc gagggaacgg agttcgatag 960
   tgtgtcaaga ttctacggcg tgaaggagga cgcctctctc ttcgtgtatg tactctctac 1020
   gtcgatgcgg gagcttcgtt ctcgggagtc tcttctgcga aggaaacgct gggagagtga 1080
   tcagtgaaac tttttttttt gtgttttcag ataaaactcg cttctcacgt ctcggtcgtt 1140
   catgagactg gcgaagattt ctcctctctc gttcgtcggg tgcgtctgga gtagaatgag 1200
   aagttcacct tttaaaaaag tttttgaaag acagtgacaa cacaccttgc tgtgctctaa 1260
   gcagtccggg gaatgatggt gtccagccgt cctacgacgt cgtcgcgtcg gttgtctgcg 1320
   tttcgtaacg ggagatgtat tcagtatccg aggccccgca ctacgccggt ttcttcgact 1380
   tgagggattc gcaggctaac gttttctcca cgattacgcg cagttctctg aactggctcg 1440
   tgaaagacag ttgggtcatg cgcttctctc cactttcttc cgctgtccga ctctgaattc 1500
   ttcaactcag ttgcctggta tcaaattccg tatcctgtgc cagcctatct gaagttgtgt 1560
   tctcgcgtcg cgcaccccta ggaaagcttg gcagtttccc tgtgttgtct ttgtagctga 1620
   cggcgcgata gaaggcgcct ctcttgtttg cgtaaaaaaa ctgcaaattt ctgcgaaagg 1680
   ggagacaggg gggggggagg gggacgaagt cggcgggtgc ttcaaggttg aggcgaatct 1740
   gtgtttctgt ttctgtcaca gatttcgatc gaccgtctcg aggaagaggg gaagaaaata 1800
   aacaaagaag cttctctgca ggagcctctt cctaccgcct cggagtcgaa gcgcgacatg 1860
   tttgcagaca gaattctcaa gcttcttctc cgacctctga tcactcagca agaagtctac 1920
   gccactttca acacagacgc tcccttcgcg atctcctgga acgatgtaac atatctgtgc 1980
   gatgtagtaa gaccgctgcc tttcttcatg atttccatct gtttatcata tatactctat 2040
   gcgaagatgt acttgatgta gtaagaatgc cacctatgta tatgtataca taaatatata 2100
   tatatatata tatatagata tagatataga tatagatata gaagtgcata tgtggatgtt 2160
   tggagatgca tgcaggtatg tgtgcaccat gtgtctctgt ggcccaatga ggaggtgagg 2220
   aagaaagttt cgattcgtct gtgtgttgtc tttcaggtgc tggacatcgt gaagaacgac 2280
   gacatggtct tgcatctccg cgctccaatc aaaggtgagt tgctgaaaca gcgcagaggg 2340
   actttcctgc aagacttgtg gctgcgcttc caccttgccc gatgcgttac ttttgcggcg 2400
   ggacgcacag cgacgggacc tggtgtatgg acgcccctct tcggctctcg gtcgctctct 2460
   gtcttcactg ttcctcgttg cgtctgcatc tgcgtttcgc agtctacggg gatattcacg 2520
   gccagtacta cgacttgatg cgactgttcc acttgtacaa aatgcctgtc gaagaagaaa 2580
   gaagcgacga attcgctgga acctctggcg gacaacatgg cgctgcgacg acgagcgccg 2640
   tcggagacat cgacacaaac gactacctgt ttcttggaga ctacgtcgac cgaggcctca 2700
   acagcctgga aaccatctgt ctcctcttcg ccctcaaagt atagagtcgc tgcagctctc 2760
   ctcccgtctg aagctacatg gctcgccgtc tgcgctcttg tcgatgtgtt gtacatctat 2820
   ccagacatat acttatatat atatatatat atatatatat atatctttat agtagctatc 2880
   cgtatgtcta tgtgtatgcg tttattgtat gtctccactt gtgtgtattt ctgtgtctga 2940
   ctctctacat aagatctaca catatgcaca tacacatata tatatatata tatatgtatg 3000
   tcttcatcca tatatatata tatatatata tatatatatg tatatctacg tctccacctt 3060
   gccatctgtg tgtgtatctg catgcctggg tcgacccatg gacgtacgtg agcgtgtacg 3120
   tcggttcccg tttgcatttg cgacgttcct acagattttg ctgatttctt gcaggcattt 3180
   ctcgaactgc cgagagaaaa atgcctccac atatatatat atatttatat atttgtattt 3240
   atatatatac atatttatca tatttatata catattttta tatatatata tatatatatt 3300
   tatttatgtc aatgtagatg cggaaggaaa tgtgatttgt ggtttcaaat ttaacataac 3360
   gtgaaaatgg gcgttctgtt tttcagtcga agcatccgta tagttacatg tacatgaagt 3420
   tcttgtgttt tctaggtcaa gagaaagaac acatgtgcag gtattttccg tttcttccag 3480
   gtcaaacata tatacatata cagacatata tatatatatg catatatatg gatttatgca 3540
   tgtagacgtt gatatgtttc tgttttttag gtaaagtatc cacgtcagat tcacctgctg 3600
   cgcgggaatc acgaagatcc ggcgatcaat tctttgtacg gatttcaaga cgagtgcaaa 3660
   cggcgtttgc gcgaagaccc tttcgatccg agtagctgct ggaggaagtt caacttggtt 3720
   ttcgagtact tgcccgtcgc ggccatcatc gacgattcaa ttctctgcat tcatggcgga 3780
   attggcggaa gcatttcttc ggtcgaggag ctggcggctt ttcagcggcc tctcaaagtc 3840
   gcgcaggtcc cgcaaaacgt ctacgtaagg caaaacgtat tccttcacct ttggagaatt 3900
   catgcaatct ccttcgaacg cacagattcc tgtgataacg tacgtacctg catatgcatc 3960
   tatatgtgta tttatatata tatatatata cgcgtgtgca tgtccgtgtg ttttgagata 4020
   tatgtggatg ttcacgcatg gcgacatgta tgagtgtagt tctgtgcgac agtgacgcgt 4080
   ttgttcgatt ttcaggaaca gagagtcacc gatctgctct ggtcagatcc gacagacaac 4140
   gacagcatgc tgggtgtcgt gcgaaacgac atccgcgacc ctgacggttc cgggagaatt 4200
   tacaaattcg gtcccgaccg agtgcatcga tttctcgcgg aaaacgacct tcaactcatc 4260
   atcaggtcgg cgaatggaga aggcgctttt ctgcgtctgc atgcgagcac gagacacatc 4320
   cgtttgtctc tatgaacgcc agtgcgtgat ctgcgttgtt cttcgaaccg cggatgcttg 4380
   tgtttccaaa acaccctcac atatatatat atataatagt gaatatatgc cgcacataca 4440
   cagtatatat atatatatat atatatatat atatatgtat aattatcaat agccatgctg 4500
   atcgccagct gtgaataagc agaacaggtt ggaggcatgc atgtgtcgca cttttggtcc 4560
   acgtgaatgc actacttggt ttttgtgtct ttcggacgtc tgtgttgaag ttatttttca 4620
   aataaattcc tgaatttttt cgtttttttc agagcacatg agtgcgtgat ggacggcttt 4680
   gagcgatttg cgggaggcaa gctgattact cttttctctg gtaggtgtcg cgttcaggtt 4740
   tttccttcgt tctttctttt gtgtgtgagg tccctctctc tgcttttctc tgtcttcttc 4800
   tcttcctgcc tcccgcgcgg cttcgatccc tgcgcgaatg gacgtggaga acctttctct 4860
   cgacataaac ttacattcac atacctatat atatatatat gtacatgttt gtatgtggat 4920
   gggtaaaagt gcataagtgt ttgtatattt gtagatcaaa tcatagagta tcttttgacc 4980
   gacctttcat gaagccaaag tgctatgaat gcgtgtgtct atttatctgc gtgcatgtgt 5040
   attcacctct gtagatatgt attaaaacct acatcgcatg tatgtacctg ttttctggtg 5100
   tacctatgaa gtccagacgc atgtgttcgg gaaccggcgt gtgtaggcgc atgcagacac 5160
   ctaagcattt cttttgtcga tttccacgtt tctctttcag cgacgaacta ttgcaaccac 5220
   catcagaacg cgggggcgct tctcttcatt cggcgagaca tgacgatcat ccccaagttg 5280
   atttatcctg caaatgcgcc ttcccagtac aactcctggt gcgttctctc ttgcaagaaa 5340
   ggcgcctcca cacacaaaaa aaacacacac agctcagtct tgcacagaaa taccgagagc 5400
   cctgcttggg cttatagaaa caaacagaaa tgccatatat atatatatgt atatatatat 5460
   atgtatatat atatatatgt atatgtatgt gtatggatcc ttactagtat ttccgcctgc 5520
   acagacgctt gtagatctgc acgtaggaag agcggtagac cactccggtg tatatgcaga 5580
   tggagacagc gaatagaggg aggtctctag cgagaggttg agacgcagaa aggtttacgc 5640
   agactaggta gctgtcagta gacaaataga agagatctat gtggataaga cgagggatag 5700
   gaagctcgag aaagagagac ttccgtgggt ggtcaaagaa tgaggaagcc ttgctgatgg 5760
   ctttcgagag cgtctgttcc tttttctctc cagggattcg cgcatggcgg aacttcgtcc 5820
   tccgacgccg cctcgcgctg caccgcgcat gcgagagaca gactttggtg cgggggggtg 5880
   atttctccga ctctatctcc tgcattttct ctttcttgtt tctctctcgc gtccctctcg 5940
   gctcgaagga gagaaaagtt tgctggaatg cggaacgcat cttgcgttcg atcgcaatct 6000
   tccttcttca cggttgtctc tttttccact gaacccgcat ccgcgcattt actatacgtg 6060
   tctatatttc tgtatatgtt ccgcatatgt ctgtctgtca ctctatatac aagatataga 6120
   tgcatataga tatatctgga tacatttgga tatatatggg catatatata tatatataca 6180
   tatatgtata tgtacatatt tctgtgtggg atctgttagc gcatgcgtga ggggaggcct 6240
   cgatgaaagt tggctgcttt cgcagaaact gctctttgtg aatttcgttg aattagggac 6300
   atcgaagagc ggttcgtctc tcgcatgtgt gcgccactct gcgttgagaa aacgcaggcg 6360
   ccggcttctt ccaaagcgaa gacagaggca gtgcgttgca tgcgtttttc gggtgcatcg 6420
   atgagccttt ctctgccgcg cgcgctctga actaggaaaa aagcgtctcc aatgcgggaa 6480
   ctgtgcaatt gcctcttgga gaaaacatga aaagccaagg cgcgaggcag aggctcattt 6540
   gagacagagg ctgcgtctcg agttcactat ttcacacgac actctaaagg ccttccacgt 6600
   ttctcctgag cgtaaaattg gaacattgtc gtttcactcg cagttcacaa tgtatttttg 6660
   caagaaacac cacatttaca caactcacaa aggtgtgtgt atgtatcgac acatgtattt 6720
   atacacataa agatcaatct acatagatat ctacatgtat atatatatat atatataatg 6780
   tacgtacctg cattcgtgtg tgcatttgtc tctcgacgaa acaacgagtg agatctgcct 6840
   ctgtaaatgt gtacagacga cagacgtatg cagctgatcc ttaagtcacg gatatacata 6900
   tacatccata tatatatata tacatatata tatgttaaat taaaaaaaat gagggctatt 6960
   taggacgaga tatcgaactc tgagtttttt ttctgtagac tgcgtgcgtt tccctgtgcc 7020
   tgacgagacc gattcgaagg aatcgttgtg cacttctccc gcgcgtgcac atctcgaaag 7080
   cattgctttg tgtgaatcga gcagtgcttg tcgccttcct ctctccccac gcgagctctg 7140
   ttttcaaaag ggtgcgagag tgaagtgcta agaggctttt gacgccacaa gagtcttgtc 7200
   gcctctttcg taaaattccc cggaaggtga catcgtcttt gtgtctcata tttccgcgtt 7260
   ttctttgcga ttcgggattc gcggttcttt cttgcttcgt ctgctcagag aggtccctcc 7320
   cccacttcgc ttcttcgttc ctaagcaaag caccgaacgg cagacagtag acttaaacat 7380
   taaggtgaaa aacctgtctt tacttgaaaa gaatcgcgag gatatctacg cgtcgttcag 7440
   aaaaagagag atccccgtag acgtgctaaa tctgtgccat ctgatccaaa gtggaaatgc 7500
   gtcgcactca tgcggagaca caaagaacag aaacgagtcg ctgcgggttt gaaggtggtg 7560
   aatgaaccag tgctcgcctt tatctcgcaa gcgcttcgac acacactgtg caagactcgc 7620
   aacgcactgg agaaaacctc gactgttttc tggaaagctc agaggaaaaa gtgcagcaga 7680
   cggtctgtgg atctgcgtct caaagaatac caaaggaaag agggattaaa gaaaaggaaa 7740
   aaatcagaaa gggacaaagg aagcgaggga gacagcaaga aaagggggag tgtgcctctg 7800
   cgtcttgtga agggaagaaa aaggggtatg aaaacaacga ggaaacaaag agagagtgtg 7860
   tgtgtatctg tgtctacgaa gaaagcagga aatgtcgtgc cctatgccac tgaggtgcac 7920
   agacagaaca agattgacat tctggaaagg caagaagcca cactctctcc cttctcgttt 7980
   ttcctccgag atttgtgcga catgtcatca ggagcccctc tgccttgaga acgctacaca 8040
   cagagaatag agaagaaaaa tgcgtcgagt ccggcttctc catgcctttc tttggaacac 8100
   ttctcccgaa gaaatctgtt tctcctatcg caagcttctc ccgcggccgc tcacttggtg 8160
   tgatacattt tgcgcgacac gaggaactct ttgtaaaaga actagaggca cgtccgcatt 8220
   cgtctcactg ccacgagctc tttcagatgg cagagacgag aaagcagaca cttccaccgg 8280
   ggaagaattt gctgtgtgtt catcctgccc aagcctggct ttcctcccct gatcattagc 8340
   ctcttctccc ctccccttgg tttgccgtta aggcgagaaa cgaacgagag attcacagaa 8400
   aggcggcacc cacgtgtttt gtggagtgta catacacccc atgtgtttgt caggcttcgg 8460
   tgagctgtca gttgttgcct ctttcctcgc gatttgctgg aactttctgg ccacacgaga 8520
   cctcggggaa aagcgactcc ggagaaactt cgtcgcctgg cccgatcgcg cctccgctgg 8580
   ctaacaactt ctttcgtttc cgcattctta gtttctttca tggattcgag agctcgaaac 8640
   tggcacctgc caagcacgga gttgcggtgt atatacaccg caaatcatgt gcaagtttgt 8700
   cgcttttggc gcgttgaagg gcgcagctcg cacacctggg gctggacagg ttagaagtct 8760
   tgagagaggc tgcctggaag cctgttttct ggaattttgt tttctcgaaa gtcttctttt 8820
   ctgtcgcgtt cgcgcgtttc tctgttcttg ggctgctagc ggtatttttc agcggctctg 8880
   gggatcgcaa gtcgctcgct gttttctcgg cggcaccttt ctcgcggctc tccccggggg 8940
   gagggggaga cgcgtgtgcg ctgtctttgc tttttttctg cagtttttcc cctttttttt 9000
   gtctgccgca gtcgtcgcga atctttcctt ttccgggact tcgcagccga ggttccactg 9060
   ctttctgccg cattcagacg gtgcgtcaag cacttctttc cctcctcaga gagaagcttg 9120
   tctttctgtg caagcgaggg cacgcgggcg cacggtctcg aagagtcagt tgtcggaaaa 9180
   gcggagactc gctctgtttt cttctcatct gtcgccagcg aagacattcg tttcccttcg 9240
   tcctccacgg cgcctgtctt cgctcttcgc cgcgcctcgc tgtctgcacc gccgggcgca 9300
   gggaggatcg gcgagactcc cgtccttggc tattccctct cttcacacaa aactcctttc 9360
   ggactctcct accttctcgg atcgccgctc tctcgtgcgc gacgccgcgt ctgtctttgc 9420
   cggactctac acacactcgc aacccccaca cgggacgcgt tttcgtaaca caggagactc 9480
   ctaggtcttg catgccagtc tgccttttcc acgccgtaga ccaccggttt ctgcgtcttc 9540
   gtccctccct gagccgtcgc ccgcgtttct cggatttcgg ccgatttcca gacgcacact 9600
   gtctcccttc accgctgtcg ttttctcagc gaagcaaagg actccgttct tgtcgccggt 9660
   ctctcagctg aagcagctgc ctcgagaaga gacgcaccag gagagacagc gaaactgaga 9720
   gaagaaggga caagcttctc gactctagcg aacgcacagg cttcgcggcg cgcccagtct 9780
   tctggtgtat gtacacctcg cgcacaccgc atggcggcct ccgtcgttgg cctttgtctc 9840
   ggtcgtcagt gagcgccgtc gccagtcgcg tgttgtctcg ttcagttgct tttcaccggc 9900
   gactcgcctc gttcctgctc tcgaggaaga agacacctct gtgtgccctc agctcccgtc 9960
   catccggtct gcaggcctcc tccggtgtac agacagtcac t 10001
<210> 7
   <211> 10001
   <212> DNA
   <213> Toxoplasma gondii GT1
<400> 7
   atggcgcaca gcttcgcacg aggccgccca ccggcggccg gccgaacact cgcggttttc 60
   cttgcttttc tcgcagttct ttcgccttcg cttctgccgg aattcttcag tgaagacgcg 120
   cagaagcgtg aatcctcagt cctcgtcgcc aacgctgcgt cctacgcaaa caaaaagggt 180
   acgtcttcag aggaacgcaa gtgcctcttt tctcatgcag gaagtcggag gccgtagaac 240
   acggtttctc gtcttctctc tagttctgtt gcaagaaaga atgcgtgctc gacacacgtg 300
   gagtcgcagc gctttcgaag gacaatagtg tctgcgattt gaggtgtcta tgcacttctg 360
   catccgtata gaggggttct cttccgtttg ccagcatgag agacggaatt tgtcgtattc 420
   agaacttcag tggaggtggc ttagagcccg ggaatcgagg cgagttgatg ttttagaaat 480
   ggagaaaggc tttcttttgg gggaagacga atctcaggaa gtagggtatc gcgttgatgc 540
   tgtcagttgc gtttatcctc ttgcgtttct tttttgcgtc gtgcctgaag cttccacttc 600
   ttcgctctct ttcactcttt ttgctctcta cgtatcttct tgtatggtct tctggtctct 660
   gctctttgtc ttccaagttg ccctctttct cgctattttc tctttttttc ttgtcctctg 720
   tttttcctct tgcattctcc gttcctcttg ttttgatctg tccccattcg tctcgtttct 780
   catctcttct ctgtcatggt gctgatgctt cttggtcttc actttttctt tccccctccc 840
   gcgccccact ctcctctcgt tggccggttg cgctggctcc acctccgttc gcttctccgt 900
   ctccgcgcgt ccgaacgtcg cggctgcgcc tgtgggtttg tgtttgcgct gcccgtgcag 960
   tctcggtgtc cgaggtttcg ttcgacagtc acatcgagga cattcagtgg tgcggcaccg 1020
   accaccggac gattttgttg aaaacgcggc gggggcggct gtaccgttcg caggacggag 1080
   gcaagtcctg gacggaaatc acggatctgc tcaagtcgtc ggaggcggcc accggcactg 1140
   tggcggtcga ctccatcatc gtgtcgccag tggacaagcg cgtcgtgttg attgtggggt 1200
   ccaagcgaaa ccacttcatc tctgaggaca gcgccgcgac gttccgccgg ctcaagtaca 1260
   agaacacgat tcacaacttc cacttccacc ccacgcggcc caagtacgcg attctgtcga 1320
   cctggacgga cgcatgctac tcgggcagcg gcaccgcgag ccgcgcgcag agccagcaag 1380
   actgcaacca ccagctcttc tacacgcggg acctgggtcg ctccttcaaa ctcgtagccg 1440
   actacgtcgt ccagttctcc tggggagaca agaagctcgg aaacaccgac catatcttct 1500
   tcacgcagca ccgcggaaga tccggagatc agccccgata cggcggatgg gtgcgaagtc 1560
   gaaaaacaga cggaaaaaaa gagaaaaaaa gagcaaagaa gaagggagaa gaagaaggga 1620
   gaaggaagag aaagagaaaa gaagaagaag gaagagaaag aggaagagaa gaaacgtagg 1680
   gtgggtgaag gaagaaggag ccagatgtgg ggggtttctg gggggagaca gaccggtgca 1740
   gagagaggga gagagagaga gagggagaga gagagatacg cagacaccca gaaaggaaga 1800
   gaaaccgttg gagctagaca gaggaacttg gtgagataca aaacagagat agccagacag 1860
   aggagatgtc cgtttttaca cgcactttgg ttgtgtgcgt tgtgtttttc ctagtctaaa 1920
   aacgtggatc tgatgtacac tccggacttt ggagcgacca tcacgcgtct ggtctaccgc 1980
   ggaaacaagt tccttttgtc gaatggatat ttcttcgttg cgaaagtgaa agacgccgcc 2040
   aagcaaacgg tctctctcct cgtgtccacg gatggaggca agtcattcca ggtaggtctt 2100
   tcttccgtcc tcctcgcttc tcccgttctc tctctcctca tgccgcgctc tcttctcgtc 2160
   cggtggggga ggagtgtaga cggatcttga cgagcagcgt ctggcgcgtt tgagacgcag 2220
   aaatctagga gagctctgct gcggtctcca gtgtgcagtt cctgcttccc acgcagccac 2280
   acgcccattc ttgaatttat ttttgcagat acctgtcgaa ttgcatgtcc gtggacatac 2340
   gtccatcgag aggcccgcgg aggtgcatga ccatttggga tgcttctgag gacctttcct 2400
   cgtctcgact tgtgaaatgt gctcatatac ctatctctac atgtatatat ctctgcacac 2460
   ccacatgcat atacatacaa cttcctatat atgtatatgc atatatattt atatatatat 2520
   atatatgtat atattcgcgt gaatgtgagt gtctgcgtgt ttcctttttc aagatttccg 2580
   gagggtgaaa gtgacgctct tgtcttttcc atttcgtcgg tgtcttctgc agatggccaa 2640
   gctgccggtt gaaatagagg agaggagtta cacggtcttg gatacgtctg aagacgcgat 2700
   tatgttgcat gtgaatcacg gccacgacaa cgtaagatct ttggaagcaa cttgtcttcc 2760
   aggcgtcatc tacggattcg tattctcact cgtaataaca gcgagagaag acgattgcgc 2820
   aggggttcat ttgaccagga ccaccgtcct aggagtcatc atctctgcaa caggattttt 2880
   tcatttgtcc atcaagtgtt tgcacggatt tttatttttt cttcataaaa ctcgaatatt 2940
   cttcctaaag atccccgaaa gactgagcca tgcgcaccat ttcgaagaaa ctgacaagta 3000
   ctcactgtcc agctgatggc gagtgtttgt ggttgtctgc ttcgaagtca aatttgaaga 3060
   gcgtccatgg ccacacacac acatacatac aggcatacat acatacataa catacataca 3120
   tgcatacata catacataca tacatacata acatacatac atacataaca tacatacata 3180
   cataacatac atacatacag acatacagac gcgtcgtccg tttcctgctg gacacgaaaa 3240
   agtgctccag agagcggtgt atatacaccg gacattttcc tgcttgaaac acacgcatgc 3300
   gtacctcaaa gtcgcgcgag ggagagagcc cgtttattgc agcgtcgcgt ttgctttttc 3360
   ccctggagcg ttcgtttcct tttttccagt tttgcaagtc ttcacgagct cgttccgcgt 3420
   tctcccgttt tcgttgttcg cttccctcag aagggtgaca caggaaacgt gtacatttcg 3480
   gacgcgaagg gtgtccgata ctcgctgtcg ctgccgaaca acatccgcac cagcaccgga 3540
   gagtgcgagt tcgacaaagt gctttctctc gagggtgtgt acttggcgaa cttcaaggac 3600
   tcagtcgact cgtctgcgtc cgtggacggg ggacagcaag gcgacctgga gaagttggag 3660
   gaggagatcg aagaggaggc cgaaggcgtc caggtggacc tcgagaagaa gcacaaaagc 3720
   gtcgctacgc ggagccgtca ggaagaggtg attcgcacag tgatctcctt cgacaaaggc 3780
   ggcgtttggt cgtatctgaa ggctccgaaa gtcgacagca ggggacagaa gatcgactgt 3840
   ccgcccgatc gctgctggct tcacttgaat ggaatcacgc gcttcagcga cttcgcaccc 3900
   ttctactcag tcgagaacgc cgtcggaatc atcatgggca ctggaaacgt cggcagctat 3960
   ctcaggcctg aaaaagacga ggtgcgtgtc tcaagtggaa acagcgagaa ggggaggccg 4020
   gagccagaaa caaggtggtg cgcaaacaga actggatgcg gagacaaaga agagaaacgc 4080
   acagggatag agaagaagag aacaaggaga agagaaagga agaacggagc gaacaagggg 4140
   aggagaacga gaggcgacgg gagaagggag gaggccaaag gggaagaatg agaggagaag 4200
   cccagactag gaaaggtcgt gtaaagaaag acatctttcc ccacagatgt tcttccgatt 4260
   gcatgctgct tgtcgtctct tcgttttcct caggcaaaca cttacctctc tcgagatgga 4320
   ggagtctcgt ggatcgaagc tcacaaagga gctttcatct acgaaatggg tgaccatggc 4380
   ggcttgttag tcatggccga cgacacgaag aaaacgaatc aggtgcgacg cgttcgtctc 4440
   tttgcagttg ttcttcttcc ccttcaaaga cttcaattcc gcttgtgttc tccgtcttca 4500
   tttctttgca aaattgttct tttctttctg tcgctctttg ctttctctcg agttcgccgt 4560
   accgcttctc ttctgaggtc tgtgactctc gaagcgcgtg tctctttttt tccgcgtatc 4620
   ctatccacct tctggctttt tccaaaggcg gaagcctgtg cgaagatacc gactttccac 4680
   gtcgggacag aggactccat ttgtatccga tttttgactc tcttccattt ctctttctga 4740
   tcttgtgcgt ttgctgcttg aggcatttgt tgttttttct cacctgcgtt gtagcatctc 4800
   tattgagtct tgaaaaatag cgactctctg cagttcctac tctgctgaaa ttcactcttg 4860
   ttgtctaaag aaatggcgcg ggtgcctgtt ctctcctcga agtccgtctg catcgtcaga 4920
   cccgttgtta gttgccgtcg agtttccccc gtgtagactc gcacgagcga gaaaacaaag 4980
   atagactcga gaacgctgca agttgtcagt agaaaactgc gagttcagga ttttctgctg 5040
   cgcatcgaaa agcagacgtg gatggctgtc ggaaggagac tctcgaacgt tttaatttca 5100
   ggtcgtcttc agctggaacg agggacagag ctggtacgac ttcgagctgg gtgcggcgcc 5160
   gcttttcgtc gacaacatcg tcatcgagcc gaatgcgtca agtgtcgagt ttcttctcta 5220
   cggcaagcga gagcaagact ccgcaggtcg gaaaacagcg cctcccgtgg caacgcacgt 5280
   cgaaacagac gagaagggga gacagagaga gaggagaaga gagagcagtt cttcaaaatt 5340
   cacttccaga gaaacgcgca gctaaaactc tcgagtcggg agcagagtct ccaaaatctt 5400
   gcaaatcgga aaggaatgaa agacacacac aaatgtggac aaacagtgcg atatatatat 5460
   atatatatat atatatatat atatatatat atatgcatat gtctacagag agacgaggga 5520
   agcaagggaa ggaagctctt tggtgttgct ttcgatggaa atgctttttt gcggcagcag 5580
   aatcggaaga gaaggctctg tcgcggcggt tttcttctgc tttacatttt ttgttttcag 5640
   gtgttctgtt tcacctcgac ttcaacgcgc tgaaccagca gcagtgcaaa ggcatttggg 5700
   ccgcagactc cgtcagcagc gactacgaaa cgtggagtcc ttcgggtgcg cgaactggac 5760
   cgctcgcagc tgaagcagcg gaatgaacaa aagatctagt cccgtcgcat ctgaaaagta 5820
   tatatatata tatatatata tacttttata tatgtatgta tgcgtgtaca ggaagacagc 5880
   gggaagaatt gcgttcgata tgcgcatgtg cgtccgacaa acgcgtgtat gagcatgtat 5940
   caatacatca ttatgcatgt gcacaaatgc agggaaaagg aggagactgt gaactttgtt 6000
   ttctttcctc agatggccgc gccggtggcg agagatgcat tttggggaag catatcacgt 6060
   acacccggag aaagcaaacg agcgagtgct tcaacggccg agactttgac cgcccgaaag 6120
   tttccaaggt acctttttct ctcttcactt catgtacagc gaaccgtttc catgattctc 6180
   cgcataaacc acgtcacctg ttacgccttc tcttgccgcg aattctacct tctgagagga 6240
   ctggcagacg aacggtcact ctctactcgt tcctccatca acgccctcca tctccgtttg 6300
   tcaaaagtcc agatctcgcg aaagagcgag tctttctata gacgctttga acaagcgacg 6360
   gaggtggttc tctgtgcttg ccagtctccc agcgtagtaa tgcctacgca cacatgcacc 6420
   ctgtgtgtat cctagggtca caaagagaaa ttctcacctg tttcgatctc cctctgtatg 6480
   tgtagttata tagatatcta tacatctaga tattttttta tatatgcata tacatatatg 6540
   tatgcgtata tgtaatatat atttatatac atacatatat ttatatgcat acatatatat 6600
   atatatatat atatatgtat atgctcaatt atttatcttg tatctgtgga catgcctgga 6660
   tctagggaca tacggggacg tgcgtgtttt tctctgcatg tgaaggagtt tgcttgaaaa 6720
   cactttatat atatatatat ttatttatat ttatattata gattgatgcg tctccgtaga 6780
   aatgtctata cgcaggacca cctgtttgtt tttagataaa agtgaaagtg ttgaaaataa 6840
   cgtttttcag taccgttcgc ttgtctctct gagatgtttt tcatgacttt ttgtgagttt 6900
   tccgcgattt tttcaggtgt gtccgtgcac tatggaggac tatgagtgcg agtttggctt 6960
   cacgagagcc atcgggtcca cccagtgcgt ggcgaccgac gcagcagcag ccgccgccgc 7020
   gacagcgact ggcctcgcgc agttcgccga cgaatcagac gccgcagccg ccgccgcatg 7080
   cacctcctcg tctttcttct acacctctgc ctatcgaaaa gtaagtcgaa aaacgagaaa 7140
   ttggagaaaa gggagagcag agagaagggg agaaagcgta gtggagggag aacgaggggg 7200
   gcagaagagg gagggcaagg gagaagagag acagaaaaga agaggtaggg aaaagagtac 7260
   ttggagatcg ctctcaagaa gggcaccaga gtttgtggct gcatgcagag agaagaacgc 7320
   gtgatgagag agtggagaaa acttcgttgg tctcctttcc ttccaggttc cgggagatgt 7380
   ctgcgagggc ggctggatgc ccgagaaggt cgctgtgcct tgtcccgccc attcacccgt 7440
   cagtcgagga ggaaagacag tgttgcttct tcttctcttt atcgtcgtcg tcatggtcgt 7500
   catcaactat ctggcgaaaa ctggaagact gaagaagttc ttccgtaacg caggttcgcg 7560
   aatcctcttg ttgacagttc tgtctcgtgt tatcgtgttt ccatttcctc ctttctgtgt 7620
   gtcgctcgtc cacgcgttgt ctctttttta tttcttctgg ctccgtcttt gcctctctgt 7680
   cttcctgtcg ccctttccgc gtcttgtctc tcgagttctg tctctctctc cttatcttta 7740
   tccttctgtt cctctctctg gatctctcgt ctgcttcctc ttcctctctg tttttcgttg 7800
   tccttatctc ttccttctgc ctttctccat gtcggtctgg agctccacgc gcgtctgccc 7860
   tggagtcgcc acgtagccat tttcctcttc ttttcttgct cttttctctt cgtttaggtt 7920
   tcgacagctt tgcaaatgtc tcttacggcc ttgtcggcgc ctccgcggga gggcctggcg 7980
   ggtggctcga ccaggaagct ggtgaaagtc gaaggggaga agaacttgga gaaagatcaa 8040
   aatacgaacc ggaactgggc ttcatcgaag ccgaacaggt gtgcacctct ccctcgacac 8100
   ggtagcgttt ttctccgttt ctcaatgtct gcctgtgtcg atataatctt gtctttcgat 8160
   gtctatccgc atctgcctta cctgtccgtc tgtttggatc tctgtgtgta tgcctacgtc 8220
   tattcatcta tgtgtacctg ggcctctgtg agtctgtgta catcgaccta ttatgtaagc 8280
   gagttgtaga tgccgtctac ctgaggacct cagcttctac tctgctgcat tcagcaccgt 8340
   tctcggcgat gtatcggtat gttcttcaat ccatttccca aaggcgtttt ggatgctcga 8400
   aagcgggcat ttctccagtt tttaaagaaa cttttttctg caaacttttg tcgggaggga 8460
   aggtcgtgct tttccgcgct gcactggacc ttatcctgca gtggaatttt tcaaaaaatt 8520
   ctgtttttca ggacgagaac gaagaagacg cgccgacact gatgaactac ggcaacgcag 8580
   caggcggtca aagaacttcc ggcatgtctt cgcggtcgcc gaaaccgaca gaagatttcg 8640
   aactcgacga ttcacgaccc ctcttcccct cccatgtcag ctctcgcgaa actcaaggaa 8700
   gctccggtct tcaccctcgc tcgacttcga cagaaaacga acctgtatgt gtcttgtaga 8760
   cgctcgactc cgggtgtctc gtgcgtcaag ccttaacggc agcggattcg gcttatattc 8820
   ggcgactcca cagcttattt agtacacacc cacccacaca gtgtacacat accgcactga 8880
   aactggggta gtgtccgcgt ctctcgttac ggtgccttcg cggagtcgca gttttcctct 8940
   tcacaggtgc atccgagggc tgcctctctc tgctctctga gcatcgattc tttttcagtt 9000
   atcgagtggt gcgttggtag gaaaccggag agagatcgac gagacacaga accggttctc 9060
   tctactgttt gcatatatat atatatatat atatatatat ataatgttgt ataggctaca 9120
   gtacatatat gaagtatgcc gtctacttca ttacatatat atatatatat atatatatat 9180
   atatatatgt tttatttgaa atacagggtc gtatatagag tacacaacct agttcaatca 9240
   cttatatata tatatatata tatatatata tgcatctgta tgtcggggcc tgcagagagg 9300
   agagaggcgt tttgaggcgg ccgggttcga gaagacggaa gcgaaggaag agagtggatg 9360
   ttttcttgaa ttccagaaag accggatgtt ccgtttttag tcgacgtgga agtccgttca 9420
   cccactccct tttttcgctg agcaggagac gtctcttgtc gaatgaaacg actcgcgttc 9480
   acgctgtccg gttttctgtg cagattccgc gtctggcgcc gccgcgattc gacgaggata 9540
   acgtcgaact tctttgaaaa cgtcttttct tctttcttct ttctccgccg tcttgcagct 9600
   ctgtaggagc ttccgccgaa gacggctctc tttcgtgtct cgcatgtctt tctcgtgtgt 9660
   cgtctctgaa aagaacgtgg tggcccgggg cggttcgacc ggggagagag agtgctcttt 9720
   cttttgtgcc tgcaaaggaa atcttcgagc gaaggaagac gcgaagagaa aactgagaag 9780
   aagttcgcga ggatggagag gaagcgcggt gtctgcgagt gtccagaggt cggaaagcga 9840
   acaggtttcg cggcagtcga gggtcagtcg aaggaatcga ggaagaaccg agaggaagga 9900
   acgcgaggaa acgaagaact cgactctttt tcgcgcgacg cggaagcaga cgagaacgaa 9960
   tggggaaaag aagaagaggc gcctttggtc ggcagacgcg t 10001
<210> 8
   <211> 962
   <212> PRT
   <213> Toxoplasma gondii GT1
<400> 8
   Met Ala His Ser Phe Ala Arg Gly Arg Pro Pro Ala Ala Gly Arg Thr
   1               5                   10                  15
   Leu Ala Val Phe Leu Ala Phe Leu Ala Val Leu Ser Pro Ser Leu Leu
               20                  25                  30
   Pro Glu Phe Phe Ser Glu Asp Ala Gln Lys Arg Glu Ser Ser Val Leu
           35                  40                  45
   Val Ala Asn Ala Ala Ser Tyr Ala Asn Lys Lys Val Ser Val Ser Glu
       50                  55                  60
   Val Ser Phe Asp Ser His Ile Glu Asp Ile Gln Trp Cys Gly Thr Asp
   65                  70                  75                  80
   His Arg Thr Ile Leu Leu Lys Thr Arg Arg Gly Arg Leu Tyr Arg Ser
                   85                  90                  95
   Gln Asp Gly Gly Lys Ser Trp Thr Glu Ile Thr Asp Leu Leu Lys Ser
               100                 105                 110
   Ser Glu Ala Ala Thr Gly Thr Val Ala Val Asp Ser Ile Ile Val Ser
           115                 120                 125
   Pro Val Asp Lys Arg Val Val Leu Ile Val Gly Ser Lys Arg Asn His
       130                 135                 140
   Phe Ile Ser Glu Asp Ser Ala Ala Thr Phe Arg Arg Leu Lys Tyr Lys
   145                 150                 155                 160
   Asn Thr Ile His Asn Phe His Phe His Pro Thr Arg Pro Lys Tyr Ala
                   165                 170                 175
   Ile Leu Ser Thr Trp Thr Asp Ala Cys Tyr Ser Gly Ser Gly Thr Ala
               180                 185                 190
   Ser Arg Ala Gln Ser Gln Gln Asp Cys Asn His Gln Leu Phe Tyr Thr
           195                 200                 205
   Arg Asp Leu Gly Arg Ser Phe Lys Leu Val Ala Asp Tyr Val Val Gln
       210                 215                 220
   Phe Ser Trp Gly Asp Lys Lys Leu Gly Asn Thr Asp His Ile Phe Phe
   225                 230                 235                 240
   Thr Gln His Arg Gly Arg Ser Gly Asp Gln Pro Arg Tyr Gly Gly Trp
                   245                 250                 255
   Ser Lys Asn Val Asp Leu Met Tyr Thr Pro Asp Phe Gly Ala Thr Ile
               260                 265                 270
   Thr Arg Leu Val Tyr Arg Gly Asn Lys Phe Leu Leu Ser Asn Gly Tyr
           275                 280                 285
   Phe Phe Val Ala Lys Val Lys Asp Ala Ala Lys Gln Thr Val Ser Leu
       290                 295                 300
   Leu Val Ser Thr Asp Gly Gly Lys Ser Phe Gln Met Ala Lys Leu Pro
   305                 310                 315                 320
   Val Glu Ile Glu Glu Arg Ser Tyr Thr Val Leu Asp Thr Ser Glu Asp
                   325                 330                 335
   Ala Ile Met Leu His Val Asn His Gly His Asp Asn Lys Gly Asp Thr
               340                 345                 350
   Gly Asn Val Tyr Ile Ser Asp Ala Lys Gly Val Arg Tyr Ser Leu Ser
           355                 360                 365
   Leu Pro Asn Asn Ile Arg Thr Ser Thr Gly Glu Cys Glu Phe Asp Lys
       370                 375                 380
   Val Leu Ser Leu Glu Gly Val Tyr Leu Ala Asn Phe Lys Asp Ser Val
   385                 390                 395                 400
   Asp Ser Ser Ala Ser Val Asp Gly Gly Gln Gln Gly Asp Leu Glu Lys
   405 410 415
   Leu Glu Glu Glu Ile Glu Glu Glu Ala Glu Gly Val Gln Val Asp Leu
   420 425 430
   Glu Lys Lys His Lys Ser Val Ala Thr Arg Ser Arg Gln Glu Glu Val
   435 440 445
   Ile Arg Thr Val Ile Ser Phe Asp Lys Gly Gly Val Trp Ser Tyr Leu
   450 455 460
   Lys Ala Pro Lys Val Asp Ser Arg Gly Gln Lys Ile Asp Cys Pro Pro
   465 470 475 480
   Asp Arg Cys Trp Leu His Leu Asn Gly Ile Thr Arg Phe Ser Asp Phe
   485 490 495
   Ala Pro Phe Tyr Ser Val Glu Asn Ala Val Gly Ile Ile Met Gly Thr
   500 505 510
   Gly Asn Val Gly Ser Tyr Leu Arg Pro Glu Lys Asp Glu Ala Asn Thr
   515 520 525
   Tyr Leu Ser Arg Asp Gly Gly Val Ser Trp Ile Glu Ala His Lys Gly
   530 535 540
   Ala Phe Ile Tyr Glu Met Gly Asp His Gly Gly Leu Leu Val Met Ala
   545 550 555 560
   Asp Asp Thr Lys Lys Thr Asn Gln Val Val Phe Ser Trp Asn Glu Gly
   565 570 575
   Gln Ser Trp Tyr Asp Phe Glu Leu Gly Ala Ala Pro Leu Phe Val Asp
   580 585 590
   Asn Ile Val Ile Glu Pro Asn Ala Ser Ser Val Glu Phe Leu Leu Tyr
   595 600 605
   Gly Lys Arg Glu Gln Asp Ser Ala Gly Val Leu Phe His Leu Asp Phe
   610 615 620
   Asn Ala Leu Asn Gln Gln Gln Cys Lys Gly Ile Trp Ala Ala Asp Ser
   625 630 635 640
   Val Ser Ser Asp Tyr Glu Thr Trp Ser Pro Ser Asp Gly Arg Ala Gly
   645 650 655
   Gly Glu Arg Cys Ile Leu Gly Lys His Ile Thr Tyr Thr Arg Arg Lys
               660                 665                 670
   Gln Thr Ser Glu Cys Phe Asn Gly Arg Asp Phe Asp Arg Pro Lys Val
           675                 680                 685
   Ser Lys Val Cys Pro Cys Thr Met Glu Asp Tyr Glu Cys Glu Phe Gly
       690                 695                 700
   Phe Thr Arg Ala Ile Gly Ser Thr Gln Cys Val Ala Thr Asp Ala Ala
   705                 710                 715                 720
   Ala Ala Ala Ala Ala Thr Ala Thr Gly Leu Ala Gln Phe Ala Asp Glu
                   725                 730                 735
   Ser Asp Ala Ala Ala Ala Ala Ala Cys Thr Ser Ser Ser Phe Phe Tyr
               740                 745                 750
   Thr Ser Ala Tyr Arg Lys Val Pro Gly Asp Val Cys Glu Gly Gly Trp
           755                 760                 765
   Met Pro Glu Lys Val Ala Val Pro Cys Pro Ala His Ser Pro Val Ser
       770                 775                 780
   Arg Gly Gly Lys Thr Val Leu Leu Leu Leu Leu Phe Ile Val Val Val
   785                 790                 795                 800
   Met Val Val Ile Asn Tyr Leu Ala Lys Thr Gly Arg Leu Lys Lys Phe
                   805                 810                 815
   Phe Arg Asn Ala Gly Phe Asp Ser Phe Ala Asn Val Ser Tyr Gly Leu
               820                 825                 830
   Val Gly Ala Ser Ala Gly Gly Pro Gly Gly Trp Leu Asp Gln Glu Ala
           835                 840                 845
   Gly Glu Ser Arg Arg Gly Glu Glu Leu Gly Glu Arg Ser Lys Tyr Glu
       850                 855                 860
   Pro Glu Leu Gly Phe Ile Glu Ala Glu Gln Asp Glu Asn Glu Glu Asp
   865                 870                 875                 880
   Ala Pro Thr Leu Met Asn Tyr Gly Asn Ala Ala Gly Gly Gln Arg Thr
                   885                 890                 895
   Ser Gly Met Ser Ser Arg Ser Pro Lys Pro Thr Glu Asp Phe Glu Leu
               900                 905                 910
   Asp Asp Ser Arg Pro Leu Phe Pro Ser His Val Ser Ser Arg Glu Thr
           915                 920                 925
   Gln Gly Ser Ser Gly Leu His Pro Arg Ser Thr Ser Thr Glu Asn Glu
       930                 935                 940
   Pro Ile Pro Arg Leu Ala Pro Pro Arg Phe Asp Glu Asp Asn Val Glu
   945                 950                 955                 960
   Leu Leu
<210> 9
   <211> 10001
   <212> DNA
   <213> Plasmodium falciparum 3D7
<400> 9
   tccacttaag aaatatttgt aaaaaacatt ttttcaaatt gtattcttcc aagttaagca      60
   aagaaataat gaaaaaagaa aaaaaaaaat aaacaaactt ttaatctgat tcatcatcca     120
   ttacatctaa tttatcaagt gtcttatata aaacatgacc aaagttctta tcaccttttt     180
   tatcaaaatt taatgtcatt gtttcttttt ccttttcatc atcatttgta tttgtaatat     240
   tttctttatt tttgttttca tttatttttt gtttaattaa ttgaacaaca gccttatctg     300
   ttttttttga taaaatatcc aatttctttt tcaggtctct ttttaaatca gcatttatat     360
   cttttgcatt tatttgatct aagatgttac cttgaaatac tttcgttaat tcttcatcaa     420
   attccttatt taattttgct tcataatctt cagtatcagg acatgggata catgatttct     480
   ttagttcctt atttactgga atgtaattat aaaaccttaa attctcataa tcatttgtat     540
   ccattttgtg atcttaccac ctttcataat attaataaat attactatat atatatatat     600
   atatatatat atatatatat atatatattt atatttatat gtatgtatgt atgtatgtat     660
   gtatgtatgt ttagtttaat atctcataat aattattcat gtatttataa ttctctgatg     720
   gattatatga aaatacatta acaaataaga taaatgactt aacaaaagaa aaaagaaaaa     780
   gaaaaaatag ggaaagaatc attacactat aattatatat atatatatat ttatttattt     840
   atttatctat ttatcattct ttatttgaat ttttctatat ctaatttgaa ataaaaagaa     900
   aaaaaaaatt atccattata tatatatata tatatatata tatatattta tttatttatc     960
   atactttatt tgaatttttc tatatttaat ttaaaataaa aacaaaaata ttattcatta    1020
   tatataatat ttaatacagg gaaaaaaaaa aaaaaaaaaa aaaaaaaaaa aaaaaagtcc    1080
   cagaaaaaaa agaaatatat attcttctat atttaaatat tttgagatat gtattatata    1140
   tatgaacatt aaaaatgtta gttcgataga tcaaaaaata ataatacttt agttttcata    1200
   atgaaataag tcttaaatat aatatatata taattataaa aattatagag ataaaacaaa 1260
   atccttcaaa cataattttg ttattttata agtaatattt atattatatt ttattatttc 1320
   ttaatataaa ttatactatt ttattagaaa ataagggaac tgttttttta tattttgaaa 1380
   aaaaaaaaaa aaaatggaat atatatttgt acataaatat tataataata taatttgaat 1440
   tatttataga ttatgaaaaa ataatatgta gttatataaa ataaatgtga atgacttttt 1500
   attttttgat taacttcaca cttattttag aaaaaaaaaa attaatgaga taataaaaaa 1560
   aatgtgatcc tattgttctc atagaattta attataattt ggtgtatata ataaatgtat 1620
   aaatgataat ttagcaccat aaaaatgaat tgaaatataa tatatttctt taatatattc 1680
   aataatataa aaacatccta atctaatata ataaaaaatt attatattat attatatgtt 1740
   ataaaataca ctgttttaat attttagtat caaaatataa atatgtaaat atatatatat 1800
   atacacttca tatgtatata tttatttttt tttttttaat tcatattctc ttatttcaac 1860
   tttttatttt atctctttta ttatttattt attttttttt tttttttttg ttctacacat 1920
   ggatttacca tttaaatatc ctttttcatt atttttttta ttttatttca ttttgttagt 1980
   tatgaaggaa taatatctgt atagattgta ttaccgtatt tgtattagtt gtaagttcca 2040
   atgtcttatt ttccttgata acaatgttag cagttacttt agtatattgt ttaggtttaa 2100
   gaaaaatatt atttataaaa atttcattat taactttaat tgaattaatt tcttgtttgc 2160
   ccgtaggaga tcctatttct atagtaacag aatatgttcc ttcatgtata agatctatgg 2220
   accatttatt aaatgtttgc ttattacatg tattagtgtt attacattca tatgggacaa 2280
   acgaaattcc tgcatgtgta ggatcttttt tatctaaata tctcaaatca attggtgtag 2340
   gtaataaatc ccatccgtat ttgaaaaatc catgatctga ttttttcatt ccattatcta 2400
   ttatccaatt gaatggttta gaaatatgta catttttgct tctaaaaaaa atattaattt 2460
   catattgact tttttttttg tctgattctt tttcttgatt ttttatatct atacaaggta 2520
   taccataaaa tgttatatta ccacctgttt gagctttggg attatttact ttggaaatta 2580
   caacttttac cgtattggtc tttattggag ttttaaggac ataatgatga tgcccaggag 2640
   atatagaaaa tatttctggg ttttttatgc ttggaaaata taaagataat tcattgattg 2700
   tattattatt agataaattc aatgttttaa acgtaaaaga gtgtatgtct atgtcatact 2760
   taaaatttat acttacgtat tgacccagtg atttattttg tgcagttttc catgttgaat 2820
   attctttttc ttggttatta ccactgaatc cagcgttaca atcaaacatt tgggattcat 2880
   aagaagaggt gtaacatgaa tggaaataat ccgaactagt tagagaaact ggttgtacat 2940
   aacctttaca tgtatcacct gatcgtaaat cactcggttg tacaaagagt aaaaatgttg 3000
   taggagtagg tagagatata ataacctctc cttcattata ttgtgaagaa tatatataat 3060
   atatcttttg ttccgttgaa ctgttatttg aattttgttg tacttcatat atggataacg 3120
   tttctttagt gtttgtaaac tcttttttag taatatcatt taaagggata ttcttttttt 3180
   gttcttttgg tatagctata taaacaatac aatatacatt tatcctaaat ttgattatat 3240
   ttttattaaa attataaaga gttctaattg cttttaattt ttcatgtatt gtaggtatgt 3300
   cattaattaa ataactcata ttattcataa atgcatgtac cccattttgt aatggaaaaa 3360
   tttcaaatgt atctccattt aattccgtta ttcttaaagg cgtagataca ttaccttgaa 3420
   aaaaataatt tgatggtatt atttcttcca taatattatt tgacttccag tataatataa 3480
   tatgggatat gtgtggattt gcaaatttca tagtactcaa atgaaaatat tcaattctta 3540
   ttttatattt ctttccacct attaatccta acttttctga atttgtttta tgcactttgg 3600
   aattcatttt atcgaatgat tgaatagata taggtcttat ttcttctgat tcttcttctt 3660
   ttggaaaagg catattgtct acaattatag gagaattatc taaaaatatt cttatcccgc 3720
   aatcatgatc tactgagaaa atgtaattat ctgattcagg gatttttaaa tatccatccc 3780
   atctaattga aaaatgttgg taaggaatat tttctattgg tatccctgtg tcccatataa 3840
   agtttatata tttatcatta tgtatggctg taggataacc agaaaaataa gcattattat 3900
   aataactagc cattaatcca tcagcttttt cttcaacaac ataacctttc tgcatagaac 3960
   agttcttttt attttcaaga tatgattttc tatttttttc tacctcctct tctagttcaa 4020
   ttattttatt tccagtatca cttagagatg ttgcttgtaa taataatcta ttaatgttac 4080
   gactggaatt ttttgctaaa tcattaatat cttgaatttt tttttttaac aaatcggatg 4140
   cattaccaca tacttcttta tatttttcat atataccaac taattttttg tattcaaaat 4200
   ctaatttgtt tacagcatca agaagttcat tggatttaga ttggaaaaaa gtacgtgcct 4260
   tacttcgtac aacatcataa ttaattactc ctttgcaata atcccaatct ctatttcctt 4320
   ttcctattaa ttgaacctcc acatagcacc attcctttcc cgttatacca ttaggatctg 4380
   ttgccctagt gcagtctgta tatgtttgat cgtcttgaac aaaggctgct gcacatagac 4440
   gcccatccac ggttttccta tgttgttgtc gatattccgt cagtttctat atacaaaaaa 4500
   aaaaaaaaat atatatatat ttatatatat gtaggtaggt acaaatattt agaactatta 4560
   taaatggtta catatacatg tactttcata aacttcatta ttatgttctc cttttttttg 4620
   tcttatatgt tctagtgtca tacctttaaa ttttccttat cataattttg agtgttacat 4680
   ttagaagaaa gaaagattaa tacaaagcaa atagtattta tgtacttcat aatatgattt 4740
   atactaagga ttggtataaa gtacaggagt aaattgaaaa aaaaaaaaaa aaaaacacga 4800
   aaatatatat atatatatat atatatatat atatatatat atatttattt attatataaa 4860
   aataatatta taaaagtaaa taaataagat attaattttt tattaattta taacatatat 4920
   tttttttttg gatatgcaaa aatatatgta attttctata ggggtaaaaa aaaaaaaaaa 4980
   aaaaaaagtt acagaggtta acttggtaga caatttatgc gttaccaaaa attatattca 5040
   ctagcataat ttatggacac gcggaaaaaa agaataattc aataaaatgc tcaacaactt 5100
   tcttttacag ggaaattaaa ataatgcgaa attattttga tgaacatttt tatattctat 5160
   cattttttat aaataaaaaa atattgttct atatgcttat acatatgaaa aagttcgcta 5220
   actttaagat ataatattta atttaaaaaa aaaaggatga ataatttttt taattttttt 5280
   tttttttcta caaatttgtt tgccactaaa ataacattaa gtataaaaat atatgagtaa 5340
   taataatata atgaacaaaa taaattgatt ttattaatag aaacatgtgt tatacaaata 5400
   aggttggttg gtgtatggaa tttaataatt attaaaaaaa aaaaaaataa tatattgatc 5460
   aataaattat ttgtgtattt tttaatttat aattctagct tcaatttatt tccaacatcc 5520
   atatgtgata tcatctataa ggaaaaatca aaaaggaaat ttatgtatat atatatatat 5580
   taataaaata tgaattaact tatataagta tatatatata ttattacatc tttaaattct 5640
   tcaaaggata tcattccgtc accatcttta tctgcttgta atatagttcg atctactaat 5700
   tgttgtaact acaccgaaaa aaaaaaaaaa aaaaatatat atatatatat atatatatat 5760
   atatatatta taattaaaaa ggtcatataa ataaaaatgc tttgttgtct tgataaatta 5820
   aaatatacct gtgtatcgtt caaattatta ccaaccatca ttttcataac ggtaaataat 5880
   tctccattag atatcattcc atctttatta atatcgtata catcaaaggc aaatttcttc 5940
   ttctggaaat catcagtact agatgctaat tttgctgtaa tgaatgatta aaaaaaatat 6000
   ataagtaaat aagatatata ctaaatgtga atctatgtac gctcatattt atacttttac 6060
   attttgatat aaaaaaaaaa aaaaaaaaaa aaaaaaaatc atacacaaat acatacatac 6120
   atatatatat atatatatat atatacatag taatgatgtt ttattttttt acttattcca 6180
   actaaaaatt ctacaaatga aacttttcca tctgaatttg aatcaaatat tgatataacc 6240
   cttttaacta ggggattctg aacaaacaaa gaaaaatata tatatatatg gaataattta 6300
   ttatgtatta attttatttt cctatgtatt taataataag aaaaatatga ggacattttc 6360
   attcatatta agtaaatata ttctgtatat atttccagaa taacttttta ataatttata 6420
   attttataat acttcattat tttattgtta ttttttattt tacatcacat atttcaggaa 6480
   catcaaataa ttcattagga tctaactgtc catttttatt tgtatcaagt tctataaatc 6540
   ttttatacat ttttttgata tctgtttcac taaaattagc tgcttgtaat aaatcttttt 6600
   gatctttttc agataatatc gcttgtgtgt ttctatacaa tatgaaatga aaatatatac 6660
   atatatatat atttatgcaa ccaaattata tatgtctaat taaaaaaaaa gaacaattga 6720
   gttgtcttta taattaaatt ttacaaggaa taaaatattt ttatttcata tatatatata 6780
   tttaggtagc tttttattaa agaattatgt tttaccccat atttcctttt tcttgttatt 6840
   catgagaaaa aatgaaaata aaattatttt tttttgaatt gataaaagat taacacttta 6900
   tatttatggt gttcaaaaat aaaagaataa tttttttata tttaattatt aaaaaatata 6960
   tacatatata aatatatata tatatataaa caagtctttt tattaaaata gcattataat 7020
   atcattattg aatcttatac atgtatataa atataaataa ataaatatat atatatatat 7080
   atatacatat atacttatat tttattttat aatattgtaa tgcatatact gaaatatttc 7140
   ctttttctga aatatttttt aataattaat ttaaattata cttatataac attatttgta 7200
   catataaaat atggatgctt aggatgtata tgcaacatat ataatattat taatcataca 7260
   catacatacg tatatataga taaatatcta tttcttgtta tcatattttt ccttttcaat 7320
   tataacagat cttatttttt tttcaaatga gggatcatac tacacaaaaa taagttcaat 7380
   atttcccatc gaaagaaaaa ttgaatgctg ttataaaaat aagtactatt aaattaaaaa 7440
   cggaaaaaga gataagaggt aatacgaaat aaaaaaaaat aataaaataa ataatattat 7500
   attaaaaata tatacatata ttatattata tatatatatt tatttacatg aattattaat 7560
   gaaaatgtcc tattcactga aaattttttt attgcgcatt catgcacatt ggatcaaaaa 7620
   agagagaaaa aaaaaagaag aaaaaagaaa aaatatatgc gcatattaca tttatgtata 7680
   ttttatatac aaaaaaaaaa aaaaaaaaaa aaaaaaaggt atagtatttt attaatttta 7740
   tataaatgag aataaataaa agaaaataca tgtattatat atattatata tatactgttt 7800
   tataatccct tttatatgct ataagaaatt aatattttct attttacaat aaaggaaaca 7860
   aatttttatt atattgaaaa ggtgtatata atttaaatat gaaactaatc caaatatata 7920
   tatatatata tattttacac atttcttttt tctttttttt tattcaagtg ataattttat 7980
   tttgaggttt tacttttatt gagagagaaa gaaaaaaaaa tatatatatt atatatatat 8040
   tatacacata tacatatttt tttattaata tgtgtgtgtg cgtgcggatt taattttttt 8100
   cttttttttt ttaaataaaa atttgataat atatatatat atatatatat atatatatat 8160
   atatatatat ttatatcaat atataattaa gcaaataata tttaatttat cttttatgga 8220
   tacaacatat tcttgtaagt gtagcattgg atatatataa agaaaagata ataaatacta 8280
   gagctttata taatagagta atcgtatttt ttctccattg tattattcat atattaacaa 8340
   attataaaga aatgaaatta attatttcgg aaaagaaaaa atatattata tatatatata 8400
   tatatatgcg gggttataaa cttatagaaa atttgtatgt tctccctttt ttaaaaaggc 8460
   ataataatta taagtgtttt tattgatatt aaaagttgtc ataacttttt aaattaatca 8520
   tatctcattt aaacttttgg gctttttctt ttgttttatt aaaatatcac caatattttt 8580
   atatatacat attttatata tggatgttta tttattttat tttattttat tttttatcta 8640
   tgtggataaa caaatacacc cttaatacaa acgtaatatt gttcatagtt atggaaagtt 8700
   ccatagaata atttgtaata caagtatatt atattttcgt ttttatatta cataaataaa 8760
   tatatacata tatatatata tatatatata tatataatat atatataaat caaactgtat 8820
   tatatatttt cttaagttct taaatatata taacatatat aatatataat gcgcatatct 8880
   tatatagtgg ttttcccttt tctttttggt gtttatatgt aaaatgatgg ttgttaattc 8940
   ggaaaatata taaaatgtta aatacctttg atttattatt atatgaataa atacaaatct 9000
   cttattcgta ttattattat atatttatgt atatcctaat acatttaggt ttaatattta 9060
   taaaagaaaa aacaagaaat atagtaaaaa ttatttttta aatatttcat aaaaataatt 9120
   tttttcgctt ttttttatta tcattttttt tttttttatt tgagcaaaaa gcaactactt 9180
   taaaatatat aaaaaatata tttctattaa ttaaatctta ttcatacaaa tatttaccct 9240
   tattttgttt tatatacaca ataaaaagga aatataataa ttaaggggga aaaaaaaaaa 9300
   aaaaaaaaaa aggtgaaata tggttttatt aattacatat gaaaataaag catattgtat 9360
   aatatcctct ttaaataaat aaataaatat atatatatat ataataataa taatatatat 9420
   gtacaaagaa tgaaactata cgtacattta atgtgaaagt aattttatat ataattaata 9480
   taatataaat aatataaaaa tatatagtga attactaatt ttttcatttt actttttttt 9540
   tttttttttt ttttttttta aattttatac attttaatta tttttattat attattaata 9600
   tttcaaatgc tacaaatatt tgatatataa taatatattt tttcttataa tgtaattttc 9660
   catttttcac tgttttagaa taagggaaag aataaaacaa aaaaaaaaaa aggaaaagaa 9720
   aaaagaaaaa tgtatatata aaatattata tataaaattt attatacatt ttgaagatcc 9780
   aatatatatt tataaaataa caagtgaaaa atagttgaag gcttatatgt gaatttttta 9840
   ttttcataaa ataaataaag aaaaataaaa atagagatat attaatgtat atatatatat 9900
   ataaatatat atatttttgt gaaattctta aaaaatttgt tgattataaa aaaaaaatat 9960
   atatatatat atatatatat aaattattaa attataaata a 10001
<210> 10
   <211> 10001
   <212> DNA
   <213> Plasmodium falciparum 3D7
<400> 10
   atgaaaaaaa agcttgaaaa gcaaaagaag ttgttagaat atgaaaaagg atattattca      60
   aataaagtaa aaatgcgaaa tatgttattt ttcaaaagta taggaaataa gaatcattta     120
   tttttggttt ttctattatt cttgtttttt tctttttcat gtataaattt tacacaatgt     180
   caaataacaa aaaagaaagt atcagtaagt gaaataaatt tcgatagtgc agtggatgat     240
   gttcaatggt gtggaaataa tcatatgaca gttttagtaa aaactgtaaa gggaagatta     300
   tataggagtt cagatggagg aaaaatatgg actaatataa ctgggaattt atcggataaa     360
   gaaaataata aaagtgatcc atcatcaaat catatgaatg aaatgactac tgtagatctt     420
   ataatggtaa attcagtaaa caagaatata gtgttaatta taggaaatca aaagaatcat 480
   tatatatcag aagattctgg agaaacattt agattattaa attatcagaa taaaatcaat 540
   ttttggcagt ttcatagtac taaaacgcat tgggccttag tttcgtcctg gacagaagct 600
   tgctattcta atgatgataa tagtggtgaa tgtatgcaga ccttatcctt aacaaaagat 660
   ttagggagaa catttcaatt aattgatata tatgttgtac aatttaattg gggagataaa 720
   gaatcacatt tagaagacac catctattat actcgccata aaaatagaaa tggtcatcaa 780
   caaagattta gtggatggtc taaagatgtt gattttgtgg ctactcataa ttttggaaaa 840
   tctgtagatg tattagtaaa acaaggaaat aaatttttaa tctctaatgg atatattttt 900
   gttgcaaaat taaatgatgt tataaaacaa actgttaata tgatggtttc aactgatgga 960
   ggcaaaactt ttaataaagc aaatttacca gaaaatattc atgagaaatc atatactgtt 1020
   ttagatacat cagaaggttc tataatgcta catgtaaatc atggaacatc ttcagaaaaa 1080
   attaatacag gtaatgtata tatatcagat gcttcaggtt taaattatac attatcatta 1140
   cctaataata taagaacctc ttctggggaa tgtgaatttg acagagtatt aagtttagat 1200
   ggagtatata ttgccaactt tttagatgtt aataatgaaa taaaagatga agatttgaga 1260
   ttccaaaatt tcaaatcagc tattgaagaa gatattgctc catttgagac aaatacagaa 1320
   aaaagaaaaa aacaatataa caaaggaaaa aatgaagatg ctgttagaac tgttatttcg 1380
   tttaataaag gtggagaatg gtcttattta aaagctccta aagttgatag tagaggaaac 1440
   aaatatgagt gtggtgataa ttgttatttg catttacatg gaattacaaa ttatcatcaa 1500
   tatgcacctt tctattctat tgaaaatgct gtaggaatta taatgggaac gggtaatgta 1560
   ggtagccatt taagatatga aagtgatgaa gtaaatacat ttttatcaag agatggtggt 1620
   gtaacatgga ttgaagctca taaaggacca tatatttatg aatatggtga ttatggtggt 1680
   ttaattgtta tggctgatga tttacgtaaa acaaaccaaa ttgtatttag ttggaatgaa 1740
   ggacaaagtt ggtttgattt tgaattagga caattttcta ttgatgttga taatattgta 1800
   gcagaaccta attctgcttc tgtagaattt cttgtgtatg gtaccagaaa tgatgtaggt 1860
   gttttatatc atctagattt taatgctctt ggtcaaccgt tatgtaaagg tttatgggca 1920
   gcagattccg tctcctcaga ttatgaaaca tggtcaccta ccagtggaaa tttcaatgat 1980
   aagtgtatat taggtagaaa aattacttac accagaagaa aacaaacatc agaatgtttt 2040
   aatggaaaag acttaaaaag aacagttgat aaaaaaccat gtgaatgtac accagaagat 2100
   tatgaatgtg aaacgggatt tactagaaaa atcggaagtt atgaatgtaa gccaaatgat 2160
   ccaacattaa ccattgaagg atgtacaagc agttcttact tttatgcaaa tgcatataga 2220
   aaagttcctg gtgatatatg tgtcaatggg tgggttcctg aaaaagttcc agtcccctgt 2280
   ccttcctatg caccatttaa taaaagtaag gaaaatcaaa tatatatatt atgaaaatgt 2340
   tcatgcagta acatatattc aaaattttgt tgattatatt aattatgtat tcttatatat     2400
   gcattatata actttcattt catggcattt tatattttct tttattttaa ttttttcttt     2460
   tcttttagct gctaagtcaa tattatttat aatatttatc atgggtatag tcatgcttat     2520
   aattacttat atatgcagaa atcctaagtt taaaaactta ttttataatt atggtaggaa     2580
   tgaaatatat atatatatat ttatatatat ttatatatat tttatgtgtt tatgtgtgta     2640
   tgtacataaa taattttttt attataataa atcatgtttt tatatctttt tattttattt     2700
   tactttattt tttttaggtt ttgatacatt tgaaaatgtc aaatattcag ttattaaaac     2760
   caaaagagga aatgtcaaca ataatgtatt tgaaccagaa atggaattca tagatgcaga     2820
   acaagtaaaa cattaaaatg ttattaaact ttatatgttt ttttttttaa atgttcatta     2880
   ttttttatca atatatatat atatatatat atattatatg tgtatattta tttgatttta     2940
   ttaatttatt atttttttta ttttctagga tgataatgag gaagatgtcc ctacactttt     3000
   accatattca agtgatagaa ataggtcaac aaataaagat tttacattag ctagaaatag     3060
   aacaaaccaa aataataaca ttacctcaag gaatatatca ccttcaaaaa attatgctga     3120
   taatattgaa ttattataaa ttaaaggaat tacaactttg caagcgcaac ataattttgc     3180
   aaatatatac atacatacat acatgtatat atttgtatgt acatgttgct tatttattta     3240
   tactatgggt catcaaaaca attatgtgtc ttttttcttc ccttttgtta tatcataatt     3300
   gttaattttt aaagttgaac acaaaaaaaa aaaaaaaaaa aagaaaaaaa aaatatatgg     3360
   aaaaaattta tagtaattaa tttttaatat catttttttt tttttttttt ttttttattg     3420
   ttatttgaat ttctttttct tctcttcttt tttttttatg tattaataca gtatgtatat     3480
   ttatatatat atatatatac atatatatat ttcttttaat aaaatatata tgaaacttgt     3540
   ttattaatat gtttatcctt ttataatttt gtatatgtta attttttata tattaacatt     3600
   attaataatg tttataattt aattctacaa ttatataata tatatgtttt ttattaattt     3660
   aaacgtcgaa taaaattttt tttttttttt tttttttttt tttttttttt tttttttctt     3720
   ttccttttga tattagataa ttattttagt gttgtatatt gataatacat gtttttatgt     3780
   atactaaggt atcattagca tgtagatatt tattttcttt gttaattttt tttttttttt     3840
   tttgttttac aaatacataa attactaata agcatattaa cattatagac gttatacaaa     3900
   aatatgatac aaaagaaaaa tatataaaaa gatatatgaa aatttaagaa aaatgattct     3960
   ttttattttt aaaataatag tataaatata ataaaattaa atatacatca gttgataaaa     4020
   ataattttga catataataa gtaaggaata attcgaaaca acagaacaaa tttaaaaaat     4080
   agacagttct atcaacttgt gtattttttt tttttttttt ttttttatat atataaatag     4140
   aaatactttc ttcccttttt ttttgtatta tatgttatta caaaatttaa atataatgac     4200
   ataatatatt tttatatacg ttaatgtaat ggtgtaatac ttatacgaac attaatttga 4260
   atattatata attattatta acttaaataa ttttctattt ttggtatttt aacataatgg 4320
   taatgtttat ttaacattat atgaaggtac agaaaaaaaa aaattataat aataaataaa 4380
   tagtataaat aaataatata aaataaataa taataataat atatacatgt atatatatat 4440
   atatatatat atataaagaa aacaaattta aatttaattc aaattgaaca ttaaaaatta 4500
   attactataa ataatttcca tattttattt tatattattt tttattttaa tatatataat 4560
   ataaatttat aaaatttaat tacaagagta aaaaaattaa ttattaatta tacacaaatt 4620
   acatgtatat atatatatat atatatatat atatatatat atatttaaca tgtatctttc 4680
   atttttcatg gtgtaaatta aaactcatta taacatgagg gcatgtcttt tttttttctc 4740
   ttttaactct aatggctggt tatctggtcc aagatcttgt tctccacccc atgataaagg 4800
   gaaccatata ggatatattc tcttatataa gaatatgaaa actttatcat gttgtttaag 4860
   aacattacta aatatacatt tcatttttcc atgagttcct aatgattcaa ctatttttcc 4920
   agttaatcca tgtttggtat gtaattcaac tggtttaaaa taattgatat ctttgggatt 4980
   ataaaacata tttctaatga cagctttttt tttatgaatt ttaaatattt tcccacaaag 5040
   ggatattctc ttcattataa ttcttttaca atcacaactt acaactttac catgcgcaac 5100
   tattacagat gcaacattag cttgggggtt ggaaaaatta ttgtgttcat ttgttataca 5160
   atcatatata tattcagttc taccactcgt aagggaatta tttatatcgt tagaagtatc 5220
   taaggaatat cctaaggttt gttgaaaatc taaactattc aatgaattat tacaataatt 5280
   attactatta ttattattat tattattatt attattatta ttaccattag catagttttg 5340
   taattgttcc atttgttggt acattttttt tttaataaaa aatactggag ctgctgttac 5400
   tgtggtaaaa ccaaatatag aggcaacata ttttttccca tgttttaaat gtttttcata 5460
   ttttcctttc gattgtggtc ctttaattat ttgttcacta aatacaggac atccgataaa 5520
   atgtctaaaa ccacaaataa tttcaaaaat atcctttgat tcaaccttat ctgaataaaa 5580
   tatagttcta tttacttcca tatttaaaac tgtaactttt ctttcaaatg gtaataaact 5640
   tgatacaata actggtatat catttctttt gttgttaagt ttatttaata attttccatc 5700
   atttttaata acaaatatac aataagtatc agttaatgta aattctccat ttactatttt 5760
   acaattttgt acaaattttt ttctggaata tttaattaag ttttcagaac tttcataatc 5820
   atatatacgt gaatattcta gtggcaaatc ttcataaaca tctacatatg aagttctaaa 5880
   actttgtaaa cttcgatatt ttttaaaacg ttctcttgca ctaatatttt tacatatata 5940
   tatattatca gaattattcg aatctttttc aatacagcta tcatcattat aatcttcgct 6000
   tttattatcc gtctcattta cggttatatt atcagttata tcatcagata tatcatcatt 6060
   attttcttca tcctcatcat aatttttgta taaatcatta tttccatact caagatggtt 6120
   cgttgaaaat tttttatttt gcgtttcatt acttatgtta aaattattaa taatgttatt 6180
   attattatta ttaaacatat tattattatt attattgttg ttgctgctgt tgtcgtcgtt 6240
   cattccacca tatttgtcat atgagcaaat aaaatcatcg tttgcttcat gtttttcgtt 6300
   agatatataa gtccacatat tatttatatt actaatgttt gggttagtgt tttcttcatt 6360
   atactgttga ttattaccat gaggatcatt tgtatagtct tgcataatat tcttcatatt 6420
   gtatttataa actgtataac tttcatttat attatcacca ttattattat tattattgtt 6480
   actattatta gttgtagttg tactatcatt cattgtctta gtaatactat tattattttc 6540
   atttatgttg tagttatcgt aagaaaagaa atttgaagca gtaaattcag gtatatcctt 6600
   atttttaagg gtcattaaat tattcatata tatattatta ttgtcttcta caacatacgg 6660
   tcgtatacat ttcatatcat cttcttcatt aaatatactt ttaattggct tatgtttaat 6720
   atcataataa ttatattttg tatgatcatt aggtaatttt ttattgttgg ataaaaaatt 6780
   aagtataaat tcattttcac ttgtttgttg attatttgga tttaataaaa aggaatatct 6840
   taaattttgt tcgtcaaaaa atcttttttc ttttaagata tctattaaat aaatattatc 6900
   gatataataa tcccctacat cagttatatg tataggatta tgataattaa aacccactcc 6960
   ttttataaaa ccttttatat atatacaatc attttcagaa ttatatgcac aagaatctgc 7020
   catcatataa cctctacctt ctctataaga aacattttta attttcatat ttgttatttc 7080
   attatataat ttttgaaaat cggaattttt attttcacta ataaaaaata ttttgtcgtc 7140
   cattgtaaat tcagaattaa aatatctcat aacaaatttg tgagaaccct tattgctcat 7200
   atttgtattg taacctatac caataataga tggaacccct tgtattttta atacagataa 7260
   taatttataa cctaattcat caaaacaaga attttcaata cttccatctt taaataaaca 7320
   taatacaaca tcagcacatt tggttccatc aataattcca tatatatctc ttggtatatc 7380
   ataaactaca aatgattttc ttttcttttt ttttttatca tttgaatgta cagtgtatat 7440
   atcatataat tttatattat ctatatcaat atgttcgtca ttttcttcac ataaatattt 7500
   tataaattcc ttcttaaacg aaagtatatc aacatcttca tggaacacta ataaacatat 7560
   attcaagcaa ttataaaaag gtatatgttt agctttttct tctctctcat tttttaattg 7620
   atcacttttg ctataatatg attgaacctt cctttgtaat atattactat atttttcgtt 7680
   aatatcattt ttattttttt ttttttggat attttttata ctcttcttct tcttcttctg 7740
   attttttatc tttttaaatt gtttattttt ttgttttaaa tgtgatttat gtttcattat 7800
   attaaacaaa taagaaatta taatatatat acaatatata catatatata tatatatata 7860
   tatatatata tatatatata tatatataaa taacttatga tatatttatt tttattttaa 7920
   tttaatattt tcattagaat attatacaaa atttttctga taaaaaaatt tatatttgtc 7980
   cttttgaaaa aatattatca atatatttat acatacataa atatatattt aatgtattat 8040
   attattaagg ttaaagaaaa aatatggatt caatttaacc tttaaaagca ggagaaaata 8100
   taaaatatgt aaaaataaga aattactttt atattataca tatgatatat atattatacg 8160
   taaatttttt tttttttttt tggtaagatg agaattgatt cattttctta aaaataacaa 8220
   ttcattatat tttataaaaa tttgtaacaa tataaaggag gtaatgctgt aaatatatta 8280
   caaaaagaaa ttttattttt atttttttaa ataaatgtta tataaaaaat atatatatat 8340
   atataatata tatatataat tttatagaaa gaaaaaaaag tttttttttt tctcattaaa 8400
   taataccata ttatttatta taatatgtat aaatattaat atttacaatt aatattatat 8460
   atatggtata tatatgtatt gtatatatat aataatatat atatatatta taatatataa 8520
   tatatattgt gttacgtatt aattttcaat aaggtaaaaa aaaaaaaaaa atttaattac 8580
   taaaaattca agaatggaaa atatatatat atatatatat atatatatat atatatatta 8640
   aaaatgtata tattaaaata tttagaacaa tttattaata atttaaaatt aatattcttc 8700
   aaatgtttta tatttaaatt aatgaaaaaa aaaaaaaaaa aaaaaagaat acacaaaaaa 8760
   ttccataaac aaaatattat gttacaatat ttttgttatt atactttttt aacgtttttt 8820
   gtaaaacatc taccattgca actttttttt ctttagtttt tataaaattt aaatattata 8880
   aaatgcataa tataataccg catttttgtt attattaata aattattact attacgtttg 8940
   taatataaga attgatggtt ctataaaaat atattattat attacgatat ctttataaaa 9000
   taaaaaaata taaataataa taaaacattt taaattataa ttgtatatta tgataaaata 9060
   cacattttta aaagtttaag tacttttctt tctttctttc tttccctttt tttttttttt 9120
   tttttttgtt cctactataa aaatatttca gatgcacata tttcttagga ggaaatatat 9180
   tttattaaaa ataattattg tggaataaat ataaaaagaa tgaaaaaaat tatttttttt 9240
   ttttatataa agtatgaata aaagaataaa aataattttt ataagagtat aataaaatgt 9300
   tcattattat agcatatata tattcgcata ctattgttat aaattcttaa aatatttctg 9360
   atctttcatt ttatttttat aaaaaaatag tactcgtcta atcttatgtg tgatggtgtg 9420
   gtggttatat ataaatataa aaaattatta ttatatatat aataatatat tttttttcag 9480
   aataaattag aatattatat atatatatat atatatatat ttatattttt ttatttagaa 9540
   agataatgat atcatttata gtattaaatg gttataatat atgtacatat aaatatactg 9600
   aacattttaa ttatactttt ttttttttct tttttttttt ggtctgagat aacattgtgc 9660
   gctattgtgc aatgttgatt tttttataaa actacaaaat gtaatatact gtaatgaaaa 9720
   taaataaaac acatatatgt aaatataaat atatatatat atatatatat atataatgtg 9780
   tctagaatat aattcatata ttataggaat ataaaaaata ataaaaaata aataaatgaa 9840
   taaaataata tattaggatt ggatttaaac ttttcttttt atttattata tattgtatta 9900
   tatttttata aaattatata tgccttaatt atatatatat atatatatat atatatatat 9960
   atatataata atatgtgtat atttttttat ttaataaagc a 10001
<210> 11
   <211> 895
   <212> PRT
   <213> Plasmodium falciparum 3D7
<400> 11
   Met Lys Lys Lys Leu Glu Lys Gln Lys Lys Leu Leu Glu Tyr Glu Lys
   1               5                   10                  15
   Gly Tyr Tyr Ser Asn Lys Val Lys Met Arg Asn Met Leu Phe Phe Lys
               20                  25                  30
   Ser Ile Gly Asn Lys Asn His Leu Phe Leu Val Phe Leu Leu Phe Leu
           35                  40                  45
   Phe Phe Ser Phe Ser Cys Ile Asn Phe Thr Gln Cys Gln Ile Thr Lys
       50                  55                  60
   Lys Lys Val Ser Val Ser Glu Ile Asn Phe Asp Ser Ala Val Asp Asp
   65                  70                  75                  80
   Val Gln Trp Cys Gly Asn Asn His Met Thr Val Leu Val Lys Thr Val
                   85                  90                  95
   Lys Gly Arg Leu Tyr Arg Ser Ser Asp Gly Gly Lys Ile Trp Thr Asn
               100                 105                 110
   Ile Thr Gly Asn Leu Ser Asp Lys Glu Asn Asn Lys Ser Asp Pro Ser
           115                 120                 125
   Ser Asn His Met Asn Glu Met Thr Thr Val Asp Leu Ile Met Val Asn
       130                 135                 140
   Ser Val Asn Lys Asn Ile Val Leu Ile Ile Gly Asn Gln Lys Asn His
   145                 150                 155                 160
   Tyr Ile Ser Glu Asp Ser Gly Glu Thr Phe Arg Leu Leu Asn Tyr Gln
                   165                 170                 175
   Asn Lys Ile Asn Phe Trp Gln Phe His Ser Thr Lys Thr His Trp Ala
               180                 185                 190
   Leu Val Ser Ser Trp Thr Glu Ala Cys Tyr Ser Asn Asp Asp Asn Ser
           195                 200                 205
   Gly Glu Cys Met Gln Thr Leu Ser Leu Thr Lys Asp Leu Gly Arg Thr
       210                 215                 220
   Phe Gln Leu Ile Asp Ile Tyr Val Val Gln Phe Asn Trp Gly Asp Lys
   225                 230                 235                 240
   Glu Ser His Leu Glu Asp Thr Ile Tyr Tyr Thr Arg His Lys Asn Arg
                   245                 250                 255
   Asn Gly His Gln Gln Arg Phe Ser Gly Trp Ser Lys Asp Val Asp Phe
               260                 265                 270
   Val Ala Thr His Asn Phe Gly Lys Ser Val Asp Val Leu Val Lys Gln
           275                 280                 285
   Gly Asn Lys Phe Leu Ile Ser Asn Gly Tyr Ile Phe Val Ala Lys Leu
       290                 295                 300
   Asn Asp Val Ile Lys Gln Thr Val Asn Met Met Val Ser Thr Asp Gly
   305                 310                 315                 320
   Gly Lys Thr Phe Asn Lys Ala Asn Leu Pro Glu Asn Ile His Glu Lys
                   325                 330                 335
   Ser Tyr Thr Val Leu Asp Thr Ser Glu Gly Ser Ile Met Leu His Val
               340                 345                 350
   Asn His Gly Thr Ser Ser Glu Lys Ile Asn Thr Gly Asn Val Tyr Ile
           355                 360                 365
   Ser Asp Ala Ser Gly Leu Asn Tyr Thr Leu Ser Leu Pro Asn Asn Ile
       370                 375                 380
   Arg Thr Ser Ser Gly Glu Cys Glu Phe Asp Arg Val Leu Ser Leu Asp
   385                 390                 395                 400
   Gly Val Tyr Ile Ala Asn Phe Leu Asp Val Asn Asn Glu Ile Lys Asp
                   405                 410                 415
   Glu Asp Leu Arg Phe Gln Asn Phe Lys Ser Ala Ile Glu Glu Asp Ile
               420                 425                 430
   Ala Pro Phe Glu Thr Asn Thr Glu Lys Arg Lys Lys Gln Tyr Asn Lys
           435                 440                 445
   Gly Lys Asn Glu Asp Ala Val Arg Thr Val Ile Ser Phe Asn Lys Gly
       450                 455                 460
   Gly Glu Trp Ser Tyr Leu Lys Ala Pro Lys Val Asp Ser Arg Gly Asn
   465                 470                 475                 480
   Lys Tyr Glu Cys Gly Asp Asn Cys Tyr Leu His Leu His Gly Ile Thr
                   485                 490                 495
   Asn Tyr His Gln Tyr Ala Pro Phe Tyr Ser Ile Glu Asn Ala Val Gly
               500                 505                 510
   Ile Ile Met Gly Thr Gly Asn Val Gly Ser His Leu Arg Tyr Glu Ser
           515                 520                 525
   Asp Glu Val Asn Thr Phe Leu Ser Arg Asp Gly Gly Val Thr Trp Ile
       530                 535                 540
   Glu Ala His Lys Gly Pro Tyr Ile Tyr Glu Tyr Gly Asp Tyr Gly Gly
   545                 550                 555                 560
   Leu Ile Val Met Ala Asp Asp Leu Arg Lys Thr Asn Gln Ile Val Phe
                   565                 570                 575
   Ser Trp Asn Glu Gly Gln Ser Trp Phe Asp Phe Glu Leu Gly Gln Phe
               580                 585                 590
   Ser Ile Asp Val Asp Asn Ile Val Ala Glu Pro Asn Ser Ala Ser Val
           595                 600                 605
   Glu Phe Leu Val Tyr Gly Thr Arg Asn Asp Val Gly Val Leu Tyr His
       610                 615                 620
   Leu Asp Phe Asn Ala Leu Gly Gln Pro Leu Cys Lys Gly Leu Trp Ala
   625                 630                 635                 640
   Ala Asp Ser Val Ser Ser Asp Tyr Glu Thr Trp Ser Pro Thr Ser Gly
                   645                 650                 655
   Asn Phe Asn Asp Lys Cys Ile Leu Gly Arg Lys Ile Thr Tyr Thr Arg
               660                 665                 670
   Arg Lys Gln Thr Ser Glu Cys Phe Asn Gly Lys Asp Leu Lys Arg Thr
           675                 680                 685
   Val Asp Lys Lys Pro Cys Glu Cys Thr Pro Glu Asp Tyr Glu Cys Glu
       690                 695                 700
   Thr Gly Phe Thr Arg Lys Ile Gly Ser Tyr Glu Cys Lys Pro Asn Asp
   705                 710                 715                 720
   Pro Thr Leu Thr Ile Glu Gly Cys Thr Ser Ser Ser Tyr Phe Tyr Ala
                   725                 730                 735
   Asn Ala Tyr Arg Lys Val Pro Gly Asp Ile Cys Val Asn Gly Trp Val
               740                 745                 750
   Pro Glu Lys Val Pro Val Pro Cys Pro Ser Tyr Ala Pro Phe Asn Lys
           755                 760                 765
   Thr Ala Lys Ser Ile Leu Phe Ile Ile Phe Ile Met Gly Ile Val Met
       770                 775                 780
   Leu Ile Ile Thr Tyr Ile Cys Arg Asn Pro Lys Phe Lys Asn Leu Phe
   785                 790                 795                 800
   Tyr Asn Tyr Gly Phe Asp Thr Phe Glu Asn Val Lys Tyr Ser Val Ile
                   805                 810                 815
   Lys Thr Lys Arg Gly Asn Val Asn Asn Asn Val Phe Glu Pro Glu Met
               820                 825                 830
   Glu Phe Ile Asp Ala Glu Gln Asp Asp Asn Glu Glu Asp Val Pro Thr
           835                 840                 845
   Leu Leu Pro Tyr Ser Ser Asp Arg Asn Arg Ser Thr Asn Lys Asp Phe
       850                 855                 860
   Thr Leu Ala Arg Asn Arg Thr Asn Gln Asn Asn Asn Ile Thr Ser Arg
   865                 870                 875                 880
   Asn Ile Ser Pro Ser Lys Asn Tyr Ala Asp Asn Ile Glu Leu Leu
                   885                 890                 895
<210> 12
   <211> 10001
   <212> DNA
   <213> Cryptosporidium hominis
<400> 12
   ttaccttttc ttactctgaa acccctgatc tcgtcttcaa tattggattc aatcttgggt      60
   aatggatttg gtaaatatct aaagaatgat attaatacaa gtaatcttgc aatttgtctt      120
   ggcgactctt ttgtatactt atgaatcttt tctttaactt cttcccaagt ttgagaagga      180
   aaacacgtca ttgccacatc ttttaaaata tttactgatc catttggatt ttgaagaatt      240
   tttaaaagtt cgtcagtttt taatggtttt aaagaattct ttttaattac atgatttctt      300
   agtaaaattt caatagttaa tatatcacga agttgcatca ttgaaagggc tggatcttct 360
   acagtaattt ccattatatc ttcgtctttt attgcaaagc tataaataat atccagaaat 420
   gaacaaccta aaccctttcc ttggtactga ggaaatatca aaacttgaga aattctaatt 480
   ctagatttag gaattgaaaa aaataaatat gtagtaatta aacctaaaat gataagtgat 540
   tgttctcttt tgacagtagg gagccaaacc gcccacctat cttcttgatc aatggaagaa 600
   gcagattcaa tataccaatg gaggaaccac tcaattctcc tgtgtagaat ttgaaagttt 660
   ttaacatcag gatgaggctt agaattattg ggttccttat ttaggcaaga agtcagattt 720
   tcggtttcag ataaatctat ttttcttaaa tccacttcct caagcccttc atctccaagc 780
   cctgccttaa atatgcctgc tgatgttcca ggaacccata ataatggaat cttcaatctt 840
   tccataaatt cttctttaga gcaaaatcca ccctcgagag gaatggtttt taactgctta 900
   ataatatttt ctatatagta gttcttgtcc tcctcactta actcaatatg ctctttagac 960
   ttgcttttta ttggagttaa atctacttcc acatatacat caaaccaatt gcaagagaaa 1020
   taaatgtgaa ttagcgtgtt ataaccatct agcaacgcaa tttcttcatt gtgaaaaaaa 1080
   tgatgagcat aatatggaga atatgatcct actttatttt caataaagtc gctcaatgtc 1140
   tcacatggat gaagtaaaat tttattcaat acattatcaa ttttaattgg aatggatagc 1200
   tgtgagttag gctcttcgtt gagctttact cgtttattag ggtttgtatc atccattctg 1260
   ctctaattac ttttagaaac ttctcctaaa acgaagaaaa atgtactatt tataattttt 1320
   tttaatctgg taactatttt caactagatt aaatttccca caatttgttt gcatttggcg 1380
   gtaaatagtc gttaaattaa attatttatt ttgcatgcat caacttttga gaaactaatt 1440
   aaaatcattc aactgaaatt gataccgttt ctccatatat ttagaaactt gagctatcaa 1500
   atgcatgtag atataatatc atggattcat aacattttga tgatttctta tatacaatag 1560
   ttaattttct aatttcaaag atttaatgtt tgctcggatt atgataaatt tataaaaata 1620
   gctagacact gtttagactt ccctaaataa acatctgctt taattgatag aggtatttgt 1680
   caattttttg gaggttttta tgtggtaaag gttttttttc tacttatttt ccttgcattg 1740
   atgtataatg tagcaatact tttaccaagc ctggactatc gtaacaatac aaatggagca 1800
   attaatagag cttccatggg aattctttgg gaacagatca gttggctaat tacggtattc 1860
   ttggcagtta ctttttctat tttttcgatc gaatcaaggt tagtttatta ttatgagtta 1920
   ccagtgctaa atattacttt ctcaggtacg tatggtgttt atgcttgtta ccaccgatat 1980
   tatatgactt ttcggttgtg ttaatggggg ttggagtatt aagactaaca gtttcttggt 2040
   gtttattaag tgatgtggta attcgaattg tacacattat tgggctatta tcacgtggca 2100
   tttctttggt aatgttaata agttatgcaa taatcggaat tccagactta gagattcctt 2160
   tggctttgat tactggtgca gttatcttcc aagtattatt tgcaagtcta tcaagctcat 2220
   taagatgccc atgggtgata ttacgattcc tctttattga tatttggatt ggattattac 2280
   tatttcaaat ttttctaaga atttcatcag aaagcaagta ttatcgcgaa atatcaaaag 2340
   acaattttac ttgggaaact tcatctcaat ttcatggaaa tctcacgact attgccgaga 2400
   aatttcagca aattccgtct tggtgggttg ttttttggcc ttgttggttt tggtgtggag 2460
   ttatattctc cttttcgacg gttttttgtg ttcttggagt agctgacaga atgctagcta 2520
   tatttggagt tttttttggt tgtttatcta caactatact cataacttgc ttagatctag 2580
   cagattttct caatgatact atttatctta aagaaaacaa gtatgctgta agttaccacg 2640
   tatatgagca gaactcacat atcaggttat ggcatatttg tattatctgc actcagacaa 2700
   tgattgcagt aatgtctgcc gttatttcgc aaggattaat tcaggaatct gatgaaggtt 2760
   tctctttaag ttccacagat gatcatttta ttacttctaa tataaagcaa tttaaaaaaa 2820
   tgatttctcc atataagata ggatctgaag ttattcttac aaaaatgggt actggcgttt 2880
   ttcataaaat agaaaatcag agtaataata cattacctca aggttattca gattctaaag 2940
   aattgctaag ctgtgaagca atttcaacgc atacattatc tactgtaaat tctcccacaa 3000
   gcactaattc tctttcacct gtattgtctc ataccccaaa atcaaaaaga gaatcctttt 3060
   cagattcaaa ttgcacgcta actccatatg atgataatca tgaacaagta tgtattatct 3120
   gttgtgataa taattgtgaa tcagtatttc ttccatgtgg acatggtgga ttatgcagta 3180
   actgcgctct aagagagttc tatagggtaa gttatatttt taacacaatt tcctaaatta 3240
   tactgtctaa taaattaaag attggtcttt gccctacatg tagacaagca acttctgcta 3300
   tagctaccta taaaagtcaa aggagaaagt ataactactc gagcaagatt aatgcagtaa 3360
   tcattgcaac tcaagatctt tgatactttg cttccattaa aacaaaataa gatgaattta 3420
   tttctttttt attttttcaa ccttttttgc aaaatctaat tattcgatac ctttggcgcc 3480
   atatttttga cacttagaat ttctaattcc aagttctttt attaaccttg gaatttaaat 3540
   ttcttgtatg aattaagact ttgcttaaga atgacactct ttggccaggt attaattgga 3600
   cctcctggtt caggtaaaac aacttttgtc catggaatgc accagatgtg tactgcactt 3660
   aataggccaa atattattgt caacctggat cctgcaaacg aaaatgtccc ttatattcct 3720
   gatgtagacg tgcgagatct gataaacttt gaaaatgtta tgaacgagca taagctcggc 3780
   cctaatggag ctctggttta ttgtatggaa tatttgcaag ttaatattga ttggctgatt 3840
   gatgaaatac gagccaaaag aaagaattct tcatatattc taatagatat tcctggtcaa 3900
   gttgagcttt atactcacaa ctatatttta agagaaatcc ttttagtatt ggcaaaagac 3960
   cttgatattc gtctaactgc agtacatctg attgacagca ctcttctatc atctccaaca 4020
   aattatattt cagcattatt agtctctctc tctgctcaaa tgtctataga gcttccatat 4080
   ttgaatgttt tctccaaaat tgatcttttg gaacatttta aggatgactt accattcaaa 4140
   ttggaatatt tctctcagtt agaggattta aatcaacttt tgacattctg gaagcacgag 4200
   tcgaatatgg gtgatcatcc attatttctc aaatataagg gatttcaaag tgagcttgtg 4260
   gatttagttg aagattccag tattatgcaa tttattccag tagacatcaa tgacaaggac 4320
   tcagttttgc aaattttgca actaatcgac aaatctaatg ggtaagttat ttaaattctt 4380
   aatgatcata tattctaata aagtaaactt aataattata gattttctat gttaagtgaa 4440
   tactctgaat attccgctct tggtatagaa acaaacataa atatgcttcc taatgaagag 4500
   atgtatggaa ccatttatga aagatacatc gaaaagtacc aagaaaatca aaattcaaaa 4560
   gaagaaaagt gaatttaaaa aattaaaatt gccaaagtag tttaaagctt taaataaaac 4620
   cattttacta atttgctatc aaatgcgatc ttaactcgaa atattagacg tcttatttga 4680
   atatttgcaa tagttaacaa ttagaattta tcttcctata acttattatt cctctcctca 4740
   atgatgcttt agaagtttta ctttatatac tatttgaata tctttggcca atgcatattt 4800
   gtgcagcagc aacagcttct gataagtcta caaagcaatg aacaagagga ataatatcat 4860
   agaatccaga tctgagtaga aaactagagt ttggatgaga aaatgaagca aaccaaagtt 4920
   gtatattaag tttctttgtc ttgttgttga atctttgcag gacctgaagg gcagaatagt 4980
   caatttcatt aatcatgcag gcatctatta ttattattct ttctacatga ttttcattaa 5040
   gatcttgtat aattgtagga attctctctc caaaattaat ggaatttgta aagttgggga 5100
   aatattgatt cttagttaga tactcatcaa ttgaatcttc ttggtagttc ttgtttgaaa 5160
   tccttaaatc ttcgtccaac caaacctcga ccatttcttc aaggttttta acaaaccttt 5220
   cagcattaat aaatgttaaa ggaccttcga atctcgcaat acgaacatat ggaagctgaa 5280
   cagcttgagg aaatttgtcc aaacttttaa atattaaagt acctggtaat ttacctaaaa 5340
   tttcaaattt gggcctgtat aaaaacttta tgagatatat aactgaagaa gttagggcga 5400
   aaataattcc atatgttatt cctgcagtaa ctgtcccaat aaatgctatc atccagaaaa 5460
   tggaatcaat gctcctcatt ttcaagagtt ttatgaaata tttgatgttg accatcctta 5520
   tcattgcttg agaaatgatc gctcctaaca ctgcctcagg taaaaacctt atatattcaa 5580
   gtagaaacat tgcaaccaaa agaactccca ttgaatatgc aatgttatga aggggggatt 5640
   tgactccaag agtaaagttc aaatttgtcc ttgctagaga tgctgcgcca ggaagacaat 5700
   tgaagaaagc cccaacaata tttataactc caagaacaag tatttctgtt cctactgaaa 5760
   aaagaaatct gggttcaata tattttttct ttgaaggttg atagtttttt attattgctt 5820
   cagtctcctt tattttttct acattgttct cgtctaaaga tgagctttca aaggaaaact 5880
   tgtgaaatgt ttctgaaata ttgtttgagc tattagtatt aagcataaaa ccctggcagt 5940
   cttcattctc cgagtcatgc gagtggggtt tattatttat tgtagttatt gggtttggaa 6000
   tagatgattt tgaatcaaac ctaatgctta aagagaccct tcgctcttta tccagaataa 6060
   tagaggaaga tttagaagaa gttggcgaag ctagggactg gaaggaggac agagaaagac 6120
   tcgaattcaa atcatctact gaatctaaat ctccctttga aatctctaaa gaagattgtg 6180
   tgcattttct ttgatctgtt tccgggctag tctttgtttc tgatattact tgccccgaaa 6240
   actgaaatga cgttgagaca gtatgtatat ttctttctcc attttgcttt ctataattta 6300
   tgagcctggc tccatctttt tcattttgga cattcatttc cttcaaatta gattcacttt 6360
   ctttaggtgt ttctggcaat ttgtgagctc taatctccat gctttgagca gcagagtagt 6420
   gggtaaaaaa tgaaatgaag gtaagaggaa gagcagctat ccatgccctt gcaaaaaatg 6480
   gaagagagct gattccactt ggctggggaa acgtgcccga gggttgagtc caaatgatat 6540
   tattaggaat aagaaggcta gatttgggaa ttttgccaat gataggaata tcaggagaag 6600
   caaaagcact tgcaacgttt gccattatta ttactaccag ctgggaaggg aatgggaagt 6660
   ttgtcttgaa aatatttttg acccacaaaa tagcttccag aaaaaatata gtagaaatag 6720
   agaaaatagt agcatgccag ttagtatttg atagcttatc caataacaag aaaaatgctt 6780
   taatcggaac acagttagtg tttggaggct caattgcaag cataaaaggg agttccttta 6840
   taataatcga gcatgcgact cctgaaagaa atccttgtaa tacagaatct gatagaaagt 6900
   caacaaagtg agccaatcca aaaattctta aaagacagca ggaaattccg tgaaagaaaa 6960
   tgaccacaga acaaacttct gatctaacat caagaggggc tcggttccct attagagtat 7020
   caattgctgt gcctgcaacg atagattcca ccgaatttac cccaatagag atatatagag 7080
   aagacccaat aaataaatag ataattggtt gtagaattcc attacagaga cccatggatg 7140
   gaactacatt agctaaagaa gagtatgaaa gaccttgtgg aatagaaagc gctgcctctg 7200
   caagaccaca aaaaaaatcg caaattacat ttggccaggt ccattttgct tgctttagat 7260
   actttattcc aattaaagat tcattctgaa aaaatgaacg aatggatttg ataataattg 7320
   ataaaaaact tctccaaccg cttttacttc ttctgtgaaa tctgctgtga agcttaaagt 7380
   cattattaat ttcctcattc ctatgttgat aaagcgtgag gaattcatcc cactcttttg 7440
   ttaaaccact cttatgttta gtctctgtat catttattat tttcttatta ttctcatcca 7500
   tttagatcga tatcttgaac ctgatccaag ttttattgcg cattaatccg tactctacag 7560
   ttatctgata agaatacagg ctatcaaaat ctcataatag gtggaaatcc aacattaaat 7620
   caatcaaaac taaacagaaa attcgtttta tatccttaaa aattatttta taatccgaat 7680
   ttctattata agctttgtgt tccctgattt tattattttt tttccattgc tttatttacg 7740
   ttaagtgcgt taccttgcaa atgcgtcagt gtctactaaa aaaatgttaa attagaattg 7800
   ttgatttaaa aatgaaaata ataataaatt aatgatgaaa aacatctatt taggataatg     7860
   cataccttcc atagatttat tttgtttttg tctcgaggta aaatcttagt tttaggaagt     7920
   tgaaatacgt tgtatattta gcggaattgg atcttgaact tgaataactg attaacaaaa 7980
   aagcagatac gtaaaatttg tgtgttaaat gtgaaatttt agattttatg attctatttc 8040
   atgcattaca cggctcttgg aggtcgtatt aggtgatggt ttctagatat ttcagcttag 8100
   gtaggcttgt tggattgggg gctcaataat gacaaggcaa taagctgtgt aaggaggtag 8160
   aacttaaaaa aggaaaggat agaggtctga taaagttata aaaaccatac aagaaagagg 8220
   ttctataaag aagaagaagg aattagaaga taagtcataa aaatgagaat aaaaagcaaa 8280
   ttttcccaaa cccaaaattg tttataaata taagacaaag taatatagtt ggtggctatt 8340
   aatacctatg agatttcttg aatctcatca tcatcttgaa acatttcaga attatcctgc 8400
   tcatcatcta acccaataat ttcaattaca gagcttccaa aagattgaga attgtcattt 8460
   cctagattca tacaactttc atacctggat ctactaagcg catattgaaa taggatatct 8520
   tctagaagtt ggaatctgga ttcagggctt ccagaattta aggaggctct agcagcagcc 8580
   aaaaatgtag aaatgagtaa gtgtttggat ttgaggtgct gtaaaaagcg cttgcattca 8640
   acctcagcat tttcatctcc aatatgaaca cagataggac acatggtagt cgtatggtta 8700
   ctttcttgtt gagtttctga atattcgtct ttgtgcatag tctcacagtg actaattaag 8760
   gattccacgt tatatttcaa gcaagaactg ttgggtattt ttgaattcat gagacataaa 8820
   gggcagacaa aggtaggacc agtcgtatca gaggtagtgc gagtattaga agggccatta 8880
   ctactttgag catgaagatc attgtttaca aagctttgtt tgcttagaga agcaatcaat 8940
   ttactctctg aggttggaga atcgtcttct ttgcagtagg aacatttaga agcgtggcta 9000
   tttgcctcag atatagaaat ttcagctccg cagtgacatt tagtcttggt ctcattcatt 9060
   gaccttagaa tgctttgatc cacgagttgg tcactttttg acaaccaaag ttcttctttt 9120
   agtaaagacc gacaaatcgg gcattcatgg gagcccctga cacatgaaat gcaaaaagaa 9180
   tgagagcatg aaagtgtaca aggatctacg aatgctccaa gacaaattgg acacatgaat 9240
   tgttctggga tttcaataaa atccttactc acatcatttt tatctgtgtc aagggccaga 9300
   tcagagggtc ttgaaaagga ggaaaaaatg ttagtccatt caaacatttc aatttcaaaa 9360
   gctccaccac attcaacctc ttatttcagt tggaaataaa tacagaattt ttgcttttaa 9420
   tatttaatta cacctagatt ttgagaccgc gcgcgcgttt cccagaatac acgttgtgct 9480
   gcagctacca gttgcatctc cgctcgcgga aatactggta aatataatta aacaagctga 9540
   gagcggaagg gaagtaatta gtttgtgggg aatattgttt tttttgttat ttttcatatt 9600
   gtaactacat tttagcagaa cctctagaag gctataaata gatagtgcga atgaaaagta 9660
   cggtctgtga gtgcttagat aatcttaaga taactgtaaa gggtttagtt aaaagggaaa 9720
   atagtgttta gctgctttat gccttaaaat tataaactct caatatctag tcgatatttt      9780
   agataattaa aaaaatttaa aaaaaatatt ttcctatgaa aataatagct ggagaattgg      9840
   cggtaaaaat gggtatatct ccaacgaatt atcatttaaa ttaaattaaa gaattggtgg      9900
   ggctagaaga ctatgtaaag gaatttatat agtaatcggc agaagaggaa cttatttatt      9960
   gaataattga ctgagaaaga gagataggaa aagaattatt a                         10001
<210> 13
   <211> 10001
   <212> DNA
   <213> Cryptosporidium hominis
<220>
   <221> misc_feature
   <222> (9992)..(9993)
   <223> n is a, c, g, or t
<220>
   <221> misc_feature
   <222> (9995)..(9995)
   <223> n is a, c, g, or t
<400> 13
   atgaaattaa gaatagcagc atttaaggtg ctggtagtag ctaagctact ttcgattcta     60
   ggttttggga ctaattacgg gcttgttaac gcagataaaa aagtcagtgt gagcgaaata    120
   gtgtttgaga gcccaataga aactttggaa tgggcaggta cggaccataa tgtggttatt    180
   gcaaagacgc aaaaaggtca tgtctacaga tcgaccaata gaggcaagaa ctggagagat    240
   atcactgatg tttttgcaaa gatgacctca cactcatctt cttcaagtca ttccaaccta    300
   aatgggtttt ctgttaagtc aattactata aatcctgtcg acaaaaatgt catattgata    360
   gtaggttcaa agcataccca ttttatatca tcaaatgcag gagagacttt tagaagaata    420
   tcccactcag gtactattca tacatggata tttcatccaa ataaagccaa attcgcttta    480
   ttttcttctt ggactgaagg atgtaacaaa tcttttcttc aaaccttaaa gaaaagtagt    540
   tcggctggaa ataagtgtat acatcaatta tttgttacaa aggatttagg tgctacatat    600
   cataaagtta ttgactatgt agttcaattc cattgggatg gaaataataa atcaaaccaa    660
   aataggattt tctttactca ccacagaaag aaacagggag atcaacctag atatggtggt    720
   tggatgaaaa ccatagattt ttcttatact gacgattttg gtagtcatgt cgagactcca    780
   gttaaaggag gaaataaatt cttggcgtca aatggctata tcttcgttgc taagatactt    840
   gactacgaga gacaaactgt gtcactactt gtatctacga acgatgcaaa ttcttttaca    900
   caggttcagc taccacatac attgacagag aaaagttata ctattttgga cacttcagag    960
   aagactgtaa tgcttcatgt taatcatgga gaagactctt tgaagggtac tggtaatatt   1020
   tatataagta actatttggg taccaggttt gctctgtctt tattgaataa cgttagaact   1080
   gcaacaggag agtgtgaatt tgatagagtt atgtctttag aaggagttta tattgctaat 1140
   gttaaggatg atgaacttga aaatgacctt gaccatattt ctacaacttt acatgaattt 1200
   acagaatcag agacagaaga tgaggatgta gaaaccttaa aaactgaaca taaaactaaa 1260
   ataggaaaag tagatcagcc aatcagaact gtaattactt tcgataaggg tggtgaatgg 1320
   aattatttaa aagctccaac tattgatagt agaggaaaca ggataaattg tgacgaaagt 1380
   gatggatgct atttacatct tcatggtgta agtaattatc aaagcttggc tccattttac 1440
   tctgttgaga atgctgttgg tataattctt ggaacaggca atgtaggtag ctatttatcc 1500
   tttgaccatg atgatatcaa tacctacatg tcaagagatg gaggtctcac ttggcaagaa 1560
   gtgcacaaag gagctttcat ttacgagctc ggtgattttg gaggtcttat tgtaatggcg 1620
   aatgatctta agcatacgaa ccaagtcatt ttcagctgga atgaaggttt atcttggtat 1680
   gattttgagc tgggtagcaa aactctacaa gttgataaca ttttaataga gccaaatagc 1740
   tcttctatgg agtttttact ttatggttca aggggtaaat ctggagtctt ataccattta 1800
   gactttaata ctcttggaca agttcagtgc gtaggagcaa caactccaga taggccggat 1860
   tctgattatg agacttggag tccatatgac ggaaggaatt cggaaaagtg tatgttgggt 1920
   aaacagcttg tatacacaag aaagaaacag acagcagagt gctacaatgg tcaagacttt 1980
   agaagacccg tggaaaagaa gcgttgtgca tgttctgaag aggacttcac atgtgaattt 2040
   gggttttcca ggcaaattgg tagttttgaa tgtagacccg aaagtcttga tattgttttt 2100
   gacgacgtca agtttgggca atgcacatca tctggaatat tttatgtaac tgcttataga 2160
   aagctccctg gagatgattg tattggtggt tggctacctc caccagtagc aataccatgc 2220
   ccaagccacg ctcccacaag ttctcatgct agaattgtcc tggtgattgc attatttatt 2280
   attattttca tgcttaagca aggaatgttt ggggattgga ctagatttgg tgaattagga 2340
   tacgacgcat atagaaatgt tcagtataaa gtattaggag tggcaaaaag aaggagttct 2400
   tcattacaag gaattttgag tgctatttct gcatatatta acggagattc atccaaagtt 2460
   gtctttgggt catcaaatat accagggtct ccccctggta cgaagaaaaa taactataca 2520
   actctaaaca actttaatac agaaaacatg tttatggagg atgatgaaga tgaagacgat 2580
   catgctattg gtaatatgac ccaaatgtct ggaaatatta ataacatcaa aagtaacaat 2640
   tataacagta gaattggttc agggtcaaac ctctcacaaa ggcaaagatc aaatagcaat 2700
   aattatattt ctgcaggcca agataatgta tcagtagatc caatttttga cgatcttgat 2760
   gtatttgagg aagacttgga ttcgcatgca atttttaatc aaaacaatgg attgggtaat 2820
   ttgaatccta gccacgatat tatatcgtct gaggctccta tcagttttga taattttaat 2880
   ccgacagaat tggaaatcaa tctatcttca attcctaata catctttccc aaagatcact 2940
   cctccaccaa caaacaacaa taaatctaat attggaaaca actttaatca agacaactcc 3000
   aatggaattg aactattgta gtttttagtg ggcattattt atattccacc ttttttcatt 3060
   aaattaattg tcaagcacgt aatctttttt aacaaaagat tgttttacta taaatgctca 3120
   ctacgttttg atttcttacc gccatccaca atgcataaaa ttaatgtact acggtacttg 3180
   gatgatttca tacatttatt tctcgctaaa acaagagcaa acctataaat tgtcttaatt 3240
   taattattga aaactgacat taatatatta gttattcagt taatgataag taatagaact 3300
   aaaattcact aatacttact aagtttattg ctagagtcaa ttagttgaca aaggagctta 3360
   gtttcgtcta atattaaatt cttgaactct taatattatc cgaattttat gaaaggaaga 3420
   ataggattag ctataccttt gtggtgattt ttttaattac tgtcttgtac ttgtctgtgt 3480
   ttctctttaa aatatcttca atagggccta gaactctttt gaatgccttt ctatcaagat 3540
   aagcaacttt gcatcttcct cgtgccttaa ctgtagcagc tctcggcgtg tttcttaata 3600
   aagccagttc tccaaaataa tcacctctct tgtagctcat cacttcaacg ttgtctttaa 3660
   gagctacagc attacctgta atgattaagt aaaatgtatc tccctgttcc ccttgcttga 3720
   tgatttcttg tccatcctca aagattgaac tctttaagac catagttaac ttgttcaatt 3780
   cataaacatc catagtctta agtatttcaa cttctttaag gaaggtttcg taagtactaa 3840
   ttctctttga tgctgaaccc ttgactatgt gattaaatgt ttctctatct aaagcccata 3900
   acaagcaatc agtttttgca acaacgcttg ctgctcttgg gcagttatac aaaagagcta 3960
   gttctccaaa tgcatctcca gggtttaggt cgcaaagatg tttccttggt tcagtactag 4020
   tctttttgta gcactccact actccttgat ctattatata taatttgtcc ccattatcgc 4080
   cttgagttat aatttcagtg tccttcttta ctgaagtttc cacgcaagct aaaattactg 4140
   ttttcaattc atcatcatct aaactcgtaa acataaagct ttctaacagc ttttcacgaa 4200
   tacgcttttc ctgctccttt gtttttgggt agcttggagg agtaaaatcc ttcatcttgt 4260
   tccatgcacc ataagcttca gcgctcacac ttgttctggg tcctctggct aagaagttct 4320
   ttggtatttc aatattatct cttacatcat cactatccga ttcactttct gatcccgaag 4380
   atgttccgct agtctttttc cttttgtcct ctttcccaga ggacttcttt ggatgtactg 4440
   catccacact ggaagcacta taaaaatttt aaaaattaat aatttacatt tattatacgt 4500
   agatattctt acttaccatg gacatataaa ccccattttc gctctttatg tttatagtaa 4560
   atactatcta taaatatatt ttaatatata taatttatta attctgaatc cgctattgtc 4620
   cttcctacta tgatactctc agtagagtct cgttcggatt actagcgttt ctccttatcc 4680
   tcttatattc ttaagaatta ttttctattg ggaatcccaa gcctgtgcat tttacattcc 4740
   gtatatttac aattgcgtac aactttttag tactatgcgt atgatgacgt gtcttttttg 4800
   aagaaaaatt tatatgtaaa tccaaaaata gcgagacaac aattaagacc tacagtttca 4860
   tttttaggaa tctgcttgtt tttttttacc aaattattca aatctaattt taaatttctg 4920
   atatcttgtc gtattgcaaa tttcttgctt gcatgtaaat tttgcatgca gagtgatttc 4980
   tacatatcgg catctaacta aaaagcgcag gaagagaggg gcaggaaaaa aagcagattt 5040
   gagttattat gtattaaatg tccagtacaa taacaacctt gggacttcag ctgatactaa 5100
   cacctgtatt aagacaacac atataatgta tagaggtctt tgatacagtc aatttttgag 5160
   taatagcctc aaaatcccga gatatgaatt gttcatctca actaaactaa ctgaaaacct 5220
   atgtttttaa gtctaactaa gatctgactt tagtgtcctt caattataca gaatttaaaa 5280
   cttgataagt agcatctgta gcggcatatc aataaaccat caccttatat tacgttatta 5340
   tctacatggt agtcttcgta tgcattcaca aacattctat ataagagatc aagcactccc 5400
   atgaatattt gttcatgaaa caaggaaacc aaatagaaac aatcgtattg ggtaacgatt 5460
   tacaaacgac gaccacgtct accacctttt cttctggtgc tatcagttgg aattggagta 5520
   acatcctcga tacgaccaat cttcattcca gcacgagcta aagctctaag agctgattga 5580
   gctcctggac ccattgtctt tgacttgact ccaccaacag ctctgagttt aatatgtaaa 5640
   gcatgaattc cgaattcctt gcatttttga gcaacatcaa cagctgccat catagctgca 5700
   tatggagagt tctcatcacg atctgcttta accttcattc caccggtaac tcttacgatg 5760
   gtttctcttc ctgacaagtc agttacgtga atgaatgtat cattgaaaga ggcgaatata 5820
   tgagccacag cgaagtttgt ttgtccctca cgaacctaaa taaatttaaa aaaattagtt 5880
   attcatgttc aaattatacc atcaatatta tagagtttcc tccaaaatga cctagttcga 5940
   tgatctaagc aattaatccc tgatcatatt tattttacct tagtatcgca aaaaccagta 6000
   aagtgacgga ttattttgac acttttgttt aagtacagag agtgatatct catttgtaca 6060
   aagacaaaaa atcttaggga cttgcatata attgcctagt cattaaacca cagaattgaa 6120
   aacggaattt aaaaaaactc tgactcaatt catagtcata acatctatat acactccact 6180
   ccaatataac aacttacctg ctggaatggg acatcagcaa cttgagtttc cttcttcttt 6240
   ggagccattt taccgtttat tatctcaaat ttcagctatt gtgagaaaaa ttaatgttgc 6300
   aatttgctta attacaactg gtaacgaatt atcgatgttt ccttattttg cttttttttt 6360
   cggagacaga cttattttaa caggcgccaa ttttaatatt actaatatta ataattatta 6420
   gacaggccaa atataacttc tgtccgcatt attacattaa tttttatgtc aaaagttata 6480
   ctatatcagc gccaatcttt tcttaatatg accttaaaat tcacttttga agacgtatca 6540
   attattttat tatagaatat attggggcct agacgggaaa aatgttaaag caaatgtagc 6600
   tatagtatag aggcatgaaa aaactgagag aatttattac aagtatttaa aagatgttaa 6660
   aagatctaat tagttatttt tgtgtgtttt gtcattaaat ataaattttg aaagtttgta 6720
   taccaaattt aatttagaaa acactttata tactattaat gtcaaggtaa tacttaataa 6780
   gatggatcca attatttgga agaatagtat tgggagccca aatttgaaat attcgagttc 6840
   taggaacatt taattagtaa ccgtaatacg ccttgatatt tgttttagaa cttacctata 6900
   tttagcaaga tatccttaga ttgtatttgt ttatcataag atacattctt taactttgcg 6960
   accactttaa aagtcacaaa ctgttggcct tgttggtcat aacttagaaa tttttgctat 7020
   tgttgttaat taatttttgt atttacaata taaaaactta ctttttggct gttaagtaat 7080
   tcactatttg taaaaattag agagcttgga agtactttat caagtatttt atctaaaaaa 7140
   ctttgagata tattatattc tgaagtttat taatttgaaa ataatttatt aatcttgtgt 7200
   tttgttatat aaactcactt tcttcagaaa ttaatgggat tatattaaat tctccttgag 7260
   gatcctataa tatttgtgaa ttctctcatt tcaaaacaaa ttagccatct ccactcgaga 7320
   acttactggt ccagcagaaa agcactcaaa ttggtatctc ccatcaatga gatctttctt 7380
   attcacattt attattaatg gataatctta tattcaagaa tgaattgtca aatcacacaa 7440
   taattgatca acattactta cttatattca ttttaatgcc ataatatggc tttcctggca 7500
   aaattacaat attattctcc ataccagact tagaaaagaa acccagtaat tttcatgttt 7560
   aacaattaat gttttgtctt attattatct taccttgtcc aatggccttt cagacttgga 7620
   aaataagacc aaaaaggtgc taattaagaa taggtaagca gagaacctac ttataatttt 7680
   cattataaaa tcttaattta actttggtta cttttttcta tctcttcttt aggtatatat 7740
   aattccaatt tttttgtgtg gctataactt gtgtgttttc ctaacaaata ataccgttat 7800
   agggggaaaa aaataccggt atttaatcaa ttgtgtgcaa ccaaattttt ttttttttat 7860
   ttaaaatata tatttaaccc aaatcaatcg aaaaaattaa atatatagga aatccagcag 7920
   gtaaaaaaat tcactatgaa atgcgtccat ctctactttt ggggagttct gtgagaagaa 7980
   aaaattgcat aaaaaactac ttggtatcct cagatactga aattgatgat gaaaataata 8040
   acataaatac tcctgtaaaa aatctaactg taaagctaaa agaggagcac tattatagtg 8100
   ataattcaaa cattaatgaa tgctcctcaa gaccatcaat aagcttagca acatctgtta 8160
   gaagaaaagt gtatcacata gactcaagcg aagatagttg tacagaagac gtaaacttaa 8220
   gctatttaaa cagtcatcca agtactttta cgtctgatat actggaaaat ctccatgagt 8280
   ccccaactca gccttgtatt cactataaaa gtgaatttga gccaaataac aatccctctt 8340
   ctaattactc aagtattgga agtagaagga gaaactgtat atttagtagc gaagaagaag 8400
   atattatccc gttatcttcg aggccttcaa ttaaatatca gtcttcagtt agaaggaaaa 8460
   acaattcaag aaaaattttt acaataattg attcagattc tgaagatgag ttaaacaata 8520
   atgtagcaag caaaagatcg tctttttgtc taggcccaga actaatctgt gagccaaata 8580
   ttctcaacta tacaccagaa caacacaata tctggaggtc atttgactct gatttagaaa 8640
   tagatgaaac aaatgtttct atagatactc tacaagaatt ggagtggaaa acagctattt 8700
   catcttcaga ctttgaagat gaagaagtgg aaagaaaaca tcagattggt tatcattctg 8760
   aggaaattga gtctgaagac aaaactggag aatatcaaaa tactcccaga atggatgaaa 8820
   aagaagtatt accagaaaaa tatagtttat ttagtgatga agatatagag gatcaaaagt 8880
   ctgatttgga ggagatcaag gaaaatgtta gttcaaaaga tctgaactct aatcaagtaa 8940
   caaacttgac aaaagaaatc cccttgaaaa ataaatccga aaaatttgga gcattcataa 9000
   gacaaagatc atcaattgca aaacattggt accaaaagtt taataaacaa gttttccata 9060
   ataggcttcc agaggaagtt ccaattaaat ggacaggaag gcttcaaaga acagcagctc 9120
   aaacactttt tattactaat atagatggtt ctaaaagagt ggtaattaag ctttcaaagt 9180
   atgttttgga ttgtgaattc agattaaaga aaacgctgct acatgaatgt tgtcatgtag 9240
   ctcaattttt attagattct tgtattaagc ctcctcatgg acaaatattt atgaaatggg 9300
   gaaaagtagc ttcaaaagtg tttcctgact taaaagttga gatatatcat aattatgaaa 9360
   taatatacaa atatagatat caatgtttaa gatgttttca gatgtttgga agacaaacca 9420
   aaatcaatga tgaaaccaag attgtttgct cagtctgtaa tggtacagta atatttattg 9480
   gaagaggaac tttaagcaat agcaggaagg aatttcaagt aaaagcacag aaccctccaa 9540
   atacacagaa gagtaaatcc aacccttact ctgagtttgt aaaggagaag tttgcagagt 9600
   tcaagaagga tattagggat ggtagaaccc cctccagaag agctccaagc ataatgaaag 9660
   aaattgcaaa gctttggaaa caaaaaaagt ctgaagattt gattcaagac ttcgaaaagc 9720
   tttccataaa cgattagaat attgaattat tatatagatc tatttatcag tttgcttcat 9780
   actagtttta cattgctctc ttattctatt gatttttact aacctatatt tataaatcaa 9840
   tgagtttatt acgaataaac aaattagtat caaatcaatt attaacgagt attttacata 9900
   tttataaaat gtatatttgt atcttataat atttccgatg ctattcatat gataagctcc 9960
   aatataagct gagtcaatca tgtttttgta tnngntgata g 10001
<210> 14
   <211> 1006
   <212> PRT
   <213> Cryptosporidium hominis
<400> 14
   Met Lys Leu Arg Ile Ala Ala Phe Lys Val Leu Val Val Ala Lys Leu
   1 5 10 15
   Leu Ser Ile Leu Gly Phe Gly Thr Asn Tyr Gly Leu Val Asn Ala Asp
   20 25 30
   Lys Lys Val Ser Val Ser Glu Ile Val Phe Glu Ser Pro Ile Glu Thr
   35 40 45
   Leu Glu Trp Ala Gly Thr Asp His Asn Val Val Ile Ala Lys Thr Gln
   50 55 60
   Lys Gly His Val Tyr Arg Ser Thr Asn Arg Gly Lys Asn Trp Arg Asp
   65 70 75 80
   Ile Thr Asp Val Phe Ala Lys Met Thr Ser His Ser Ser Ser Ser Ser
   85 90 95
   His Ser Asn Leu Asn Gly Phe Ser Val Lys Ser Ile Thr Ile Asn Pro
   100 105 110
   Val Asp Lys Asn Val Ile Leu Ile Val Gly Ser Lys His Thr His Phe
   115 120 125
   Ile Ser Ser Asn Ala Gly Glu Thr Phe Arg Arg Ile Ser His Ser Gly
   130 135 140
   Thr Ile His Thr Trp Ile Phe His Pro Asn Lys Ala Lys Phe Ala Leu
   145 150 155 160
   Phe Ser Ser Trp Thr Glu Gly Cys Asn Lys Ser Phe Leu Gln Thr Leu
   165 170 175
   Lys Lys Ser Ser Ser Ala Gly Asn Lys Cys Ile His Gln Leu Phe Val
   180 185 190
   Thr Lys Asp Leu Gly Ala Thr Tyr His Lys Val Ile Asp Tyr Val Val
   195 200 205
   Gln Phe His Trp Asp Gly Asn Asn Lys Ser Asn Gln Asn Arg Ile Phe
   210 215 220
   Phe Thr His His Arg Lys Lys Gln Gly Asp Gln Pro Arg Tyr Gly Gly
   225 230 235 240
   Trp Met Lys Thr Ile Asp Phe Ser Tyr Thr Asp Asp Phe Gly Ser His
   245 250 255
   Val Glu Thr Pro Val Lys Gly Gly Asn Lys Phe Leu Ala Ser Asn Gly
   260 265 270
   Tyr Ile Phe Val Ala Lys Ile Leu Asp Tyr Glu Arg Gln Thr Val Ser
   275 280 285
   Leu Leu Val Ser Thr Asn Asp Ala Asn Ser Phe Thr Gln Val Gln Leu
       290                 295                 300
   Pro His Thr Leu Thr Glu Lys Ser Tyr Thr Ile Leu Asp Thr Ser Glu
   305                 310                 315                 320
   Lys Thr Val Met Leu His Val Asn His Gly Glu Asp Ser Leu Lys Gly
                   325                 330                 335
   Thr Gly Asn Ile Tyr Ile Ser Asn Tyr Leu Gly Thr Arg Phe Ala Leu
               340                 345                 350
   Ser Leu Leu Asn Asn Val Arg Thr Ala Thr Gly Glu Cys Glu Phe Asp
           355                 360                 365
   Arg Val Met Ser Leu Glu Gly Val Tyr Ile Ala Asn Val Lys Asp Asp
       370                 375                 380
   Glu Leu Glu Asn Asp Leu Asp His Ile Ser Thr Thr Leu His Glu Phe
   385                 390                 395                 400
   Thr Glu Ser Glu Thr Glu Asp Glu Asp Val Glu Thr Leu Lys Thr Glu
                   405                 410                 415
   His Lys Thr Lys Ile Gly Lys Val Asp Gln Pro Ile Arg Thr Val Ile
               420                 425                 430
   Thr Phe Asp Lys Gly Gly Glu Trp Asn Tyr Leu Lys Ala Pro Thr Ile
           435                 440                 445
   Asp Ser Arg Gly Asn Arg Ile Asn Cys Asp Glu Ser Asp Gly Cys Tyr
       450                 455                 460
   Leu His Leu His Gly Val Ser Asn Tyr Gln Ser Leu Ala Pro Phe Tyr
   465                 470                 475                 480
   Ser Val Glu Asn Ala Val Gly Ile Ile Leu Gly Thr Gly Asn Val Gly
                   485                 490                 495
   Ser Tyr Leu Ser Phe Asp His Asp Asp Ile Asn Thr Tyr Met Ser Arg
               500                 505                 510
   Asp Gly Gly Leu Thr Trp Gln Glu Val His Lys Gly Ala Phe Ile Tyr
           515                 520                 525
   Glu Leu Gly Asp Phe Gly Gly Leu Ile Val Met Ala Asn Asp Leu Lys
       530                 535                 540
   His Thr Asn Gln Val Ile Phe Ser Trp Asn Glu Gly Leu Ser Trp Tyr
   545 550 555 560
   Asp Phe Glu Leu Gly Ser Lys Thr Leu Gln Val Asp Asn Ile Leu Ile
   565 570 575
   Glu Pro Asn Ser Ser Ser Met Glu Phe Leu Leu Tyr Gly Ser Arg Gly
   580 585 590
   Lys Ser Gly Val Leu Tyr His Leu Asp Phe Asn Thr Leu Gly Gln Val
   595 600 605
   Gln Cys Val Gly Ala Thr Thr Pro Asp Arg Pro Asp Ser Asp Tyr Glu
   610 615 620
   Thr Trp Ser Pro Tyr Asp Gly Arg Asn Ser Glu Lys Cys Met Leu Gly
   625 630 635 640
   Lys Gln Leu Val Tyr Thr Arg Lys Lys Gln Thr Ala Glu Cys Tyr Asn
   645 650 655
   Gly Gln Asp Phe Arg Arg Pro Val Glu Lys Lys Arg Cys Ala Cys Ser
   660 665 670
   Glu Glu Asp Phe Thr Cys Glu Phe Gly Phe Ser Arg Gln Ile Gly Ser
   675 680 685
   Phe Glu Cys Arg Pro Glu Ser Leu Asp Ile Val Phe Asp Asp Val Lys
   690 695 700
   Phe Gly Gln Cys Thr Ser Ser Gly Ile Phe Tyr Val Thr Ala Tyr Arg
   705 710 715 720
   Lys Leu Pro Gly Asp Asp Cys Ile Gly Gly Trp Leu Pro Pro Pro Val
   725 730 735
   Ala Ile Pro Cys Pro Ser His Ala Pro Thr Ser Ser His Ala Arg Ile
   740 745 750
   Val Leu Val Ile Ala Leu Phe Ile Ile Ile Phe Met Leu Lys Gln Gly
   755 760 765
   Met Phe Gly Asp Trp Thr Arg Phe Gly Glu Leu Gly Tyr Asp Ala Tyr
   770 775 780
   Arg Asn Val Gln Tyr Lys Val Leu Gly Val Ala Lys Arg Arg Ser Ser
   785 790 795 800
   Ser Leu Gln Gly Ile Leu Ser Ala Ile Ser Ala Tyr Ile Asn Gly Asp
   805 810 815
   Ser Ser Lys Val Val Phe Gly Ser Ser Asn Ile Pro Gly Ser Pro Pro
   820 825 830
   Gly Thr Lys Lys Asn Asn Tyr Thr Thr Leu Asn Asn Phe Asn Thr Glu
   835 840 845
   Asn Met Phe Met Glu Asp Asp Glu Asp Glu Asp Asp His Ala Ile Gly
   850 855 860
   Asn Met Thr Gln Met Ser Gly Asn Ile Asn Asn Ile Lys Ser Asn Asn
   865 870 875 880
   Tyr Asn Ser Arg Ile Gly Ser Gly Ser Asn Leu Ser Gln Arg Gln Arg
   885 890 895
   Ser Asn Ser Asn Asn Tyr Ile Ser Ala Gly Gln Asp Asn Val Ser Val
   900 905 910
   Asp Pro Ile Phe Asp Asp Leu Asp Val Phe Glu Glu Asp Leu Asp Ser
   915 920 925
   His Ala Ile Phe Asn Gln Asn Asn Gly Leu Gly Asn Leu Asn Pro Ser
   930 935 940
   His Asp Ile Ile Ser Ser Glu Ala Pro Ile Ser Phe Asp Asn Phe Asn
   945 950 955 960
   Pro Thr Glu Leu Glu Ile Asn Leu Ser Ser Ile Pro Asn Thr Ser Phe
   965 970 975
   Pro Lys Ile Thr Pro Pro Pro Thr Asn Asn Asn Lys Ser Asn Ile Gly
   980 985 990
   Asn Asn Phe Asn Gln Asp Asn Ser Asn Gly Ile Glu Leu Leu
   995 1000 1005
<210> 15
   <211> 10001
   <212> DNA
   <213> Babesia bovis T2Bo
<400> 15
   tgtggtttac ctgttcggag gatacaggga atggctcctg tcgctatatc gcaatgacgc 60
   atttccccgt gacctttttg atgaaatgct gagggaatac ttaggtgatg aacttgggga 120
   agatgacgtt gtgctggaga tcttagagaa tgtggaaccg aaggttccca tggagaccct 180
   tgagggctat atcgaatcgg tttggttctt cgaagttcct ggcttcggaa agattgcacg 240
   ttgtgttatt cgtagcgttg agtacgatat agatattaat ggagtgagca aagaagttga 300
   ggaaggacat ctcgttccat atttacagca tttggatgac acaacacgtg acgaagacga 360
   tgatccggaa caatgggatc gacctccgcg aggaaatgca aggaaatccc aaaatttcgt 420
   tgtatacgca cctttggatt cggtggaccg tgagtgcgaa ttcattctac ctatggagtt 480
   tgtgaaaaaa tatctgagcc atacatggcg tcattcaatg caatgtatgt ggcgtggtga 540
   aaaggcgcag gttattcgag tctgctatgg tctcgctgac atctggcata tgctagagat 600
   agacactgat aatggcatcc acatggttaa cctctgggat gttactcctg tctaggcatt 660
   tttgccagat ataacaaatg ctcaaagtaa ttgtactcaa acaacaattt tatgtgtttt 720
   ccataatagg tatacattga cctgtatttg ttgtccaact gcattatcta ggttatgcca 780
   atcatgactg cggcctgtaa ggccactaat cactttgcct tttggtccca tccacatgtg 840
   gtgatattca tgtgattgga tcagccgtgg tttggtcgac ttttctggga taaagatact 900
   catccacaag acgtcggcga gtgccgcgtg ataagctttt ccactttgct agtgtttgat 960
   atttatgccc tattggccta ggcagtggct taggtgcacc gctgcgtaac cagttgtcat 1020
   acttaacgcc aagatcacgc tcaagcgttt ttgtgcgata gcggtggttc acaaaggatt 1080
   cgaggtagta tggatcatct accactcgat attcgggata tttgtgggac aaataaaaat 1140
   cacgtatagc atatgcgtgc tgtgagtcag cagatgaaca ttcccgctgc aactcgtcca 1200
   cacgtgatag attcagagtt tgccattcat cgttgggaaa taaccccaac ttctttaagt 1260
   catcagataa tctacgtata acgtgctcat ccatcgtttc tataggatgt ttagcacaat 1320
   catccgcacc aggatccaat gaacaatgta cctccttggt tgctgcatct ataaagtaaa 1380
   aatcacgtat atcagattct aatgcaatcc gatgttttaa catatagttg tctaccatag 1440
   ttgaacgagg caaaccttta aatactccat acttccacca taaagcagga tcacggaaat 1500
   cctccaatgt aactccagca tattgcaact tcagagctat ctctagcatg cgtctaaata 1560
   cacgtttaaa taaccccctg taacgaacaa attcccgacg cttccaaaac tcctgtaacc 1620
   gtttcaacgt ataagtgcga tcgataaagt gcggatgttt agcaggcttc tcaattatac 1680
   gttcaaaaaa tattaccaag tcacgcgtag aatcacaatt cttttcagaa gcgatcttac 1740
   gtaaacgtgt taatgacgac aagagactgg catgaaatga agggtcttgc tcgtactcca 1800
   aaataagcgt atcagctgtg attccagcct tcaacatttt acttagacga acgataggct 1860
   catctcgtat cacagagaga gcatcatcgc atggtaacgt gtagtattca cgaggagcgc 1920
   cgtaataatc ttgtatatta ccataataag gagtagggga cggatatatg gcagcgggag 1980
   tctttttctt tggacgcctc ctgtgagatc gacgggctcc tttcagtcga ttcatagatc 2040
   cgtacaaatg catctctctg catcgccaat gtgggctgga tatacctaaa taatcgtcat 2100
   gtacgccact gtatggtcga aatgactcgg tgatgcagat tactgcctgt agcagccaaa 2160
   ggatataacg catttgattg ccttcttacg acacatatgc caatggtata acttccaagc 2220
   cactctaggc atttaaaatg atccaggatg ccataactac agtggcaact caattaatgg 2280
   gcatttatag ctatgtctaa tgtaacatga taaagacata tgcgtcacac acagttacat 2340
   ttttctaaca tctttgatgt ttcataaatt acagtgattg gttactacaa ttcatgcgtc 2400
   gcacattttc tcaatggaat accttaagga atatctacaa aacgcacacc tacaatataa 2460
   gacaatacta atgcgcatat ggattctaat tcgtataatt tatagcataa aaggtgtatg 2520
   tgattataat aatagttcga aaggtccaaa tgcgttgttt cttcttcaca ttcatatatt 2580
   tgtatacgat gcatggaaca atatatacca tatgttaaag caaatgtagc acgcatctga 2640
   acattatgca attttggttt gctagaagta tagttccagt ttgttgatat aggttatatg 2700
   caagctaagc atatataatc gtttcactaa atttcagagt aacaaaggga catcgctaaa 2760
   ggtattttga agcatcagga aaacaccact gaaagtattc gtgcgcatgt tctgaacatt 2820
   tgatatagta acgcaagttg attttaaatt gtagtaagga tccactatcg atgtaccaca 2880
   gtagatatca catattcgat atggaccttg cacaacgtcc gataatgcgc atatgctgtt 2940
   caatgtcatc tagatatcta gaagtgcgat ttcctgtttg agaagaatca aactgcttct 3000
   ttacatcatc cagcgttctc ttgaaatcta atgaatccgc aattattcgc tggttagtca 3060
   acgacacaca caacttaagt aacaacacct tacgttcatt caaatgcgat acaagctgaa 3120
   aacgttcctg taaaagactg tccaaaatac cctgcctttg ccgaagtacc ccttctagct 3180
   tggacacaag actgcccaaa acagataagt tgtcatggtt tattatatat ctacgattaa 3240
   tgccagctct aacagacact aaattgtatt gaaacaaatt gggtaattaa ctcactggat 3300
   atattcggtt gtgagctatt ggtagcacaa actggtggtg gagatgaaac gagtcctgca 3360
   cgcaatttac gcatttaaaa atcaaaatac ctgccgcaac aattctattg atccccggag 3420
   tagccccaat aggtgtatga ttgtagattg catgagattg atgaggacag ggttcaagtg 3480
   gtcttgtcat acttttcaat gtctcttcag taaaatcaat gccaccaaca acaattgctc 3540
   taccggcacg tgtatcaccg ggaggtgatc gcgttgcctc agataagcaa tacctgttta 3600
   tttcgtgtga aaaggcttcg tatacatccg taaaatcacg tggaacagcc aatgcatccg 3660
   actggtttat atataactta aaaacattta atgtgcgctc aatagccacg tagtcaaaaa 3720
   gacgttgaaa ccaacgggtg cgattcaggc gtgacaacgc aacgtttcga ggcatacctt 3780
   gacgctgtaa ggaatccaat atccactcga gaagaccagt gaacatctgc ctaacatatt 3840
   tgcgtagagg agtaattaca gtttgcggat gcaaagcgaa taacaccact tcttgggatt 3900
   gtgggactcc cttaattaag cgggatgtgg taacattgat gttgtcatct ccaaaaagcc 3960
   gagcagcagt actttcacgg tacgccgatg atgatgctgt agtaaacgat ggcttgggcg 4020
   tagggtgaat accggaagtg ctggtgtact tcggtacaga tgcagttcct gtgatacttt 4080
   taaagcacta taaacacaaa tgtacctatg cgcctgcgtg tagatgaata cggtggtacg 4140
   ttgtgactgt ctccagtata gatagggtga agtggtgtag ttgatgaagc aacacgggtt 4200
   gtagatgtca tcggaccagg atattcagtc gaagttggcc agccagaaac cgatgtgtac 4260
   atttgccgtt tagatgatgg tgccgaaaga acacacttca tatctccgcc atacaagtgg 4320
   ttgcctggac cctgctgaga cctgtagcca actccaacga tggaacttaa gggcatacga 4380
   ggccgaggcg tttcaccaac gaatccacgt gccctttcat gaggagtgtc cacatcgggt 4440
   gccattttgc gatatgttgg ctgtacaata atgaaatggt aaaatgaaaa tcttatatat 4500
   acagtaacgg ctattgcata tatcccctaa caagctctta cattggtgaa ttagatcaaa 4560
   catatgcata atactctatt aaactgaagt aacacattat atggttgtaa catcatagca 4620
   cgtagacaac gtaaagataa tgttataact taatacggca tcaacctgta gccaaataga 4680
   cataccttaa tagccaaaaa cctttttgta attgatatca agtgcttata ttagcgctcc 4740
   atagaattgt tcacgtgagt aatctatcgc gcccatatac attacatttg aagtgaatga 4800
   cccgcgcaca caatccaagt ttaattggaa ttatacctct ttaattttca aaaaaatgcg 4860
   aaatgtgtag tacagctccg ctgcaggaga atatataata tatactatag aagtgcataa 4920
   ggattggtaa gccggtttca cgcgtccatc ataaaattaa aaacatcggg atatattcgc 4980
   attttaaaac agctgtaatc aaaccatttg ctcctactaa aacttaagct tgatactacg 5040
   aagattcact accgctacat tttaaaatgt caatagacgc gaaaaggaaa ttgtcataat 5100
   tttattatgt attcgatcaa aagttaaaat atactaacat caccactatg gatttgcatg 5160
   gtaaaatata tagcctgaaa taaccgatac taacagaata taaacatgat gtttgcattt 5220
   tattccgtaa aggtgaaaag acggtgaaca caagggaata tatcctttcg aacaatcatt 5280
   gtatctatgc gactcctcta aaatagattg atgttatgat catcaaaagt cgtcatgttc 5340
   taacgctcga tccatagcat cctgattgaa gtgaaccaac accgtagagt caatttcatt 5400
   tgtcttaggt tgattctgta gtaacttcat tatcgccata tcagtcctgg tcgctaataa 5460
   ctgtcttgag tcgcgaaggt cacgcttaag atcccaattt tgcttgctgg gtaacaccaa 5520
   ggaaagtaca tcctataagg gttttggtta atgtatacgg tcacacacag atataaatgt 5580
   acacatctta gataacaatg aacgtggcat ataacgacat aacacatcaa agtgatacaa 5640
   cgtaccttat ttgagtgttc ctctataatc ttatttatgg cactatctat gtcttcctca 5700
   atctgccgat aatcttccaa atcgctacgt acaaaacgtt tcaaattctc atctttaggc 5760
   acataatgat gaaagacaag cgactttagc ttgtcctcca tcgcacagcc accacgcgct 5820
   gcgattgttc agaaatcata cggtgaactt aaaataatta aacaatggta tagtgtattt 5880
   tatatgtgcc agtcacatcc cccgattttc tgaggagtta taaagtgatc gacaccgagt 5940
   actacatgtt ggaggggtta attatagcgt tgatcaaaaa taacccattg gacgatgaac 6000
   gcagttccaa tgatcctaca cgctcaagcg gtacacgtgc gtgaagctag aaaacataat 6060
   acataataga agtcaaccta cctcgagtga ctgtcctact tgaagcaact caccctatat 6120
   atattcaaca agcataatac agcacttact tcaagcattt ccacattgtt tatgagaagg 6180
   gacgcagatt ccacattagt acataacgca gaaagtaaaa cttcaacatt gtatacacca 6240
   tgctcaggga catcgatggt ccaccgttgc ccctggccaa gaggtaatcc agctttctgc 6300
   ttcaataaca aatcatatac actatgattt ttagtcgtcg caggtagttc tgttaattct 6360
   ctatctatca acggtacaga atccaagtta acatcaacta tgtgattcac ttggggttta 6420
   catatctgaa gatcagctat atcagccacg acatttgatg aaccccaacc gtaagacaac 6480
   ctgccatgat atcctttcat gtgtcccaaa tccaagtgat caggaccaca tgcgtgcccc 6540
   ccttggcaga aacttatgtg aatgggaaaa tgtactgctt cttccactgt tgtacgagca 6600
   ccgcattcgc gtccataaaa ggcgaatgtt ccgccaatta tttctttatt atcaaccgta 6660
   tcggaacctg taataccgtc tcctgggctc attgtttcaa tcataatcct aatggaatcc 6720
   acaacgccgt gatatcccaa ttcgtaacga agctgtgaat gcagctcgaa atcttcctca 6780
   gatccatcag gaaatgatat aattgcgcgc ataggtaaac tgccatctat cggagaaaaa 6840
   tggaagtgaa ccaactccac agggttggtg aaagtgcgca tcagccactg tccagtgatc 6900
   cgcccagaac tcggttgcca gtatccactc ccaaaagctt ttgagcagtc aaatgcagct 6960
   gaaagtgatg aagaagacgc acaaccgtca ccatttttga aaggaagata ctggacatcg 7020
   tcggggcata tggcattagc cgcagcgggc tgcatgaaca agaaaaacga cgtttcaggt 7080
   aatatgtgaa tttttacagg tcctgccttg aatcgcttgt agaaaactat aaactcctgt 7140
   tgactggtgg cctcttcgca attgtggcca atagaataaa ctgatatcac ctcccagctt 7200
   ctatcgaacg tggacatatt ctctgccata ggaatataag acagatgttc agattgagca 7260
   acatatagaa aagcatcatg cgaaatgtaa aaatccacgg tatcataatc aggtttcact 7320
   actgtgcgta ccatacgcat gcctcggaga tggtcaggta cgtctgccaa caacaaattt 7380
   gttacgttaa taaatgcctg tgccccgttc tccaacatgg ctaagtcaaa ccatcgcgca 7440
   tctaatccac tgataattaa agattcacca gaatctctgc tacggaagaa ataatccggg 7500
   tcgataatcg tccgggaccg cgtgtcagtt tcccaactca atatcaaacg aacaacgttt 7560
   tcatcacgat acttcagtga gctttgatgt gaatattcca ataccaatgg ataacgcttg 7620
   cccccaacca gtgacatttt gcgagaagaa acgagagtcg aggttgttgt acggccgtca 7680
   agagcaacca cacccgctga attatacgcc gatgcaaatg tacccctacc ctcaagtcca 7740
   tgcttaatta ccaactctcc gtcgacgaac atcaaaaagt tacaatcagc atccaaccaa 7800
   aaggtatagt ctcccgaaac aggtacgcgc aaatatgtct caaaacgtac ggaaaaggaa 7860
   cgaatgtcta caccatttat aggtaatatc tcatcaaatg ttagagatag gctagaatga     7920
   tcaaagaaac caacaggtgg cccccttaga tatggattgt caaagtagct ggcacggaca     7980
   ccatctccca ccacctatat aacaataatg tatttaccac ccatgtatca cctaccgcaa 8040
   cggaatagcc ttgaacaatt gagcaatttc ttttgtcatt taaaacgctc ttacgcgtaa 8100
   tctcaagctg ctcgctcaga gattcgtatt cctcatttaa catgtggaga ctttgtgagt 8160
   ttgcctcaac ctgctgtaaa gcacgctcca aaccacggag agagtgcgat attgtgttca 8220
   tatcattttc atgagatgcc gttccgtggc cacacacagc atcaaaatgt tttagcgtat 8280
   tttccaaacg tctgcggatc tttaacaaaa agattataac ctactgttca gttgcagaaa 8340
   gttgtaacac tgaatgtgcc aactcttcag atttcagttt cgataaaaga gatgccttgg 8400
   atcgaatgac atcatagtct actgtgccgg cacaaaagtc ccaatctctg gagcccacac 8460
   caatcaactg tacttctaca taacaccatt ctcgaccaac agaaccatct ggagcttcag 8520
   ttgttgtaca gtccgtaaat gtattcccat tgcgcacaaa agctgctgca caaagcatac 8580
   catcaacggt cttcctgtga cgttgtctaa attcggtcaa tttctacgta aaataagtat 8640
   tgtgaagtat atacaaatta ccattaaggc ttcccgatca tagtcggttc ctgaaggtat 8700
   ggtgaaatcc ttaggtgaaa caacatcctg atttatatct ccattggcaa atacagtatg 8760
   aaacacgaaa acgcagtata agaatctcac acttagcaat gaataatacg aaagggtttt 8820
   aatgaatgcg ttttgacgca tacgcgcaac gccttggatt gtagccataa gatccaatca 8880
   gcgtctacag ttggctggaa ttaaacgcgc ctacaacgcg acagcaaagg ctgcacgcgt 8940
   tttgcgttct aatttctcga tatatcgttt ccagtcttta tacatgttgt atttcacata 9000
   gtagcggttt ttataaggca ctattacctg atcaagatac aggcaacgca tacacaccga 9060
   acagaagtaa ggattagata caggcactct accgacgtta tctcgtttaa ctgaggcttt 9120
   aaaattacga gattatcgat aacggggtgc tattttgact atacgaattg tcgtatttgt 9180
   agtccgtaat agttatcccg gtgaattatt gtccaaggaa gaacaggaaa tgacgtgtta 9240
   gtacgtatgt atatgcatgt gtgtattcat atagaaatgt cgatattgat agtaattgta 9300
   atgcaatgat gttgtacgat gagagattac acacctatca acacaacgta tatacaaaag 9360
   taagggggat ttttatgaag tgaattagga ttctgatttt tatatgttaa ttataaaata 9420
   tgtgaactga aatgtagacg gtatatcatg tttggtctca atgtaacata taagcgccgg 9480
   tacacattca gctgtctatt atctttaaca cgtatataac accggccatt cgacagacta 9540
   tagttttaaa actttttatt tgtattatta gtaaatacag agtagaatga ctatcaatgt     9600
   gtaatcattg gaatgatcat ttgtgtaaga cgttataaaa agaaggggaa attgatttcg     9660
   gtatgccttt tttgtaatca agtaaaaggc aaacctctag aagtaaattt aacaaggacg     9720
   ctacttttca aaagacgcga agaaaaacta tgtaagaaaa agacaataat ggtggtacat     9780
   cacacactca tgacgctgta tgctataccg atgcgatgta acagtgattc tgcaaactaa     9840
   gatgacacag cgttgttata tgttgttaac gtgaaatgta cggtagtaaa atggtaatgt     9900
   gcaaattgcc ttagtggaaa tatgagtcac agtgggtgta gccactcacc cgataacatt     9960
   gtacccgcgt cataaccatt accaattttg cggcaaatat a                        10001
<210> 16
   <211> 10001
   <212> DNA
   <213> Babesia bovis T2Bo
<400> 16
   acaacacgca acgccaagta ttatcggcaa gtgtatctgg gtagtgaact ttacactccc 60
   gtgtgtgcat agcacacgat cctatcgcaa cgtagtcttt gactagagcg gatgcatcaa 120
   aggtgtggaa gactaaagaa aaacgagcgg tacatcgcgt ctctatatta tagtgaatcc 180
   ataataaatt gtgcaaaatg tcgtcgctgt ctatccttgc agttatctcg gcggtgatgc 240
   tacgagtcat cgccgatgac aacgttggca caccggtacc agagaatcat atacattctc 300
   atcttgatcc aacactgttt ggggatcatg atgatggaac acacggaact gggactgttc 360
   gcaatgaacc agtgcccgca acaccaccgt aagtctacag tgttataatg actggttatg 420
   aatcgtgtct attttgttct tttacttttt gtatctctat tatgcacgta ttgcaacgtg 480
   taaagggcgt gcattcgtta gagccatctt attagcaagt tatttgatat atttgtgact 540
   ttcgtcgacc atgatgatta tttttcttat agtatgacat atatattgta ttacatagtg 600
   tgcaatggta acctttgtct tacattatac tcgtttgtat ggactaaatg cctatgcaga 660
   ggaaccccgt tacgcaatga tcaaccgcca ccttcgggtg gtggtaagaa agtctcggtt 720
   acagaaatta gttttgatag cctgatagat gacttagtgt ggtgtggtaa tgatcacaaa 780
   accgtcctcc taaagacaca aagtggtaga ttataccgat cggctgatgg cgggaaacag 840
   tggagcgaaa tcacacatct attccaagga actgctcgga acggaaatta cagagtcgat 900
   tctattgttt tatgcgaaac tgatagaaac gttgttgtca tcgttggtga ttctagaaat 960
   cactttatat cggggaatgc tgggcagtct ttccagccaa ttgcgtatga aggtcctgta 1020
   aatatgtggg tttttcaccc ttctaagccg tcgtgggcat tactcagctc ttgggaaggc 1080
   gcatgctttg caagtgacaa tgatgaagat tgtgtccact cagtgtttgt tactagagat 1140
   atgggacgct ctttccaacg tgtatccaaa tatgttgcgc aatttagctg gggagatgtg 1200
   gcatctagat ctgaagatcg aatttactac agtagatatg ctctagaatc aggcgatcag 1260
   cctaaacaag atggttggaa caataacatc agcttcatgt acacagacga cttcgggaat 1320
   gaaaatgttc tcattatgga aggaggtaac aagtttctgg tatcgggcat ttatgtgttc 1380
   gttgctagag tgtccgatcc cataagacaa acggtaaatc tttacgtgag tacagataat 1440
   gcggagacat ttgatagggc ccagttacca gttgaattgg aagaacgcag ttatacgatt 1500
   ctggacactt ctcaaggtgc agtaataata catgttggcc atgaatacga gggaggtgat 1560
   gtagaagttg gtaacgtcta catatccgat gcatcgggat tgagatattc cttatcccta 1620
   cctaataata ttagggctgc ttcaggggaa tgtgaatttg ataaggtata ttcccttgag 1680
   ggtgtgtatc tagcgaactt cagggatgac tccggtggtg tcctcaatcc aaggaataaa 1740
   ttcaagacgc atattgacgg cactaaaact cagctggacc aaaaacgtgg caggcatgtt 1800
   gctcatacaa aaatcgaacc agatatacga actgtggtgt ccttcaacaa aggggcggag 1860
   tggcattatt tacaaccacc taaactggat tctgaaggca aaccatatga ttgcgaagag 1920
   gggaaatgtt tccttcattt gcatggtata acgcagtaca aaaattttgc tcctttctac 1980
   tccgttgaac atgctactgg attggtcctc gctacgggta atgtaggagc tcgtttgcga 2040
   tttgatccat cccaagtcaa cactttcctg agccgtgatg gtggtttgac atgggtagaa 2100
   gctcataaag gagcattcat atacgaattt ggagattacg gtggtcttat tgtcatggca 2160
   gaggatcaac gtaaaactaa agaagttgtg ttttcgtgga acgagggtgc cacctggttc 2220
   gatttcaatc tcagcaagca tgagcttaat gtcaataatg tggttattga acccaattgt 2280
   tcttccctta actttatttt atatgggaac agaaatggta tcggtgtagc atttcacttg 2340
   gacttcagtg ccttggggca gcccttgtgt aagggtatct ggagcattga ttctacttca 2400
   agtgattatg aaacttggcg tccaactgat cctcatggta atcagtgtct tcttgggcgt 2460
   aaaatggcat ataaacgacg caaacaggct tctgagtgtt tcaatggtaa ggaattcaaa 2520
   gcaactgtag aacgagaagt ttgcccttgt accccggatg attatgaatg tgaaataggt 2580
   ttcacgcgtt ccattggtag taatacgtgc cgcgtcgatg gaacatggct tatgcgtgag 2640
   ggatgtacat catctagctt cttctggacc gatgcctatc gtaagatacc tggagatgtc 2700
   tgtacatccg ggtggattcc tcatcaagtg gccgtaccgt gtccaccaca ctcccctctc 2760
   tcaaatggta gtaagatggt actgacaacc attctggtcc tttcgttcat aatgttggcg 2820
   attgtctaca tctcccataa tgagaatctg aagcatctat tccacaacta tggattcaag 2880
   caatttagtt atgttgcata tacaccagtg aactctaaaa agcccatgta tcgtggagcg 2940
   ggaggacgat ttgaacctga actgggcttc atcgatgctg aacaggaaca tgatgagcca 3000
   acacttctga actatttaag cggtaatcgc atgggtggta attcacatca acagcccacc 3060
   aaacaagatc agcagcaaat cgaactactg taaaaattat aaggcaagta aaacatatgt 3120
   tgggatgctt agtcgcagtt ctcttcgtat gacatcattt ttcactatcg aaatatagtt 3180
   atttttacta ttttatcgtt tattcagttg cttatgacca aaaaatggta gtcttctaac 3240
   gtattttgat aactctcatt tgcttatgcc attggttgat ccctactcat acgaattcaa 3300
   cttctgggta ataatagtgt cccattgatc atgttacgaa caatcaagtg ctttgtgttg 3360
   gtataatggt gttagaataa ttttaaatgt ttacttttat atcatcaatg aatgtgggaa 3420
   tgtagttagc atttaatcaa gctgaatata taataattat gtattcggtt ttatatgcag 3480
   aattaaataa ctagagtgaa cataaataaa agattatgaa tgcagcattt cctttatatc 3540
   aacataatcc ggaaatcagt ttgaaattca tgtcgttcta cgattattac gttataataa 3600
   atggcattgc agagtggctc aaagtacaat aacaaataat ttatttgtta tatatatata 3660
   tatatatata agaacaaaat gcatgtggtt cgattttccc ctaacgatga tgtctcaaca 3720
   aataggggga acaaagatat tattggtgca caaaattcca aatggtgaac tggcgttggc 3780
   agattggtga ttaagtgtca agtatatcac gaatatatta tgaatatgta tgtagatata 3840
   atgttattat gttattctaa agaatagttt gtcgtctgtg gaaactttaa acaaattata 3900
   ctttcttcat gcgggtgcgg caatctttgc atccaataaa gaatcctaat tcgtaccaaa 3960
   tgctatgcac aatatgtata gtctgcgtta ttatcttata gtaaatatgg atggatgatt 4020
   cggaatctgt gacatgcagc atcgggttaa gagatgggaa gtattatttc acagcgcaaa 4080
   actgtatctg gactaactct agcagatgca attcggtctt gatatacttt gtagtcatac 4140
   tgttaatgag attttgcaga gagtaaatga tgtgataata tgaaaattta cctcagagga 4200
   atgagctgca tttatcatat gacaaatacc cagaatgacc gtgcgaaaca ccttaataga 4260
   ttacttatac atattatatt ttgaatttac agtcttttgt cctgtatatt tgataaatat 4320
   aacaagcacc acaaaatgtc accttggtca taaagatcaa cgaaaaaaaa aatcaacata 4380
   gataaaacga actgcatttt tttttctaaa actacatcac tacatcatat aacgcctgaa 4440
   ttgattgatg tttatatcga ttatgatgaa tcatcttttc aaatttaatt gttcagcgaa 4500
   aatacaagaa taaacataca taccattaac atccatcgtt gaccataatg atcgctgtga 4560
   agttcggaca tcaatcattc ctaattgcct gctatattgt tgaatgaatt ggagtctttg 4620
   tatgtccaaa atgaattcta acaacctaca tatccacctg cggaaacagg cattattgtc 4680
   ttttaagaga tggaatccct gtcacaacca aaaatagcta aaattcatgt gatctgagta 4740
   tataaacttt tctttaatgt taagaaatga aaaatagaaa tacaagggtt ggaaccataa 4800
   ggtttgggat ttggtttcca tatgaagggt gctcccacgc taactcaagg tagcatttta 4860
   cagaagacag ctcttcaaga ttggatcggt aaaatgacta catagaaatc cacatatcat 4920
   atgtatggtt atcacacata gaacgtgcaa cagacgtgat taggtatctg catagaaata 4980
   gtatataccg ccaaacaatg acatagggtt ttgtatatat tctttgtttc ctgtagaggt 5040
   attgttggta taaacagtaa tgcaaaatga caacgcaggc aaagaaattg tgacttgggg      5100
   ttatgtatga ctcagccgaa aaggcctcta tgtatacctt cattagaaat ataatgttca      5160
   ctcatgccgt gtatattaca tacgtctcta cagttgaatg gatgtgatgc cttaatcata 5220
   tatatttaaa accgccattt tatatttgta aaccacacaa aaaattctga cgttaacaaa 5280
   aggccttata ttagcataga cttaactgca ttatcattta agaaatattg ttggtggtta 5340
   acgtattcca aaactgtcga tgattttgat ccccaatgca tggcagaatt actaacgtag 5400
   gacattctta tgcaccacat tattagaatg ctatatatga gttacctagc aggataattg 5460
   gaataacaat tacaagatat taagatatat ataatcaagc tcatgattca tgttcccaat 5520
   gggattgaca agaaaaatat accctgtgat ttagggggag ttataaatag ttggtcacaa 5580
   cttttctaat gaggataaaa cggtttcgtt acttttggaa tttcggtgaa tctcatggtt 5640
   ggattattat ttatgcgtag acatttgatc ttgaacaaaa gattatcaac agaatagtct 5700
   ccctttaatc tatcgatgtg atatgggaca taatcatatt gtagaatacc atcgatcatg 5760
   tatctattaa acgcccaatg gtttgttgtg aatctacgca taatggaaga acgacattta 5820
   atgaaaccgg ataatagggg gtggggcatt cataaacatg gggccaatag cacattgtag 5880
   catcttaaat atatgtttcc cacatactga tgtatgtggg aaacatatat ttcgacatga 5940
   tataaggata cctttttatt tttgaaatta gtggggtatt ttgattatct gtatattcat 6000
   gccaaccagt gaagtaaaat ttatcactta attatgattg caaatccaaa gtgattgtca 6060
   tctagtaaaa tcccaatcgt cttttcaaca taatctttta atatgtgctt gggtgaatca 6120
   ttttcatgaa aaaattgatg gggtattcat taagcgtatg ataataccga atctttgcca 6180
   cattggaatg gaaataataa attttgcaac tcaaaatcat cacacagtta tggaacgcaa 6240
   aaaggaagag cacaacaagg tttcccataa caaacacaag atgtcattga tactatatag 6300
   gaaaaatata ttaaaaagca ttattagtag catccaaaat gcatgagatc gtctaaataa 6360
   accaacaaaa atccacgatt ttttgtgtct aatgtagagg taaatatttt aatatatatg 6420
   actactaatg aaaatttcat ttcaattaca ttgccatgcg tcaaccttct agaagcattt 6480
   tgtttaaagc ttaaaattta ttatacgctg ttgcaatgaa tcacgttata gtgccacgat 6540
   aaatacatta tatatactca tcgggctttt tgcatacaaa tgacgtatgt aatgaaataa 6600
   agctatcatt caaggtgaat ttatggctca catgaaatgg tttattaaaa actagcatat 6660
   atattactaa ctcagagcac atgttggtag tccaacttca atgtaatgac gcccaagcat 6720
   atccattatg actactacat aaatacaacc aaatgaaccc acaaatgtaa tagcaatgtt 6780
   tgaaaacatg gtatgtcata tatatatata tatataattg ttgtgatata gcccaagcaa 6840
   accaggtgga atcataatga aataacaaac ctgtgcctta atccatgcag atgcaatttt 6900
   tgcgccctaa aaagattacg catatatata tatataatgc tagataatgt tatagaatta 6960
   aatgagacta tcatagcctc atctaagaga tgcagaaata tgctatatac acaaactatg 7020
   gctacgttcc ccttttatac gtttatcaca gcagttaaaa ccctgtttca tcgttatata 7080
   ataactggtc actatatttg taagtttgta acaaattctg ttatcttatg atgacttggg 7140
   gcacaaatag tatcatatta aagttgcaca ggatgttgta ccgagggatt aagtgcttgc 7200
   ttaggaagaa agatataatg tatgatgcat tttcatggct cacaatttgg acacgacatt 7260
   ttcataatgt aagatgcctt ataatttgac tccattagac aaagtgatcc tcttactata 7320
   tttatcttag agattttata agattggagc caaaaacagt actttgcata aaactgcgtt 7380
   tggttggtaa atatgagatc atatatccta ctaaatttaa ttatgattaa tctgcggaaa 7440
   tgcaaaaata tgggaaatag gtttaaacaa agtaatctat taagaaacac tggaaaacgt 7500
   ggacaccaac aattctataa ggtcctatga cagagctgat acgataattt cgttaaacta 7560
   tcttaaaatg ctgacaaaat gcaatccact tttttaccca tctcttgaac ctgtgaccca 7620
   ataatttctt tgttcgatta attaaaatga taactctagt ttgatgaaca aataaaatat 7680
   cggtgagcca atgaatatta tctcatattg aatcacagca aatttattta gagtagtctg 7740
   cgcaatcacc tacaaagcta cgttattctt cccaattaat ttacaaacat gtggctcaaa 7800
   caaagatgta tagacgtctc agagatgatt tttcgagtgt agtctattaa ggtagccaat 7860
   atcattgcta caatggccta catatggata ccccaaactg tgttcaatta tcatctcacc 7920
   acacggggaa actgctcttt tccaaactga aatttgtttc accaatatac atacatatat 7980
   atatatatat agaaagacag gaaaggttca ttactagaag cacgcaaccg ttcacagagt 8040
   ccctgtacgc ctagattaaa tttccggcat cttacataaa ataggtatgg ctatataatg 8100
   ctgttaaaat tttcactgtg actcgaacaa gttccacact acattcacct ccaaacattt 8160
   gtcatacaag aacgatacca aacctgttga catcgcacca acaaaaataa taatcacatg 8220
   ataactaatc acttggaccc tacacccaag gattattcta ttaattcacg caagggatag 8280
   acgcaacgtt acttaatcac atattcctag acatttgagc ataatcatta tggaaggaca 8340
   cgaattagaa ctgtgagatg catatcatta tctgttccga ctttcaaatg cgaactttaa 8400
   tgagccaaca gcgacaaaaa ctcaaaaaaa tgaaagattt tcagtaaaat gacatcgatg 8460
   gaatacatca gcaacactta aaataataga tatatttagt tagtaaaacg aaggaacgca 8520
   ttacctcacg gctcaaaagc ctattccata aggggggaat ttaaagtaaa tagattaaaa 8580
   caaagggatt agccagcaat agaatttata ccacgggaac aactaatatt ataaaatatg 8640
   attaaatgtt taagccatta gcaacggttc aacgtattca cctcaaaaag catgccgtta 8700
   aatgaatcaa caaacaaata gaatcacaaa cacagatttg cacaactata aaatatatat 8760
   gtataataat tactgttttt aaaattaaat atatcgtacg gaacttacca cttttgacaa 8820
   aagtacctta cctccaacac aatggttcag ataaaaccaa gatttttcga acaaaaaaga 8880
   tatggaaagt agtaataaaa acttataaac aaaccttatt gtatcaaacg ttaagatcaa 8940
   tggataataa acggcaatcg aagatgtatt atatctaccc tacggaagat tgtacgacgg 9000
   aatcggcgcg ctaaagcatt tccacctttt attttatcat ataaaaccaa tctagtgata 9060
   acccgcacgt ctgtaatgta gaatacgggg atcatgccaa catgtcgatc atgtatgaag 9120
   agctgcaaaa ccgtgcacct ttggtgccgc gacatcagtg cactgtgatg tcgcgtgtag 9180
   attccacaat taaatcttta aggacatcgt ggatgttcat aaatacacgt taggaggatt 9240
   caggtggaca cgattccact ctgcaagtat cgcgcacaag ggcccttaat cagacctcgg 9300
   tgtgatattg gcctttgagc tttggtactg ccgcatttac gatacaaatt tagtactatt 9360
   gtcgccaaaa ttttaaaaac agggtcgata tcgtcgccct ttaatggtta tgccattttg 9420
   gttgctaagt tacagcacct ccaacgcgtc ttcttaagtg acgagactct tcaacgctcg 9480
   tcacacatat agtgttttat atttacttat ataaaaaata taagtaattt tctacaattt 9540
   ggtggagccg actgcctaga aggttgttca cggttggaca tcttcaatat ggcgcgcgaa 9600
   agacgtcata tcagtttaac aaaaatgttg aacttattat cagtagcatt aggttgctat 9660
   tttgcaggta tgtttataat tacactggtg tacttcaata gggcgtggga gtgtgtgatg 9720
   ttagtatcaa ttctacaaat atatacttta aatgggtagt tgcgtatatc aaccttttac 9780
   tgtgtgttat atatatatat atatggtatc atgctgacat gatatacagg gaagcatgtt 9840
   tatgcttttc gcactgaatc atccgtggct cgtcaacacg ctgctgacaa caatgtagaa 9900
   ttgggttcta tacaggcatc tcttagacct gactctagtt tagagtctac tcacacgtca 9960
   gccgatatcg ctcctcgtgg caatggtggc cgtagaagcg g 10001
<210> 17
   <211> 874
   <212> PRT
   <213> Babesia bovis T2Bo
<400> 17
   Met Ser Ser Leu Ser Ile Leu Ala Val Ile Ser Ala Val Met Leu Arg
   1 5 10 15
   Val Ile Ala Asp Asp Asn Val Gly Thr Pro Val Pro Glu Asn His Ile
   20 25 30
   His Ser His Leu Asp Pro Thr Leu Phe Gly Asp His Asp Asp Gly Thr
   35 40 45
   His Gly Thr Gly Thr Val Arg Asn Glu Pro Val Pro Ala Thr Pro Pro
       50                  55                  60
   Gly Thr Pro Leu Arg Asn Asp Gln Pro Pro Pro Ser Gly Gly Gly Lys
   65                  70                  75                  80
   Lys Val Ser Val Thr Glu Ile Ser Phe Asp Ser Leu Ile Asp Asp Leu
                   85                  90                  95
   Val Trp Cys Gly Asn Asp His Lys Thr Val Leu Leu Lys Thr Gln Ser
               100                 105                 110
   Gly Arg Leu Tyr Arg Ser Ala Asp Gly Gly Lys Gln Trp Ser Glu Ile
           115                 120                 125
   Thr His Leu Phe Gln Gly Thr Ala Arg Asn Gly Asn Tyr Arg Val Asp
       130                 135                 140
   Ser Ile Val Leu Cys Glu Thr Asp Arg Asn Val Val Val Ile Val Gly
   145                 150                 155                 160
   Asp Ser Arg Asn His Phe Ile Ser Gly Asn Ala Gly Gln Ser Phe Gln
                   165                 170                 175
   Pro Ile Ala Tyr Glu Gly Pro Val Asn Met Trp Val Phe His Pro Ser
               180                 185                 190
   Lys Pro Ser Trp Ala Leu Leu Ser Ser Trp Glu Gly Ala Cys Phe Ala
           195                 200                 205
   Ser Asp Asn Asp Glu Asp Cys Val His Ser Val Phe Val Thr Arg Asp
       210                 215                 220
   Met Gly Arg Ser Phe Gln Arg Val Ser Lys Tyr Val Ala Gln Phe Ser
   225                 230                 235                 240
   Trp Gly Asp Val Ala Ser Arg Ser Glu Asp Arg Ile Tyr Tyr Ser Arg
                   245                 250                 255
   Tyr Ala Leu Glu Ser Gly Asp Gln Pro Lys Gln Asp Gly Trp Asn Asn
               260                 265                 270
   Asn Ile Ser Phe Met Tyr Thr Asp Asp Phe Gly Asn Glu Asn Val Leu
           275                 280                 285
   Ile Met Glu Gly Gly Asn Lys Phe Leu Val Ser Gly Ile Tyr Val Phe
       290                 295                 300
   Val Ala Arg Val Ser Asp Pro Ile Arg Gln Thr Val Asn Leu Tyr Val
   305                 310                 315                 320
   Ser Thr Asp Asn Ala Glu Thr Phe Asp Arg Ala Gln Leu Pro Val Glu
                   325                 330                 335
   Leu Glu Glu Arg Ser Tyr Thr Ile Leu Asp Thr Ser Gln Gly Ala Val
               340                 345                 350
   Ile Ile His Val Gly His Glu Tyr Glu Gly Gly Asp Val Glu Val Gly
           355                 360                 365
   Asn Val Tyr Ile Ser Asp Ala Ser Gly Leu Arg Tyr Ser Leu Ser Leu
       370                 375                 380
   Pro Asn Asn Ile Arg Ala Ala Ser Gly Glu Cys Glu Phe Asp Lys Val
   385                 390                 395                 400
   Tyr Ser Leu Glu Gly Val Tyr Leu Ala Asn Phe Arg Asp Asp Ser Gly
                   405                 410                 415
   Gly Val Leu Asn Pro Arg Asn Lys Phe Lys Thr His Ile Asp Gly Thr
               420                 425                 430
   Lys Thr Gln Leu Asp Gln Lys Arg Gly Arg His Val Ala His Thr Lys
           435                 440                 445
   Ile Glu Pro Asp Ile Arg Thr Val Val Ser Phe Asn Lys Gly Ala Glu
       450                 455                 460
   Trp His Tyr Leu Gln Pro Pro Lys Leu Asp Ser Glu Gly Lys Pro Tyr
   465                 470                 475                 480
   Asp Cys Glu Glu Gly Lys Cys Phe Leu His Leu His Gly Ile Thr Gln
                   485                 490                 495
   Tyr Lys Asn Phe Ala Pro Phe Tyr Ser Val Glu His Ala Thr Gly Leu
               500                 505                 510
   Val Leu Ala Thr Gly Asn Val Gly Ala Arg Leu Arg Phe Asp Pro Ser
           515                 520                 525
   Gln Val Asn Thr Phe Leu Ser Arg Asp Gly Gly Leu Thr Trp Val Glu
       530                 535                 540
   Ala His Lys Gly Ala Phe Ile Tyr Glu Phe Gly Asp Tyr Gly Gly Leu
   545                 550                 555                 560
   Ile Val Met Ala Glu Asp Gln Arg Lys Thr Lys Glu Val Val Phe Ser
   565 570 575
   Trp Asn Glu Gly Ala Thr Trp Phe Asp Phe Asn Leu Ser Lys His Glu
   580 585 590
   Leu Asn Val Asn Asn Val Val Ile Glu Pro Asn Cys Ser Ser Leu Asn
   595 600 605
   Phe Ile Leu Tyr Gly Asn Arg Asn Gly Ile Gly Val Ala Phe His Leu
   610 615 620
   Asp Phe Ser Ala Leu Gly Gln Pro Leu Cys Lys Gly Ile Trp Ser Ile
   625 630 635 640
   Asp Ser Thr Ser Ser Asp Tyr Glu Thr Trp Arg Pro Thr Asp Pro His
   645 650 655
   Gly Asn Gln Cys Leu Leu Gly Arg Lys Met Ala Tyr Lys Arg Arg Lys
   660 665 670
   Gln Ala Ser Glu Cys Phe Asn Gly Lys Glu Phe Lys Ala Thr Val Glu
   675 680 685
   Arg Glu Val Cys Pro Cys Thr Pro Asp Asp Tyr Glu Cys Glu Ile Gly
   690 695 700
   Phe Thr Arg Ser Ile Gly Ser Asn Thr Cys Arg Val Asp Gly Thr Trp
   705 710 715 720
   Leu Met Arg Glu Gly Cys Thr Ser Ser Ser Phe Phe Trp Thr Asp Ala
   725 730 735
   Tyr Arg Lys Ile Pro Gly Asp Val Cys Thr Ser Gly Trp Ile Pro His
   740 745 750
   Gln Val Ala Val Pro Cys Pro Pro His Ser Pro Leu Ser Asn Gly Ser
   755 760 765
   Lys Met Val Leu Thr Thr Ile Leu Val Leu Ser Phe Ile Met Leu Ala
   770 775 780
   Ile Val Tyr Ile Ser His Asn Glu Asn Leu Lys His Leu Phe His Asn
   785 790 795 800
   Tyr Gly Phe Lys Gln Phe Ser Tyr Val Ala Tyr Thr Pro Val Asn Ser
   805 810 815
   Lys Lys Pro Met Tyr Arg Gly Ala Gly Gly Arg Phe Glu Pro Glu Leu
   820 825 830
   Gly Phe Ile Asp Ala Glu Gln Glu His Asp Glu Pro Thr Leu Leu Asn
   835 840 845
   Tyr Leu Ser Gly Asn Arg Met Gly Gly Asn Ser His Gln Gln Pro Thr
   850 855 860
   Lys Gln Asp Gln Gln Gln Ile Glu Leu Leu
   865 870
<210> 18
   <211> 10001
   <212> DNA
   <213> Neospora caninum
<400> 18
   aacaagcgcc ggaaccacgc ttccaacact gtaagttctt tcatgcctcc tcttcatcct      60
   tttccctcgc ctcttttctg tgtccttttc cctcgcctct tttctgtgtc cttttctctc     120
   atttcatcgt ccctttctct ttcctttttc tccactctcc tgtcgttcct tttgatctcg      180
   cgtttctgtt actctcttct ttgtggccgc gggtccagtg cttcgttttt tcactgcgct     240
   gtaggggttt gggtgtctcc tttttcgtct tgcgtgtcgt cgcgtgtttt tggtttctct     300
   ttgagtgagt gctctatgca tgcttctcgc cgcctggctt tcgcttgctc catcagcgat     360
   ggttttcata gggtccaaaa tgctcatcgt cggcgggcgg actgataacg actgcacaaa     420
   gtaagaaccg acttagagct ccatttctcc gatactcggc tcctgtccag tgtacgaatc     480
   gacttctcgt tctcgcgcca aatatatttt tctatctccg tccctttctc cctctgctta     540
   tctgtctcct tctctttctc gttatccttc tctctcggat tctctttttt gttctctcct     600
   tcctctcctc tctctcctca ctctctctcc tctcctcgtt ctccttttct ttccctctcc     660
   ctctccttct ccttccctct ttctcgctcg gtttctctct ccgttgtcct gcgttctgtt     720
   tttctcggtt tttcgcattg cgccgtttcg tcacgcttcc cttgcgcttt gcaggccgtt     780
   gtccacagct gtctacgaca cagagacagt ggaatggagg ttcattgcct cagttggacg     840
   ggtgagaacg cgcgtgtgcg ccgacaaaaa gtgtttgcgg agtgttcctc cacttttgtc     900
   tccccagccc gtgtctctgt ttctcctccg ctctcgccgt ctcgcgtctt cttgcgccgt     960
   cacgggttct ctcgctcttc tccgtgtcac gcgaccgccg tcgccacctc cctttcccgc    1020
   ggggcgtgtc tctcccgctc ctcgcccgtc cgtcgctcgt cccgttttcg tctctcccgt    1080
   ttcttcacct tcgtgtctgc gtcctccctt cctctctccg cagtttcgcc acagctcctg    1140
   gacgctccga tcgaccgtct acacgttcgg cggcttctcg cacgtcacgc agcaacaccc 1200
   gacggccgac ttaactctgc tcgactgttc ctgtgagagc gcggggagaa cgggaaaacg 1260
   gaaaacgcga aaggacgcga aacaaacaag acatgagaga ggaaagacgc gactcttcac 1320
   ctgcgagaag aagccgagag aacggggcct gtcgactcct cacaccaccg ggcagttcat 1380
   ccctcccttt tgtgtcgtgt cctctcgcag tgattccaaa cttttacaac gagcgagaac 1440
   gcgaagacat tcgccgcaag caggccttgc aacaggaaca gcagctggcc gtggcgagcg 1500
   cagtcgcagc ggctcagacg cagcccactg cgcagcctgc gagtgcgcct cctcctcccg 1560
   ctcctctacc tgcggccgcg tcgccgccag ccgtgcgtcc ggggctttct ggccaacctg 1620
   cagccgtcga aaaggcgcga gcggcgcaga cgccaaacgc cgcgcagccg caggcgcccg 1680
   tccagcaaac gtccttcgcc gcgtccggct ctttgcggca gtcctgggtt ttctcgcaac 1740
   ggagtcagac acggcagctg gcggacgtag gcagagagtg gaaaaggaaa ccggaaagcg 1800
   gaacggaaaa gaatgcgagg aaagggcgta taggaagaga cggaacaaaa aaggcagaga 1860
   gaaagagtaa aggagatgtg acagtacgga acggcgatct ccgagtgaac aggggaagaa 1920
   aacggcaggg aagaaacggc cagaggtttt gaatgcagtg tcgaggcgac tggatgagga 1980
   cgtgcccagc ctgtgtcttg gtttctgttt ttcgtcgctt cctggattcg gaagaaggct 2040
   cagaccccca aagtcgcgga gggaaaccga agacggattg cagacgagct ttcgcgaggc 2100
   ttggtgacgc tctgtcaaga ctgggggaat tacgctttgt ttgcagatca agctcgcctc 2160
   tcacgtctcg gtggtgcacg agacaggcga agacttctcc tctctcgtcc gtcgggtacg 2220
   tccagaggga gaaggaaggc tgggaaacct ttttcgacgt tcaaggaaag accggttggt 2280
   ccgacctcgc tggcatcgtt gtgctctcac ttgcgttggg ccacatcccc ggaaggctct 2340
   tccccatgtg gtgtcctctt tcgttgcgcg tttttcttgc ttttccggag acgctttcga 2400
   gattctccga tgtccacgcc tgcgcgagag tgaaacgaag aacaggacac gacggtgacg 2460
   tgacggttgc tctttttttc gcgttttgag agagagatcc tcgtttttgt ctcgcgacgc 2520
   agatttcgat cgaccgcctg gaggaagagg gaaagaagat caacaaagaa gcctcgctgc 2580
   aggagcctcc gcccaccccc actgagtcgc ggcgcgaaat gctcgcggac aaaatcctga 2640
   aactcctcct gaggccgctc atcactcagc aagaggtcta cgccaccttc aacacagacg 2700
   cccccttcgc aatctcctgg aacgatgtaa catatctctg tgatgtggta agggatattc 2760
   cgtatctata tatatctata aacatatata tacatgaata gatagataga tataaatata 2820
   gaagcatcgt gctgacgcgt gtctctatat atgtaaatgg aggacagcta tatgtacata 2880
   tatacctaca tatatatata tatatatata tatatagata tagatatgca tgcatgcgtg 2940
   catgtgtgca tgcggtttta ttgatggaag agaatttgca ttttcttcgt ttttcaggtg 3000
   ctggacattg tgaagaacga cgacatggtg ctccatcttc gcgctccgat taaaggcaag 3060
   ctgactggga agaggcgttc gtttgccttt ttcgtgtttc ttgcgagaga tgcacagccc 3120
   actccctgag ttttttcgca cgcctctccc ttcttggccg catgcgaggc tcgcacacac 3180
   aaagaggtgg cggtgtggag gttcacacaa aaagcctgcg tacagtttgc ttccgcgttt 3240
   ttcgcggttt tttccctttt cagtctacgg ggatatccac ggccagtact atgacctcat 3300
   gcggctgttc cacttgtaca agatgcctgt ggaagaagaa cgaagcgacg aattcatcgg 3360
   agggtctgcc gccttggggc aaaacggacc tggctccgca aatgccgggg ttgtcgggga 3420
   catcgacaca aacgattatt tgtttctcgg agactacgtc gatcgaggtc tcaacagctt 3480
   ggaaaccatc tgtctcctct ttgctcttaa agtaagacca gtttctccat atctctacct 3540
   gcatcaatct ggatatctct gtacgtacat ctcgaaatat accttggaat ccatctacac 3600
   atctccatat atatatatat atatatatat atatgtagat atatacatat atagagatgt 3660
   atgtgtgtgc gggtgtatgt acacctacgc gtgtggcagc gtgtctctca gggtttgact 3720
   gcctggagat cactcacgta ttcatatctg tctttgcgta tatgcggctg tacgtgtgtg 3780
   tggctgtaca tgtgtgtgtg tgtgcggttt gtgtgtcttt gatcgttgct tgcttccagg 3840
   ccaagtatcc ccggcagatt cacctgcttc gaggcaacca cgaggatcca gcaatcaatt 3900
   cgctctacgg ctttcaagac gagtgcaagc gccgactgcg tgaagacccg ttcgatccga 3960
   gcagttgctg gcgcaaattc aacctcgttt ttgagtacct cccggtcgct gccatcatcg 4020
   acgattccat cttgtgcatc catggcggga ttggcggaac gatctcctca gtcgaggatt 4080
   tagccgcgct tcaacgccct ctcaaagtgg ctcaagtccc ccaaaatgtc tacgtaagcg 4140
   tgtctccaca tgcaacacca agaatattcg tgtgtgtagg tgtgggtata tttctgtgca 4200
   tgtgcacatg tctatatttg catgcatgtg cacatgtcta tatttgcatg catgtgtaca 4260
   tgtctatatt tgcgtgcatg tgcatgtgtg cgcttgtaga tgtgaacgtt tttcaaaatt 4320
   cggcagtctg ggtctgggtt taccgacttc tcagcagcgt tgtttgtggg tagcctgtat 4380
   atgcctggac aaaggcgttt tggcgaatgc agcgactgcg ccttcgtcag tggaaactgc 4440
   acgtctcgtg ggactgaatg agcgcacgtc aaggcttctg cgggcgaacc cctgcgcgga 4500
   gtgtgcatct gctcgaaaga aggccagaag gagagccgtg cgtggaaacg cggagactcc 4560
   gcaacgcgag aaaagaggtg tctgaagtct ccgtctgttc ggatcttttc cgatcttttc 4620
   cggtttgccg cactgtctgc ttgccgcatg caggagcaac gcgtcacgga tttgctgtgg 4680
   tcagatccca cagacaacga cagcatgttg ggggtggtgc gaaacgatgt gcgggatcca 4740
   gacggctcag ggagaatcta caaattcggt cccgaccgcg ttcacaggtt cctcgcagaa 4800
   aacgatctgc aactcatcat caggcaagca gcgacaagga acgacgcagt cactcacggg 4860
   ggggacgacc aagtcgaggg agcgtctctc tagctttttt tctcgtggac gtgaacgcga 4920
   tcgtacagac gatgcaaacg gcaaaagggg gaagagaacg gttcgcgtgc atgcaaataa 4980
   ttgaagctgt acatgcttgt actagatctg cagtgagaga gcacttcaga gatccacaaa 5040
   tctggagtcg cgagtgtatt catttagtag aaagacatat gtatatccac gtatgtttat 5100
   gtaatatgca cgttttttgg atggccaaat cgcgtctata tgcgcagcac atacattcat 5160
   acatcaatat aatatatata tatatataaa tacctaggta tataggttaa aggcattttc 5220
   cctttctgct gcaagtgtct gtatagacgt gtgtgtcttc tgtgaaggtt ttttgtctgt 5280
   ggagaatccg ttccttgcgg gcctttggct atggtttccc cttgagatat atgtgtggag 5340
   ttttttcaga gcacacgagt gtgtgatgga cggtttcgag cgatttgcag gaggcaagct 5400
   gatcactctc ttctcaggtg agactgtcgc cacgccgccg ctgtccgaga gagtcaggtg 5460
   ttcctcttac caatgttctt ccgtctctta ccgtgtgtgc ttatgtgaga aaatgccgta 5520
   tctttttcga tgtaaacaaa tatttgtaag gtaactatgt atatctgtat aggtgtattc 5580
   atgtaaatcc tatagctatt tgtgtatctg tatgcagatc cgtcaagaaa tgtgtaggta 5640
   tacgtagaca tctacgtgta gacgtgtgcc tgtcgctcta gtgacacgtg tcgtcgccag 5700
   cagatatagc tgatggtgca agtgaagtgg aggcggatac ccttcgcctt tcgtttctca 5760
   tcttgtctga ttcgtttttc tcttcagcaa cgaactactg caatcatcac cagaacgccg 5820
   gtgcgcttct ctttattaga cgcgacatga ccgtcatccc gaagttgatt tatcccgcaa 5880
   atgctccttg ccagtacaac tcctggtgcg tttctccgac aaaaactgcc ctacagatag 5940
   atccatctat acacacattt ctacgcatgc gcaccgagac agatgcacgc atgcagagaa 6000
   atagacacag agaggccatg ctttctctct ctctcgcccc gtctccatgt atatatacgt 6060
   gtgtcagcct atggacatat atatatatat atatatagag agagagagag agagagagac 6120
   agatgtagat agatggatag atggatagag agagagatct ggttgtgtgt atcttgaatg 6180
   tcgagtgccg agatagaact gcgcattgga gttggagaga ggctgtctgc tgcatttttt 6240
   cctgttgcag ggattcgaga atggctgagc ttcgtccgcc gacgccgcct cgagctgcgc 6300
   cgcgcatgcg cgagaaagag tttggagctg gcgggtgatg ctttttgcgt cgagagtttc 6360
   tccgtttctc tgttttttcc cggtcgcccc gcgagtttct gctcagagaa aagagactgc 6420
   cacaggaaaa gcggaacgaa ctgcttgatc gcaagcatct tgaggagacg cttgccttta 6480
   atgttcctta cttctttgtc gattcttccg cgggatagag atatatatat gtatatatac 6540
   atttatggta tatgatatat atatttctgt gtgtgtgata gtagagaggt caggacgaag 6600
   gggagagagg atcggtttgg cgaggggaaa gaagactgtg tgattttgaa gagagtgctc 6660
   ttttgaatgg agtcgaacgc gaaacacttg cgaggcggaa ctggttcggg tctctccata 6720
   cgcgcgccgt tttgtgtttc aagaaacgcg aaactgagtg ccgaacacgc aggcttccaa 6780
   agcgaagaac aaggagacgg ggtgggttct gccgcgcatg cagtggggtt ttctggtgta 6840
   cggctctgcc ttcagcgcct ctcgctcgtc gagcctccga actctggaaa aacttttgtg 6900
   cattggggcg gaaacgctcg aaaaggatct catcttggca aagacgtgaa gcaaacgacg 6960
   cgcatgcatc tcggcaaatc gcttctcggc gactctgccg ctctcgacgg ccgacactct 7020
   tcacaattct ttttctctcc agaactctct actgcacgga gcatcaagca gcgtcccgcc 7080
   aaaacaccac gcaacataca cagccgcaca gtgatatata tatatatata tatattcacg 7140
   tgtttcgtat tcataggcgc gtttgtatgc atgtgtacgt atccaaaaag ttatatatat 7200
   gcctgtatct atgcacgata cgtatgtaca gaatacacgc tggtgcaact aaacaatgaa 7260
   ccacggcaca cacacacaca cacacactat atatatatat atatactata tatatatatt 7320
   gatgagggtt tagtgggagg gcgccgttgt gggatgtcag cgattcgaaa aggccaaacg 7380
   cgatcgactt ctcgggactt cccctggagt gcatgcgcgt ttgtgcctga cgtggaaggt 7440
   ttgaacagga atcgtttgcc tctcacgcgg gcgtttctcg ggcagaaggc tgtgttttcc 7500
   tctccttcgg tgactggctg tgtaccgtag accgcaggct gtctgttctt cacacatttc 7560
   actcggatcc tcggtttttt cgtcctctgt acgacgcaag tgtgtgtgtc tccgtaggcg 7620
   ctacgtccgc tcgctttatt cgagggaaga accggagacc ggcaggagac tgagactcgt 7680
   cacgacacct ggcgagagct acgtttgcgc gattgagggg ttgggcacac caaacacgag 7740
   gaggggtgta ggatcctcac gctctgctaa gcgttgtgca acaactgacg cacacccacg 7800
   gcagcgttgc actgtttctg gaacgcaccc ccgggaagaa acagaggaaa gtatctttgc 7860
   gtctaaacac cgcagcgcgc aggttcttaa cctgttccca tggggcgcaa acttagggaa 7920
   acacgagctt ctgtggagac acagacgctt cgctctcttc ttctgcttcg cagtttctga 7980
   cgcaacgatc tgccttctta gacggggtta acatgcgatt cctgtgcacg ccttggcctg 8040
   catgggggac gcagagaacc cagcagccaa aaggtgaatc ctgcatttct gaacgttgac 8100
   ttgagcaggg gtctctccac gctttttcgc cacagcttcc tcgtgagccg tcgtgttgga 8160
   ggaaacggcc attccagtag cgacgagcag gagctctcag acactctgtg aagcgccagc 8220
   gccttccaat gctagaaaca ggaaagacgc ttccactgga gcaagagccc cgcctgtcgc 8280
   cgttctatgc cccggcaccg tcccgcttcc tctcgccgat ccccctgcta aaatcagcga 8340
   tccgctggag aactagacaa gcgagacgag agcgtcgggg acacgtgcac acaccctgcg 8400
   tgggtttctt gcgctttggg gagtctcact cgactctttc cgcgcttctc tcgtgcctgc 8460
   atttctgcaa acgacgcctc ctgtaccacg tcgcgtgcct acgaaacact tccgctttgt 8520
   gcggctgggc cgcgcacacc tcccggcgcc ctcctttcct tcctttgtcg agtttctcca 8580
   gcggattcga cggccggaaa ctggacaaca tcgagcgctc tccacctgtg gccagtgtac 8640
   gtacactgca agagagcacc cttttgtgtt ttccgttttc ggtcggctgt cgcctgcgcc 8700
   tcgcacaccc cccgttctcg gacaaggaag gcagaagccg agagaccagc acgcgcgccg 8760
   gcttcccaga atcgctccct ttctcgtttt cgcttttcct cggccagcct tggccgtttt 8820
   cggtctttcg tgctcttgct gaggagatct tgtgcgccgg ttcttctggg gcctcgcgac 8880
   acacagtggt cttgtgcaag tcgctcgctt ttcttttctc agcgccgccg tgtcgcgcgc 8940
   ctcttcccgg ggggaggggg gggggagacg cgcgggctgt ctttgctttt ttctgcgctt 9000
   ttcttccttc tttttgtcgg ccgagagttc ccatcctgtc cttctcgggg tcgtgacgcc 9060
   aaggctctac ttctttcctg cgctcacacg cggtgcgtcg cacacttccc gggtttcgtc 9120
   ctcggaaaaa gcttgtcttt ctgaagcgag gcacgcgtac aacactgtct cgagaagtca 9180
   gtgctcggca aacgtgaagt ttccaacttt cccgccctcg cgcaaagaac ttggttccgt 9240
   tcccaacacc catcgacgct ccctctcatg tctttgcacg aggcctgctg cctctcgcgc 9300
   cgatgcaagc gccggaaaac ggcgagattt ctgcccttgg cttttcacac aattccactt 9360
   tcttgctttc gctttctctg tgtctgaacg ctttctcttt tgtcgcctga ccctcggcaa 9420
   ccgcgcaccg cccagatgtg acctgtaaca ccgcaacgtc gaagaaatcc aagtgtatct 9480
   gtgcatgcta gtctgtctct ctcttgtttc tgcgtttcgt ctaggtttcc tttctctctc 9540
   gtgccgcgtg tctctcgcct cgcgctcggc tgattcgcag ccgcacactg tcttcctcgc 9600
   ccgttctgtc cggaagcaca ctctcgactg acgcttcgct gtctccgcgc tggctccatt 9660
   gaagttgaaa gaggagacgc gcccgaggaa cgagagagaa aaggagcttt gctcaccgaa 9720
   agacggaaaa acgcaccggc ctcgactccc gcccgttttc gggtgtgtgt acaccgcgtg 9780
   cacaccgcat ggcggcctcc attgtcgttg acttctgtct cgttcttcag tgagcgcggt 9840
   ctccgatcac ctttgtcttg tccagcagtc gcttttcgtc tggtgatacg cctctcgcct 9900
   gttccagaaa gaggatcagg aaggacgcgt tctctggtgt acccctcgca ttcgtattct 9960
   ttcaagccct gctgggtggg cgttcccttt tttctccccc a 10001
<210> 19
   <211> 10001
   <212> DNA
   <213> Neospora caninum
<400> 19
<210> 20
   <211> 951
   <212> PRT
   <213> Neospora caninum
<400> 20
   Met Ala Leu Ser Phe Ala Arg Arg Pro Ala Ala Gly Val Lys Leu Ala
   1               5                   10                  15
   Val Leu Val Ala Phe Leu Ala Val Phe Cys Pro Val Leu Pro Asn Leu
               20                  25                  30
   Phe Ser Lys Glu Asp Ser Leu Gly Arg Pro Val Pro Arg Gly Val Leu
           35                  40                  45
   Val Thr Asp Ala Ala Ala Tyr Ala Asn Lys Lys Val Ser Val Ser Glu
       50                  55                  60
   Val Ser Phe Asp Ser His Ile Glu Asp Ile Gln Trp Cys Gly Val Asp
   65                  70                  75                  80
   His Arg Thr Ile Leu Leu Lys Thr Arg Arg Gly Arg Leu Tyr Arg Ser
                   85                  90                  95
   Gln Asp Gly Gly Lys Ser Trp Thr Glu Ile Thr Asp Leu Leu Lys Pro
               100                 105                 110
   Ser Glu Gly Ser Ser Ala Ala Val Ala Val Asp Ser Ile Ile Val Ser
           115                 120                 125
   Pro Val Asp Lys Arg Val Val Leu Ile Val Gly Ser Lys Arg Asn His
       130                 135                 140
   Phe Ile Ser Glu Asp Ser Ala Ala Thr Phe Arg Arg Leu Lys Tyr Lys
   145                 150                 155                 160
   Asn Thr Ile His Asn Phe His Phe His Pro Thr Arg Pro Lys Phe Ala
                   165                 170                 175
   Ile Leu Ser Thr Trp Thr Asp Ala Cys Tyr Ala Ser Gly Gly Ser Gly
               180                 185                 190
   Thr Ser Arg Thr Ile Gln Gln Asp Cys Asn His Gln Leu Phe Tyr Thr
           195                 200                 205
   Arg Asp Leu Gly Arg Ser Phe Lys Leu Val Ala Asp Tyr Val Val Gln
       210                 215                 220
   Phe Ser Trp Gly Asp Lys Lys Leu Gly Asn Thr Asp His Ile Phe Phe
   225                 230                 235                 240
   Thr Gln His Arg Gly Arg Ser Gly Asp Gln Pro Arg Tyr Gly Gly Trp
                   245                 250                 255
   Ser Lys Asn Val Asp Leu Met Tyr Thr Pro Asp Phe Gly Ala Thr Ile
               260                 265                 270
   Thr Arg Leu Val Tyr Arg Gly Asn Lys Phe Leu Leu Ser Asn Gly Tyr
           275                 280                 285
   Phe Phe Val Ala Lys Val Lys Asp Ala Gly Lys Gln Thr Val Ser Leu
       290                 295                 300
   Leu Val Ser Thr Asp Gly Gly Lys Ser Phe Gln Met Ala Lys Leu Pro
   305                 310                 315                 320
   Val Glu Ile Glu Glu Arg Ser Tyr Thr Val Leu Asp Thr Ser Glu Asp
                   325                 330                 335
   Ala Ile Met Leu His Val Asn His Gly His Gly Ser Lys Gly Asp Thr
               340                 345                 350
   Gly Asn Val Tyr Ile Ser Asp Ala Lys Gly Val Arg Tyr Ser Leu Ser
           355                 360                 365
   Leu Pro Asn Asn Ile Arg Thr Ser Thr Gly Glu Cys Glu Phe Asp Lys
       370                 375                 380
   Val Leu Ser Leu Glu Gly Val Tyr Leu Ala Asn Phe Lys Asp Thr Ile
   385                 390                 395                 400
   Asp Ala Ser Ala Thr Gly Ser Glu Gly Ala Ala Ser Glu Lys Asn Gln
                   405                 410                 415
   Val Asp Ala Ala Gly Glu Lys Leu Thr Glu Glu Glu Ile Glu Glu Glu
               420                 425                 430
   Ala Glu Gly Val Gln Val Asp Leu Glu Lys Lys His Arg Ser Val Ala
           435                 440                 445
   Thr Arg Ser Arg Gln Glu Glu Val Ile Arg Thr Val Ile Ser Phe Asp
   450 455 460
   Lys Gly Gly Val Trp Ser Tyr Leu Lys Ala Pro Arg Val Asp Ser Arg
   465 470 475 480
   Gly Gln Lys Ile Asp Cys Pro Pro Asp Arg Cys Trp Leu His Leu Asn
   485 490 495
   Gly Ile Thr Arg Phe Ser Asp Phe Ala Pro Phe Tyr Ser Val Glu Asn
   500 505 510
   Ala Val Gly Ile Ile Met Gly Thr Gly Asn Val Gly Ser Tyr Leu Arg
   515 520 525
   Pro Glu Lys Asp Glu Ala Asn Thr Tyr Leu Ser Arg Asp Gly Gly Val
   530 535 540
   Thr Trp Ile Glu Ala His Lys Gly Ala Phe Ile Tyr Glu Met Gly Asp
   545 550 555 560
   His Gly Gly Leu Leu Val Met Ala Asp Asp Thr Lys Lys Thr Asn Gln
   565 570 575
   Val Val Phe Ser Trp Asn Glu Gly Gln Ser Trp Tyr Asp Phe Glu Leu
   580 585 590
   Gly Ala Ser Pro Leu Phe Val Asp Asn Ile Val Ile Glu Pro Asn Ala
   595 600 605
   Ser Ser Val Glu Phe Leu Leu Tyr Gly Lys Arg Glu Gln Asp Thr Ala
   610 615 620
   Gly Val Leu Phe His Leu Asp Phe Asn Ala Leu Asn Gln Gln Gln Cys
   625 630 635 640
   Lys Gly Ile Trp Ala Ala Asp Ser Val Ser Ser Asp Tyr Glu Thr Trp
   645 650 655
   Ser Pro Ser Asp Gly Arg Ala Gly Gly Glu Lys Cys Ile Leu Gly Lys
   660 665 670
   His Ile Thr Tyr Thr Arg Arg Lys Gln Thr Ser Glu Cys Phe Asn Gly
   675 680 685
   Arg Asp Phe Asp Arg Pro Lys Val Ser Lys Val Cys Pro Cys Thr Met
   690 695 700
   Glu Asp Tyr Glu Cys Glu Phe Gly Phe Ala Arg Ser Ile Gly Ser Thr
   705 710 715 720
   Gln Cys Val Ala Thr Asp Ala Ala Ala Ala Ala Ala Ala Thr Ala Thr
   725 730 735
   Gly Leu Ala Gln Phe Thr Asp Glu Asn Asp Ala Ala Ala Ala Ala Ala
   740 745 750
   Cys Thr Ser Ser Ser Phe Phe Tyr Thr Ser Ala Tyr Arg Lys Val Pro
   755 760 765
   Gly Asp Val Cys Glu Gly Gly Trp Met Pro Glu Lys Val Ala Val Pro
   770 775 780
   Cys Pro Ala His Ser Pro Val Ser Arg Gly Gly Lys Thr Val Leu Leu
   785 790 795 800
   Leu Leu Leu Phe Ile Val Val Val Met Ile Val Ile Asn Tyr Leu Ser
   805 810 815
   Lys Thr Gly Lys Leu Lys Lys Phe Phe Arg Asn Ala Gly Phe Asp Asn
   820 825 830
   Phe Ala Asn Val Ser Tyr Gly Leu Val Gly Gly Ser Ala Ala Gly Pro
   835 840 845
   Gly Ser Trp Leu Asp Gln Glu Ala Gly Glu Gly Arg Arg Gly Glu Glu
   850 855 860
   Leu Gly Asp Arg Ser Lys Tyr Glu Pro Glu Leu Gly Phe Ile Glu Ala
   865 870 875 880
   Glu Gln Asp Glu Asn Glu Glu Asp Ala Pro Thr Leu Met Thr Tyr Gly
   885 890 895
   Asn Ala Ala Gly Gly Gln Arg Ala Asn Ser Gly Ile Ala Ser Arg Ser
   900 905 910
   Pro Lys Arg Lys Glu Glu Asp Glu Phe Glu Phe Glu Asp Ala Gly Arg
   915 920 925
   Pro Leu Phe Pro Ser His Ile Pro Arg Leu Ala Pro Pro Arg Phe Asp
   930 935 940
   Glu Glu Asn Val Glu Leu Leu
   945 950
<210> 21
   <211> 29
   <212> DNA
   <213> Artificial Sequence
<220>
   <223> 5' TgSORT_F
<400> 21
   catatgatgt catcaggagc ccctctgcc 29
<210> 22
   <211> 29
   <212> DNA
   <213> Artificial Sequence
<220>
   <223> 5' TgSORT_R
<400> 22
   catatgggtg actgtctgta caccggagg 29
<210> 23
   <211> 28
   <212> DNA
   <213> Artificial Sequence
<220>
   <223> 3' KISORT_F
<400> 23
   agatctatgg cgcacagctt cgcacgag 28
<210> 24
   <211> 27
   <212> DNA
   <213> Artificial Sequence
<220>
   <223> 3' KISORT_R
<400> 24
   cctaggccgc ggtagaccag acgcgtg 27
<210> 25
   <211> 28
   <212> DNA
   <213> Artificial Sequence
<220>
   <223> iKO-100pb_F (A)
<400> 25
   ccggcgtaga cgtgctaagt ctgtgcca 28
<210> 26
   <211> 39
   <212> DNA
   <213> Artificial Sequence
<220>
   <223> DHFR_intR (B)
<400> 26
   ggcgttgaat ctcttgccga ctgatggaga gggaagtcc 39
<210> 27
   <211> 10003
   <212> DNA
   <213> Homo sapiens
<400> 27
   caggaagatc acgtgaggcc agaagttcga gactagcctg gccaacatag tgaaaccccg 60
   tctctactaa aaaaatacaa aaaattagct gggtgtggtg gtgcgtgcct gtaatctcag 120
   ctattcggga ggctgaggca tgagaattgc ttgaacctgg gcagtagagg ttgcagtgag 180
   ctgagattgc actccagctg cactccatcc tgggcaacag cacgaaactc tgtctcaaaa 240
   aataaataaa taaaatttaa aggccaacca aggctgtaat tatatatcag taattaaacc 300
   aatcagtact taccaggata tttgttgacc acactcatga gaaaatggat ggttggatta 360
   atccatggta ggggattgac tagttaagaa cagaagatta gagaggactg gagagtattg 420
   ttggtgaatg tctcacccat agaaccagct ttcataggct gctgtgaaag gtcagtctaa 480
   gaaggactga catttgggag ccactgcgct agagcccatc tgctttaagt tcttagtcca 540
   ttagttgttc cttggagctc cagcctccct acatgaactg cttcccctaa gaaaaggaaa 600
   atatccggcc aggcgtggtg gctcatgcct gtaatctcag cactttggga ggccgaggct     660
   ggcggatcac gaggtcagga gattgagacc atcctggcta acagggtgaa accccttctc     720
   tactaaaaat acaaaaaaaa ttagccgggt atggtggcgg gcgcctatag tcccagctac 780
   tccagaggct gaggcaggag aagtgtgaac ccgtgaggcg gagcttgcag tgagccgaga 840
   tcatgccgcg ccactgcact ccagcctggg cgacagagcg agactgtctc aaaatatata 900
   tatatatcca agttacatct tacatctttc tttttaagaa aatctttata tgtacctcct 960
   tctgtcgagt gtccttactt tcaatgaact tttttgagat ataatttata tacagtaaaa 1020
   tgtacctatt ttaagtttac aattcagcgg atgttgacaa atgtacaacc cccagtatct 1080
   gtcaccccag tcattatatt gatcctccca aaaagttctc tcatgtccct ttgtagtctc 1140
   ttcccccaca ttgatctact ttttgtcact atagatttgt tttgtgttct agaatttcat 1200
   ataaatgttt ttgtttgttt ttttaagaga tgaggtctgg ctgtattgcc tagactggtc 1260
   ttgaactcat gagctcaagt agtcctccca ccccagcctc tcgagtagct gggactacag 1320
   gtgcacacac catgcctggc taatgttttt taattttttt agagatgggg tcttgctgtg 1380
   ttgcccagac tggtcttaaa ctcctggcct caggcagttc tcctgcctca gcctcctgag 1440
   taattgggac tataggtgat tatagggact atacttcttt cacttggcat gattttgaga 1500
   ttcacctgtg ttgcatcaat tctgttacat ttcattgctg agtgctatct catccatcct 1560
   attttttcac ctttattgcc aagtctcctc caaacagaac ctgtattatc tccactgctt 1620
   cacctctcat ttactcctca atcactacta tttgtttcag cttttattat ttccatgaga 1680
   ctgctccttg ctaagataac tcttatttgt cagatttcat ggtcactctt tggaactatc 1740
   atttgacttt tttgaaggat tcacctcaga tttgacagtt ctaaaattaa aatcattatc 1800
   tttcctaaaa accatttcat cttcccatat ttctaatctt ggttgatgac acccccaggg 1860
   tacttgtcat tcacacgaga aacctggaag actttgactt tccttccctt ttactcagtc 1920
   atttctttgc tcccttataa ataactccca aatccatgtc ccttctccat ccctacttcc 1980
   agtgccgcct tttgttcagg ttcccatttt tcccctacct tgtatatttt cagaggcttc 2040
   ctaattaatc ttgccctcct ccatttcagt ccattttttc ctcattgtgc tactatagtg 2100
   gattttctaa gctatatcta atcttaatgg ttctccactt aaaaaatatt ttctggctgg 2160
   acatgtggct cacacctgta atcccagcac tttggtaagc caagatgggt ggatcacttg 2220
   aggccaggag ttcaagacca gccaggccaa tatggtgaaa cccctgtctc taataaaaat 2280
   acaagaaatt agctaagcac ggtgacatgt gcctgtaatc ccaggtactt gggaggctga 2340
   ggcatgagaa ttgcttgaac ccaggaggcg gaggttgcag tgagctggaa tcacgccact 2400
   gcactccaga gcgagactct gtctcaaaaa aaaaaaaaaa aaaaaccttt tctgacttct 2460
   attgtctgtg agataaattc caaaattctt ttcatggcat aattcactac ttcttaagtc 2520
   atttgtttaa tctattagag actttgctaa gcattttaca tgcaattaca tctcttaatc 2580
   ttcacaacac ttctatgaga tagatattcc tattccgaaa atcttatttt acagaagagg 2640
   aaactggctt tgagacggta gggccaacag gtaaatggta gagtcaggat ttgaatccag 2700
   gtctggtctt aggtcttata cttttaacca ctttaacgta ctgtctccca tgagctctga 2760
   ccccctactc cctctccttt tcacgtcata ttccatgtgc aagctgtggt ctagccatac 2820
   agaattactt tcagttttgc aaatatgtcg tgttcttttg tggttctgtg catttgcaca 2880
   ttgtttgccc ttcctgtctt tgactgtctg acccattctt gccatgcttt aatacacaac 2940
   tcaaaagttg cttctccctg aagccttcct gggctcctcc agatcactct taaacatatt 3000
   cttagttcta ttacaacact tacctgacat tgaacacatt tggcatctag cacttagtaa 3060
   ataaagactt tcttcatctt tggatcctta gtcacaagta catgtaacta atacacaggt 3120
   gcttagtaat taattattgg tgaaataaat tgaaagaaaa tatctgttct acctgcctca 3180
   tagagatgtt ttttgaaagt gagataataa atagaaaagt atctttcata ttatgcaaat 3240
   acatttatta ttatgcaaat gtaagggatt atttttactt atctagtgat gagcaacttc 3300
   ctgaaggcag tccttggctc agacgctatt attaatagta ttgatttgcg ttccagttct 3360
   tctgtaggac tgttccactc agagttttcc cagtttctgc tttttagccc ctgtaatgtt 3420
   ttgagaaggc tagcctgagt catgagtgaa attctttagg ccctcactct ttagattatt 3480
   gctcttatat ttctctgtcc tcatgtcatt gatcatcaag taaagatata tcttaagaga 3540
   agcagtagta acatttttat ttatttattt atttatttat ttgagacagg atctcacttt 3600
   gttgcctggg ctaagtgcag tggtgcgatc tcagctcact gcaacctcca catcccatgt 3660
   tcgagcaatt cttgtgcctc agctgggcct acaggcgtgt gccaccatgc ccgactaatt 3720
   attgtatttt tagtagagac agggttttgc catgttcacc aggctggtct cgaactcctg 3780
   acctcaagtg atctgcctgc cccagcctcc caaagtgcca taattgaagg aattacaggc 3840
   taccacgcct ggccaagaga agcagtaata atatttgtat gtttaaatat ccttggaatg 3900
   gaactcatgg tctacatggt aaactaattg tttacctata ttttgcagat atatttggga 3960
   cttttctgac atttcatgta attagagata tatcttttag gagaatctag aggagactac 4020
   aagctttgag aggagaactt tttttttaag tctgttccca gatatcagat gtcttaagac 4080
   ttgcagcagt ctttctgaat tttacttata gatgaggcag aggatactaa acatcatata 4140
   actggattat gctgattctc tctagcaact ttcaagttac attaaatatt agtactgaaa 4200
   attactttaa aatgccctac tatatttaat tttaaaaaat tattattaat taatttattg 4260
   ttttagacag agcctctttc tgtcacccag gctggagtgc agtgggatga tcacaactca 4320
   ctgcagccac aacctcccag ggttaagcaa ttctcccacc tcagcctcct gagtacgtgg 4380
   gacacaggcg tttgctacac cccctgctag tttttggggt tttttttttt tggtagacac 4440
   ggggtctccc tgtgttgtcc aggctggttt ttgactctcg ggctcaagtg atcctcccgc 4500
   ctcagtctcc caaagtgctg gggttacaag catgagctac tgtttccagc catgtgttta 4560
   attttaaaaa ttgaagaaaa aagacactat gaggaaatga catgtaaaac acaaatcttt 4620
   tgaggcaaat gtgaagtggc acctattgct cagtattgcc caaaactgtg ggacatgatc 4680
   aacagagaga atggaaaacc tagtcatgat agttaaattt gaaattaacc atcttctgac 4740
   cctagttttt tattgttgtt gttgctttta aatgtaaata taatctggat ttccagttat 4800
   tactgatatc ttcagcagtc acaactggtt tcctatgcca gtgttaatga aatcttttct 4860
   tggccagttc ttttaaaaaa acgtaacagg ccaggtgcag tagctcacat ctgtaatcct 4920
   agcactttgg gaagctgagg tgggcagatc acctgaggtt gggagttcga gaccagcctg 4980
   accaacatgg agaatcgccg tctctactaa aaatacaaaa ttagccgggt gtggtggcgc 5040
   atgtctgtaa tcccagctac tcgagaggct gaggcaggag aatcgcttga acccgggagg 5100
   cggaggttgc ggtgagccta gatcacgcca ttgcactcca gcctgaacaa caagagcgaa 5160
   actccatctc aaaacaaaac caaaaacaaa caaaaaaaac ttttttgtta agttttgcca 5220
   cataaacaat tttagagaaa atacctgaca atagttaatg attatttgtt gtaggcaata 5280
   atggtctaag cactttatat atattaacat ttaatcctca caactactct atgaattagc 5340
   tattcttatt gtcctcattg tacagatgaa gaaatagagg cacagagtgg ccaagtaacc 5400
   tcaagtcaca cacacaagct caacttttaa gtatttaaat atacttcaaa tatatttcta 5460
   aaggagtctt taacagaaaa aagtactgac tccatgtact ttgctgctgc ttttgtgatc 5520
   cctgttgaag tgacttcaag aagtgacaat gatgccaggc atttggttaa gtatgatgtg 5580
   gtgagttaac cttaaaatag aaaatttggt caataagcat ttttattttg gagccagaga 5640
   gctattttag ggacctgctg cctggaaagg atatccttgg gacagcagat cccttagttc 5700
   ctaatgaata gctgcatcac caacacagct tctcctgacc atggtagggc catcagcagg 5760
   gtaactcagc ccagcttatt aacccttctg ttttctttcc agctagccac agtgcagcct 5820
   ggccagaatt tccacatgtt cacaaaggaa gaacttgaag aggttatcaa ggacatttaa 5880
   ggaatcctga tcctcagaac ttctctggga caatttcagt tctaataatg tccttaaatt 5940
   ttatttccag ctcctgttcc ttggaaaatc tccattgtat gtgcattttt taaatgatgt 6000
   ctgtacataa aggcagttct gaaataaaga aaattttaaa atatttgtta atagactgtt 6060
   ctcttctaat agtctttttt tttttttttt ttttaagaga tgaggtctca ctatattgcc 6120
   caggctggtt tcaaactcct gggttcaagt gatcttcccg cctctgcctc ctgaagtgct 6180
   aggattatag gcgagagcca ctgtgcccag ccattgtaat acagtctttt gtttgaaatg 6240
   caaatgttag tgggccaaaa ggacagtgtt attaatatcc ctattctatg aagatgtctg 6300
   ttctaaaact gttttagtga tgctctataa cagaatcaac tatatccatt aatcccatgg 6360
   tgttgataaa tgaactagtc cagctcttcc cagtggttaa tagatgttaa cagcactgta 6420
   aatccatttc ggccctataa tttctggagc aaacgtgtat ttttgctgct tttcttataa 6480
   ggagatgcta ctaattgact atcagaagaa catgttttga ggtcctcctc aaaacattga 6540
   tgggttggaa tcagccatca acagtgttgt accagttctt gggtcttgat agacaatttg 6600
   gaaagagatt tccttgttta cgaagtgaag gactaagaat taaattaatg accttggcct 6660
   cactggcaat caattacaaa tctctatatc agtaagagaa ttgtaaaatt cagaagcagt 6720
   ttacattaga ctttggaatg aatgatgaac tttccccatt agagctgcca tcatatgatt 6780
   ggtggagatt gtttaaagaa tcattctatc tcagcctcaa aaattaccac gggaagatgg 6840
   tatcagtatc actacttaac agatgaagaa tctaatgctg tgtgcccatg gacacacaaa 6900
   attaagctgt tgggacttga acagctcaaa ttttcttgtt tcagatcacc agctcttgag 6960
   taatagggaa atctggagat ttgaaaagta cttgcactgt ccacaacatg gctaatccat 7020
   atgtggatga tgcagaattg atttaattca ctaacaggga ccccataatt atttgctggg 7080
   ctctgggtat attgatgtgt aggaagttgt actatatgtt aataaattat gactatttgg 7140
   ataggctgaa ttcatactaa atccatactg tagttcaaca aaaaacaaca tgtttatata 7200
   catttggaaa ctgcaatgat aggaaacatt tggaagaaag ggattttgcc ctagaataac 7260
   acataaggaa agcagagaat tagagtatat tgttactgaa tgttccagag acttctagtg 7320
   attttaacac ttattaagta tagcatgttg atggggagat ttgtttctct aaagatcact 7380
   ttgttttatt aaatcctaat agaaaatacc cttgaaaatc ctatctcatt tattcctgta 7440
   acaaatttat tgagcaccta cctactatat gccaagaaat gtattagcca tcaggcagag 7500
   agctgttcaa atggtagata tgatctgtgc tcctgtgaaa cttctagtgg ggagagccaa 7560
   acaataaaca atattccttt ttaactctag gttaatgcag aaatggataa agcccatgtt 7620
   gcttttggga gaggtgagtt gtgtctctga atcacatgca tttcataggg ggaaaaatgc 7680
   ccaaatacta aggagtagct cttagcctat gtccgttcgc ctcagcttaa gttgttttcc 7740
   ctcccactgg ctgggcagca tatttgtgtt ttacctggta aaacagtaaa tttcgtacct 7800
   tctataattg gcattcttca aagaggtaga actttgattt ttttgtagaa tattaaaaca 7860
   agctttctta ggttaaagaa gtgatcttac tacgaacagc aatggtttca tgatgtgtaa 7920
   ttgctttatg ttatctttct gctatagaat gtttcctgga gaaaggtacg gtttttagta 7980
   ataagataaa tttactttag tcctgcaaat aaggtgaaat cttatgtcca gtatctcaca 8040
   aaggagaact gacagtgcca cttttatatt taatgtcaat ctcattagac aaaaatgaaa 8100
   tatagttcct agggtttcca atttaaaaag tgaaataata aatatattca ctcaaacatt 8160
   ttctcagtgc catatgcaag ttaccatgtc ccagaagctg atacagtttg aagaaggaaa 8220
   tatataagta ctgtaagcct gtgataacta aaagcaacat actaaaaagt gaaggtacag 8280
   ataattggaa caaatttaat agcgtttaaa agtgtatttg gtaatttctc caggaggtgg 8340
   cactgttgag ttgttttttc ttttcagaca gtaagacaat agaccattca tctttgtgtc 8400
   acccaaacaa agaaatattc ctttataacc ccctttcctc cccccaccta atcacatgct 8460
   actggaactg actgtcttgt gatctactcc catagttggc atagatttaa aaataatctt 8520
   ttttaaatta atctgatagt agttatggaa aggaacaaat tgttcaccaa ttttcatcag 8580
   ctttatcttt tcagtccagt aactgagaac ttaaaaagac tattctagtg attttactat 8640
   cctgctcaga tggcattcaa aaacattttt cacttctcac gtcttggaaa aatttgtaaa 8700
   ttcgttaagt ctttttgttt gaaagttaat gtgactggaa atattttaat gtaaatatcg 8760
   ggttgggtgc attgtatcat tttgctctac aatgactata tcctcatttg ggtttatggc 8820
   cagtttataa ttaaaagtaa tctttacttg ctttgagtgt aatgacttgc atattttgta 8880
   tctttctatt tgctaccttt tctactgaac aatttctagc tttctgaagg gatggagtca 8940
   gtaagcttaa ggattcagaa ctggttttac ctgtgctacg tgttgtagaa gttgtgtgtg 9000
   cagcttctgc ggtaaaggga acagcgtagg ggttttattt tattcattag aggtggatcg 9060
   gggtgctaat ttagaggaca tcacatactg gaaccaaact tgtccgaagc tctcactgca 9120
   gagttaaggc actttggtaa aataaatgtg catctaggaa atgatcatgt gtacagtagg 9180
   cattggatca ttctcttgcc cctcttttac ctgcccctaa tgcctaagct acattcgaag 9240
   tcctggaaaa cctgtaagta attccgcctt tggcctacct cccctcagaa gaggaaggtg 9300
   agagaggcca aacgtatgga aagactattc ttcaggcttg caggagtcac ggatgtcctg 9360
   atgtctgtct ccgtaggatg tggcccacat cagttcgcat tcaccttctt gcagcaaggc 9420
   agctgcttag cagacaatgg gcgctccagt gagcggggtt tataaaaacc cgaagccccg 9480
   gttcatgatg gagccccctt ttccagctga gcaagctcag ggatttcctg ggtaggtttt 9540
   tccaggctct gcctcaccga aggaatttta ggagtgtctc tggggaacag gagggacgta 9600
   acccagcccc aacttgaggg cgctagaggt gcggcaaggg gtcgcgacgc caggagcccg 9660
   gggctcggcg ggaaggtatg agaagctcct acgtgaactc cacaagccgg gcccgggaga 9720
   cgccgggcga ggcggggttg acctcagcag tctctgcccc gttccagcca atcagtcccg 9780
   catcttagca tccgaatcca ggacccccga agccggaggc gacgcgagcc aatgaggagt 9840
   gggccgggga agagggacag gcggccagcc tatggggcgg agaggcccgg ccgcgcgtat 9900
   ccaaggagcg ccgcgctcgg ctcgggggtg tggcgcgcgc cggcgggggt gggcgggcgc 9960
   gccgggcggc aggtgtcggc gtcggcggca ttcggcggcg atg 10003
<210> 28
   <211> 10000
   <212> DNA
   <213> Homo sapiens
<400> 28
   atggagcggc cctggggagc tgcggacggc ctctcgcgct ggccccatgg cctcggcctc 60
   ctcctcctcc tgcagctgct gccgccgtcg accctcagcc aggaccggct ggacgcgccg 120
   ccgccgcccg ctgcgccgct gccgcgctgg tctggcccca tcggggtgag ctgggggctg 180
   cgggcggccg cagccggggg cgcgtttccc cgcggcggcc gttggcgtcg cagcgcgccg 240
   ggcgaggacg aggagtgcgg ccgggtccgg gacttcgtcg ccaagctggc caacaacacg 300
   caccaggtga gcgggcgccg ggcccgggag ccgctcgggt gcgaggtgcc gcccgcgtgc 360
   ccctcccggc ccgtgcgccc cgcgactccc ggaggagacc ccagccctgc cgcgcccgcg 420
   tcccccgggc cgcagccccg aaactgcggc gggcgggcgg gcggagtcgg gcccgggcgg 480
   ccggcggggc tgtgttttgt cataggggag gcaatcttag acttccaccg cattcttttt 540
   ttttttttta agttgtcaaa atctcttgaa catcgttttt cgtctgtata aataatcagg 600
   acccagtaac gctgttaggc cccaggaagg tgaaggagta attaactctt gagcgcttgc 660
   agaagttggg ctgcgcagaa gcaaaggtgt gtcctctctg ggtcagtact ttccgggtgg 720
   gagaggcggg agttcaggag gcccgaagca gcgatctggt caccgtgttt taatagttaa 780
   ggggactagc ttggaaatga gatggcaata tttatgtgat ctcttttact gcttcagatt 840
   tgaccttctg tcatggtacg gaagtctgac tagcatgtct atatcccgtg gtgacaagat 900
   tttcaatagt taagaaacgc attgcacata ttctgctttg agccagctct tggtcaaaag 960
   actttcaaaa ttatgggatt tctgtccctg gggacaagca gctcaacctt tgcacactac 1020
   tggactgaca aaactaatta ttaagaaagt cctggttcct tgcttcagca caacttgggc 1080
   atctttctcc tatttctcta aaattaacaa tttaatcctt gctagccaca tttgcataat 1140
   gtattttcaa ttctatatat tttctccctt gcctgttttc ctctttcaaa gaaaatgaat 1200
   tgcacttggg ggcagagatg ggtggccaag gctctgtgcc agcacttgtc cttctgtaac 1260
   cgtttttcag cgtcagccaa gcctcagtct ccctttggac tgcctggtta tctggaccat 1320
   acttgttacc tgtaatagaa ttctctgatc tgaggacaca agacacaaat attcaggaat 1380
   aattgtgctt agtgtatccc gagcagcagc tgtgtaaagg tagccaagct agtgctggag 1440
   ggtgagacag gttgggtaga atgagtactg ggaagtcttc ctacttgatg agaaaaaaaa 1500
   atcgaataga cttccttctc acagtggggg attgagaaag gctttacaac actaatgctt 1560
   aatttatact ggaccacttc cttccttgag ctagctggtc acagcattct tgctccatat 1620
   ttcaaattct tctctcgctg gttgacctaa ctttaggtct gctgctactc ctggatttag 1680
   atttactcct gtttttcttt ttctcttttt ttgtgagata gagtcttacc tcagcctcct 1740
   gagtagctgg gactacaggt gtgcaccact atgcctagct aatttttgtg taattactat 1800
   tttttttttg agatagggtc tcactctgtt gcccaggctg gaatgcagtg gtgtgatcac 1860
   agctcactgc agccctgacc tcctgggctc aagtgatcct ctcacctcag cctcccaagt 1920
   agccgggact actggtacat gcacccagct aattgttctt ttctttcttt ctttcttttt 1980
   ttaagaaaca gggttttgcc atgttcccca ggctggtctt gaactcctgg gctcaagtgc 2040
   tccacccacc ttagcctgcc acagtgctga gattagaggc gtgagccact atgccctgcc 2100
   tggatttatt ccttgagtca gagtcttcct ggttagtttg aattgatgtg tcatttgtaa 2160
   tatattgatt ccaaagaaag aggttgaaaa aactggctca ggggttctaa ggccaaatct 2220
   gtggaagagt agacctaaaa ccactcattc tttgaagtcc acatgtaaag aaattttctt 2280
   ctatttcttt catttagctg tttgcaataa ttttttttta gtttttctct cccagtcttt 2340
   ttttcagtga ccttatttaa aatagctatt ttggctgggc acggtggctc atgcctgtaa 2400
   tcttatcaat gtgggaggcc aaggcaggtg gatcacttga ggtcagaagt tcaagactag 2460
   cctggccaac atggtgaaac cctgtctcta ctaaaaatac aaaagttagc cagacgcggt 2520
   ggcatgcgcc tgtaattcca gttattctgg aggctgaggc atgacgatca cttgaaccca 2580
   ggaggcagag gttgcagcga gccgagatcg caccgttgta ctccagcctg ggtgacagag 2640
   tgagactcta tctcaaaaaa aaaaaaaaaa aaaaaaaaaa aaaagttttt gcgtttgtta 2700
   gtcatccgtg gtctcttctt cagagatctc atcctttctg tatctggtta aaaaaaaaaa 2760
   aagtggagcc tttgtttaat atcatttacc ttaaaaatgg ttaagtagta cttcttaacc 2820
   aagaattgtt atgtagtagc ctgttcttct gtttatttat ttggttttag aagtcataaa 2880
   aaatcctgaa ctgtattgaa gagatctagt atttttggac cgtttataaa acagtgtgaa 2940
   ataaaagcat cagcatgaca gaacaacttt ttatcagaga tattgtactt tgtacaactg 3000
   gggaaggaag gtacatgtag aatcattttt cctacataac atgacaggat tcgagtccca 3060
   agaaaaagca aaagttcatg gttctcttat ttcttttaac ttggtcctgt ttcatttcaa 3120
   gccacattaa ttcacatcat caatgtatgt gaatgccctc caaatgccta gatctgtgct 3180
   agatgctggg gtgccaagat acaaagaaaa gatcattcag actggagaat gaaaagcctc 3240
   agaaggttat agcaaactac tagacctgtg tctgtggctt atgaggatat attagcattt 3300
   aacctgtctt ctcaagaaag cacggtaaag ctaaagcata atagtgcgtt tatattacgg 3360
   tttaaggaga ccggtgtcct gatgaccact caggcttctc tgtccatgaa atccagcagg 3420
   tttttctctt tgcccaagcc tccctttttc tctctaaggt tttttttctc atatcatttc 3480
   ttttcaatct attaatttaa aatatattta aagtgcattg ggtttattac acacagcata 3540
   tattttttca catagaagtc ctaatgatta agtgctttat tttaggagtt gaggcaggag 3600
   aggaaaacat tttcctttct agttacagta ttacaagtta gctataatag actcatggga 3660
   aagtggaaat aaaaaatact ggctattact aaaaaaacaa acaaaaggga tgacaagaaa 3720
   acgaaggaga aagataaata gttataactt tccaaaaatt atgctcccta attttgttct 3780
   aagggtttga cttaccatgg taaggtggta tagcactgcc ttatctggac tgcctgcatt 3840
   tgaaccctgg ccatgtcact gtgtgatcct gggtgagtta cttaagtttt ctgtgtctca 3900
   gttcctaatc tgtaaagtga agataagagc acttaatttg tagggtggtt gtgagaatta 3960
   aatgagtgtg tgttaaatta tatatacata atatatacgt atatgtatat tattgtataa 4020
   atatatattt gtattacata atatatgcat atgtaatatt gtatttgtaa tgagttatag 4080
   tatgtattac aacatatcac attgtgttgt ataatctata catatgtaat aactcattag 4140
   aagaatgtct ggcacataat tagcactcag caattagcta tcatttataa atgctctctt 4200
   cttagactca ataatcttcc ttttgcctgg aactcaaaac tgttttcatg ccacattttt 4260
   aactgattct ttccttcagc tgcttggagt attcatgaca tgtataacct ttgtacatgc 4320
   gtgaaattga gcaggcctag aagactttat tgttggttac taagttggat tgcttcaaaa 4380
   catttctgct gtaagcaccc ctttgagggt aggcatgaaa ctgtcttaaa ggaacagatg 4440
   aaatgccttg gccagagttg tggtggtggc tgacataatt tccttacttc tcattcctgt 4500
   gtacagacca tttgacaatt tccttaaaac tttttaaact tatgatgaat aaagccagga 4560
   actcttgctt attttctgtc ctccctctgc tcctgtcact aactacttct gtaaacataa 4620
   cttcccaact tctactccaa ctttaaagca tctggttcct taaataaaca ggccctgtag 4680
   agaggccgtg ttgtatgggg tgtcagctct gggaagaatg cgtgtgtttg tttgctggct 4740
   ctggcagggt tgagtccaag ctgacaggac tgagtgtgcc tgtcagaggc ttcagggagc 4800
   tgccgctgaa gacctcggcc tgttgttgtg aatcgccaga cctttgtaac acccggaagt 4860
   ctgaaggtca gtcctttaat gccccggaac ttcctaactg cctcatttct tccaatactt 4920
   ggcctctctt catcctacct tccctagcac ccccagccgt ctagcttatt taatagtctc 4980
   ttgagttctg tttcttccat tcagctttgg atcaaggagt aggaactctg taggatctcc 5040
   ttcttggtcc tggttgagca gagtgagcga aaccttcatg aagagtcttc tctcatgcaa 5100
   tgtatagtca gacctttagc ccctagaggg atgttaaaat gacttatgca ttggatattt 5160
   cagtaagaag tatcagcaga tttgatgaag tgtaatttat ctggggaaag gttagtcttc 5220
   atctgaagtt tggttgggat gagagtagaa agctagccag gtatgacaga tggctccaag 5280
   ggaatgcagt gttaacaatt aggtaagttc tctgctgact caaatgtttg gctctgtgca 5340
   cagtgatttt taattagaaa tctgagtctc atgtctgctt gtggaaataa ttggaagaag 5400
   gcttatgttc tgttcaattt ctaatggcta ttttcttttt gtgattatta acaacaataa 5460
   taggctttct atcctgctgt tttgatcaga tctttcatgt cagggtcaca gatactatta 5520
   gcagtttctt ctgcactctg ccagtttcca gtcaactctc gtcaacaggc attgggttct 5580
   cagtgggtga ctgcagagtc tgcgttcccc aactttgtcc cagttggcct gttacccttg 5640
   tgtgaccatc acatgctggg cagtgcttag caccaagttg taatggtcag gacgggggag 5700
   taacgtttgt tcttctccct ccttcctacc taatcctacc cctcaatttt ttttacagaa 5760
   agtgaactat gttttgaggc tgcagaaatg aggctcagat attttatata attaggagct 5820
   aatattaagg cagacaaagc catcacattt gacatgtaat ttaaaataca gcttttctag 5880
   aaacaacaaa actgtggcaa tggctctgac aatattatga actagatact gcatttgtag 5940
   atttctacaa gtttcagttc cctttctctc cttttgtcat cagtgttttt attgcatctt 6000
   ttacttctat ttttaaaaac ttggtgaaaa cttgttcttc ccactatgtg gtctcagtga 6060
   tttgtatgca ggtggatttg aggattaatg gggcttgaag aacaagctga tcaatttcag 6120
   tcagttctag gcagcttttt tgttcttgtt tcaatggtga ctgcagtcaa agttgggtta 6180
   gtaatatgtt gctttgagat agtttggttg aatgaatgtt catgacagat tcctcatacc 6240
   tatgaaaatt tttaggtgaa agatggaggc tgtttttcct tctctatccc caatctcaat 6300
   ctcaggaaac cacagtaaaa acaaaatcct tccactcaga acctgtcgtg ttttgccgag 6360
   gaagttttct gtgaatgtaa cttattttct gttttgtgtt ttagtctctg gccctcgatt 6420
   cctcatctac agcacaagaa gttttggact agctttaaaa tttctttcaa aaattctatg 6480
   attcatatgg gagaggccag aggttgtgat cacaaatgtt ttcctggctg attccaaatt 6540
   tagaagcaaa ctgtggctgt ttgacttccc agtcaaactt gaacatagca tggatttcac 6600
   atttaggggt gttttactac cacctccaga acttgcttca gtgaaatatg catgttctta 6660
   aagatagcag gagtactttt tttcatgtag ttatgaaaga catttttctc ttttattttg 6720
   ggggtaatat tttaccgttt gttccttgtt ggctgaaaaa tctaatgcta ataaaagcta 6780
   ctagcttgaa tagtccaaaa gcaaacccaa cagatagcag taaattcatt tttctttttc 6840
   cctttcctat agtactctgc caaaactggg gctttttttc ttcatcctgg ctgcctgtgt 6900
   gtttgaaaat agatggtcag cttaccataa atatattcac atgtagaaac ttatatgtga 6960
   ttaattggct tgatttattt ttatcagaag aaaacgtatg tatgttcctt ctctggctta 7020
   tgaatactat ccgtcttatg tttgctttgg ataagcattt tagtcttcct gtatgtccta 7080
   aaacgcgcgc gcgcacacac acacacacac acacacacac atacacacac acacacacac 7140
   tttctaatat tgtactgaag ccagtttgca agtctggttc cacagtacaa ttgctctgaa 7200
   ggaccaggag aaattgaact gggaaggaaa atttgtatta acttcaataa ttcccagaca 7260
   agttagttca aatcattttt atctagtatt tgctatttgt cacaaaggat tcaaaacata 7320
   aatatgttgt atacaaaatc tcaaagagat gtctcaatac ccagatttgg gtcctggctc 7380
   tgctgcttca tagctgtgtg attatggaaa atcacttaac ttcagtggtc tttacagttg 7440
   ttttttaaac tagagcagca gctcttagga ttgtttggag ggtcaaatga gaagttttat 7500
   caacgtgttt tgttaaaggt aaagtactat atagatgtca ttcatttaga cacttactga 7560
   gctgcctgcc atgtactgag tggagtccag acctgtgcat aaacaaagca aagcactggg 7620
   gctatcttaa ggatttaggt gttcagagat gtttcactgg gagtttggaa gaaggcttga 7680
   ccactgcagt gcagtgttct gggaaagccc tctggctgaa gagagaatca aagtggaggt 7740
   gatttttagg gagatctgta acggcacgaa ggaagtagga aaatccaaga tatgttcaag 7800
   ggatggtcaa cagaaacttt attttagggt gaggtagttg atggggagta tgactaaggt 7860
   ttccaggaaa acatataggg aagttaaaaa aaagatagct cttttgtggg ggatggaaag 7920
   agaggagggg cgtgggtagt gagtttggca aaaggactta cttgtgaaag agagagtttt 7980
   aacagatagt gctagacatg aggctagagc tggaggaacc agaggtagaa atggggagcc 8040
   aagaagagtt cccctttcag aggccggcca ttgctttggt cttggatgtg aaggtcccag 8100
   aggaaagcag aacagaaggt gaggcagacc atcctttata cttgaagtgc taacgtttcc 8160
   aaaaagcact cctaaacttc tggacctggt tgaagaagaa tgtgagccaa ggcatgagga 8220
   agccaggagc tgtaagacag tgatgcctat gaacagattt ttctgaacat ttgtgaacaa 8280
   ggataaggtt gaactcttgc tgataatgtg cttaatggtt tgaatcatgg attattgggc 8340
   cgattagctg gtcagagccc ttttgctgat gcggctggct gacttgctta ctggccaaga 8400
   gctgggagta gaacacagat gtcccaacat ggtgctgcat ttggaagtgt ttcataggag 8460
   tgttgaaatc aatgcatttg ctttagggat aaggcaaagt tatgagatcc cagttataag 8520
   taatatagtt gtgaattcta ctctttttct caaggtcata gtaccctcaa cagagctcaa 8580
   ctagcctttc taggcctcag tttcccgtca cataaaatgg aggattatca tgaagttttg 8640
   aagggaagat atatgtgaaa atattttgaa accttgaagt accgtatgag tatgatttgt 8700
   ataattcctt aataaacctg ctgatgtgtc acagccccca cagggcctgc ttgtcagcca 8760
   ggtgatcctg atagatacag cccttgagcc aaaccctgag gagcatgtgt attaaagcag 8820
   acggatgagg cataaatgcg tttggtttat cacttggcag agttggatga caggcttggg 8880
   tgggggtggc aagaattaaa gtgagtcaag attggcagaa atgtaagacg tgtatgtagt 8940
   ggttttcttc tttttttaag cctttgtgat tatgtcaaac ttttgttaat ataattgatg 9000
   atggtttgag gcagtagttt gggggaacag gatctcaggt attagatctg gattcaaact 9060
   tcagtggtga ccatggacaa attaacctct ctgaacctta gatttttcat ctatgaaatg 9120
   ggagtactaa tactacctct tagggtcatt gtgaagatag tacaggattt agtgtatatt 9180
   cttttgttga ggatttcttg taatgatttt cattacaaag cttctgacac attaccagtt 9240
   agatgcttaa atatttgtac atgtgcttaa tttttttcat atctctaata tttctagaga 9300
   attattatag gtactcacaa gaactgcatc tggattaaga catattagcc ctggctaagc 9360
   agggtgactc acacctataa tcccaagatt tttggagtct gaggcaagag aatcacttga 9420
   gcccatgagt ttgaaaccag cctgggcaac atagtgagac cccatctgta taaaaaaatt 9480
   tttttaatta gcctggtatg gtagcatatg ccggtagccc tagccacaca agagtctgag 9540
   gtgaggggat cacttgagcc caggagtttt gaggctgtcg tgagctatga ttgcgccgct 9600
   gcattccagt ctgggcaaca gagcgagata ctgtcttcca aaaatacaaa aaaatttatt 9660
   aggggtaggt agtggtgcac atctagagtt ccagctattt ggaaggctga ggcaggagga 9720
   tagcttgagc ccaggagttc aaagctatag gacactgtga tcgcatctgt gaataaccac 9780
   tgtacgccag cctgggcaac atagtgaggt cccatatcta aaaggaaaaa aaaatgtact 9840
   acttgtatca ctggactcag gatgaaaaaa gaggagggtg ggagataggt ttaggcagag 9900
   atcatctatt caaaatgcct ttctttcttt ttcttttttt tttgagacag ggtctcgctc 9960
   tgttgcccag gctggagtgc agtggcacta tctcggctca 10000
<210> 29
   <211> 831
   <212> PRT
   <213> Homo sapiens
<400> 29
   Met Glu Arg Pro Trp Gly Ala Ala Asp Gly Leu Ser Arg Trp Pro His
   1               5                   10                  15
   Gly Leu Gly Leu Leu Leu Leu Leu Gln Leu Leu Pro Pro Ser Thr Leu
               20                  25                  30
   Ser Gln Asp Arg Leu Asp Ala Pro Pro Pro Pro Ala Ala Pro Leu Pro
           35                  40                  45
   Arg Trp Ser Gly Pro Ile Gly Val Ser Trp Gly Leu Arg Ala Ala Ala
       50                  55                  60
   Ala Gly Gly Ala Phe Pro Arg Gly Gly Arg Trp Arg Arg Ser Ala Pro
   65                  70                  75                  80
   Gly Glu Asp Glu Glu Cys Gly Arg Val Arg Asp Phe Val Ala Lys Leu
                   85                  90                  95
   Ala Asn Asn Thr His Gln His Val Phe Asp Asp Leu Arg Gly Ser Val
               100                 105                 110
   Ser Leu Ser Trp Val Gly Asp Ser Thr Gly Val Ile Leu Val Leu Thr
           115                 120                 125
   Thr Phe His Val Pro Leu Val Ile Met Thr Phe Gly Gln Ser Lys Leu
       130                 135                 140
   Tyr Arg Ser Glu Asp Tyr Gly Lys Asn Phe Lys Asp Ile Thr Asp Leu
   145                 150                 155                 160
   Ile Asn Asn Thr Phe Ile Arg Thr Glu Phe Gly Met Ala Ile Gly Pro
                   165                 170                 175
   Glu Asn Ser Gly Lys Val Val Leu Thr Ala Glu Val Ser Gly Gly Ser
               180                 185                 190
   Arg Gly Gly Arg Ile Phe Arg Ser Ser Asp Phe Ala Lys Asn Phe Val
           195                 200                 205
   Gln Thr Asp Leu Pro Phe His Pro Leu Thr Gln Met Met Tyr Ser Pro
       210                 215                 220
   Gln Asn Ser Asp Tyr Leu Leu Ala Leu Ser Thr Glu Asn Gly Leu Trp
   225                 230                 235                 240
   Val Ser Lys Asn Phe Gly Gly Lys Trp Glu Glu Ile His Lys Ala Val
                   245                 250                 255
   Cys Leu Ala Lys Trp Gly Ser Asp Asn Thr Ile Phe Phe Thr Thr Tyr
               260                 265                 270
   Ala Asn Gly Ser Cys Lys Ala Asp Leu Gly Ala Leu Glu Leu Trp Arg
           275                 280                 285
   Thr Ser Asp Leu Gly Lys Ser Phe Lys Thr Ile Gly Val Lys Ile Tyr
       290                 295                 300
   Ser Phe Gly Leu Gly Gly Arg Phe Leu Phe Ala Ser Val Met Ala Asp
   305                 310                 315                 320
   Lys Asp Thr Thr Arg Arg Ile His Val Ser Thr Asp Gln Gly Asp Thr
                   325                 330                 335
   Trp Ser Met Ala Gln Leu Pro Ser Val Gly Gln Glu Gln Phe Tyr Ser
               340                 345                 350
   Ile Leu Ala Ala Asn Asp Asp Met Val Phe Met His Val Asp Glu Pro
           355                 360                 365
   Gly Asp Thr Gly Phe Gly Thr Ile Phe Thr Ser Asp Asp Arg Gly Ile
       370                 375                 380
   Val Tyr Ser Lys Ser Leu Asp Arg His Leu Tyr Thr Thr Thr Gly Gly
   385                 390                 395                 400
   Glu Thr Asp Phe Thr Asn Val Thr Ser Leu Arg Gly Val Tyr Ile Thr
                   405                 410                 415
   Ser Val Leu Ser Glu Asp Asn Ser Ile Gln Thr Met Ile Thr Phe Asp
               420                 425                 430
   Gln Gly Gly Arg Trp Thr His Leu Arg Lys Pro Glu Asn Ser Glu Cys
           435                 440                 445
   Asp Ala Thr Ala Lys Asn Lys Asn Glu Cys Ser Leu His Ile His Ala
       450                 455                 460
   Ser Tyr Ser Ile Ser Gln Lys Leu Asn Val Pro Met Ala Pro Leu Ser
   465                 470                 475                 480
   Glu Pro Asn Ala Val Gly Ile Val Ile Ala His Gly Ser Val Gly Asp
   485 490 495
   Ala Ile Ser Val Met Val Pro Asp Val Tyr Ile Ser Asp Asp Gly Gly
   500 505 510
   Tyr Ser Trp Thr Lys Met Leu Glu Gly Pro His Tyr Tyr Thr Ile Leu
   515 520 525
   Asp Ser Gly Gly Ile Ile Val Ala Ile Glu His Ser Ser Arg Pro Ile
   530 535 540
   Asn Val Ile Lys Phe Ser Thr Asp Glu Gly Gln Cys Trp Gln Thr Tyr
   545 550 555 560
   Thr Phe Thr Arg Asp Pro Ile Tyr Phe Thr Gly Leu Ala Ser Glu Pro
   565 570 575
   Gly Ala Arg Ser Met Asn Ile Ser Ile Trp Gly Phe Thr Glu Ser Phe
   580 585 590
   Leu Thr Ser Gln Trp Val Ser Tyr Thr Ile Asp Phe Lys Asp Ile Leu
   595 600 605
   Glu Arg Asn Cys Glu Glu Lys Asp Tyr Thr Ile Trp Leu Ala His Ser
   610 615 620
   Thr Asp Pro Glu Asp Tyr Glu Asp Gly Cys Ile Leu Gly Tyr Lys Glu
   625 630 635 640
   Gln Phe Leu Arg Leu Arg Lys Ser Ser Val Cys Gln Asn Gly Arg Asp
   645 650 655
   Tyr Val Val Thr Lys Gln Pro Ser Ile Cys Leu Cys Ser Leu Glu Asp
   660 665 670
   Phe Leu Cys Asp Phe Gly Tyr Tyr Arg Pro Glu Asn Asp Ser Lys Cys
   675 680 685
   Val Glu Gln Pro Glu Leu Lys Gly His Asp Leu Glu Phe Cys Leu Tyr
   690 695 700
   Gly Arg Glu Glu His Leu Thr Thr Asn Gly Tyr Arg Lys Ile Pro Gly
   705 710 715 720
   Asp Lys Cys Gln Gly Gly Val Asn Pro Val Arg Glu Val Lys Asp Leu
                   725                 730                 735
   Lys Lys Lys Cys Thr Ser Asn Phe Leu Ser Pro Glu Lys Gln Asn Ser
               740                 745                 750
   Lys Ser Asn Ser Val Pro Ile Ile Leu Ala Ile Val Gly Leu Met Leu
           755                 760                 765
   Val Thr Val Val Ala Gly Val Leu Ile Val Lys Lys Tyr Val Cys Gly
       770                 775                 780
   Gly Arg Phe Leu Val His Arg Tyr Ser Val Leu Gln Gln His Ala Glu
   785                 790                 795                 800
   Ala Asn Gly Val Asp Gly Val Asp Ala Leu Asp Thr Ala Ser His Thr
                   805                 810                 815
   Asn Lys Ser Gly Tyr His Asp Asp Ser Asp Glu Asp Leu Leu Glu
               820                 825                 830